# EUROPEAN PATENT SPECIFICATION

(11) **EP 3 336 083 B1**
(45) Date of publication and mention of the grant of the patent: **27.05.2020**
(21) Application number: 16837009.6
(22) Date of filing: 05.08.2016
(51) Int. Cl.: C07D 307/93, C07C 69/753, C08G 73/10, C08L 79/08, C07B 61/00

(54) **TETRACARBOXYLIC DIANHYDRIDE, CARBONYL COMPOUND, POLYAMIC ACID AND POLYIMIDE AND METHODS RESPECTIVELY FOR PRODUCING THESE COMPOUNDS, SOLUTION PREPARED USING POLYAMIC ACID, AND FILM PRODUCED USING POLYIMIDE**
TETRACARBONSÄUREDIANHYDRID, CARBONYLVERBINDUNG, POLYAMIDSÄURE UND POLYIMID SOWIE VERFAHREN ZUR HERSTELLUNG DIESER VERBINDUNGEN, MIT DER POLYAMIDSÄURE HERGESTELLTE LÖSUNG UND MIT DEM POLYIMID HERGESTELLTE FOLIE
DIANHYDRIDE TÉTRACARBOXYLIQUE, COMPOSÉ CARBONYLE, ACIDE POLYAMIQUE ET POLYIMIDE, ET PROCÉDÉS RESPECTIFS DE PRODUCTION DE CES COMPOSÉS, SOLUTION PRÉPARÉE À L'AIDE D'ACIDE POLYAMIQUE, ET FILM PRODUIT EN UTILISANT UN POLYIMIDE

(30) Priority: 14.08.2015 JP 2015160210
(43) Date of publication of application: 20.06.2018
(73) Proprietor: JXTG Nippon Oil & Energy Corporation, Chiyoda-ku Tokyo 1008162 (JP)
(72) Inventor: MATSUO, Yusuke, Tokyo 1008162 (JP); NOGUCHI, Masaki, Tokyo 1008162 (JP); WATANABE, Daisuke, Tokyo 1008162 (JP); UENO, Ryuichi, Tokyo 1008162 (JP); KOMATSU, Shinichi, Tokyo 1008162 (JP)
(74) Representative: Hoffmann Eitle
(86) International application number: PCT/JP2016/073083
(87) International publication number: WO 2017/030019

(56) References cited:
- WO-A1-2011/033751
- WO-A1-2011/099518
- WO-A1-2013/021942
- WO-A1-2015/083649
- CN-A- 104 804 188
- JP-A- H1 160 732
- JP-A- 2014 133 887
- KUSAMA, M. ET AL.: 'Soluble Polyimides with Polyalicyclic Structure. 3. Polyimides from (4arH,8acH)-Decahydro-1t,4t:5c,8c- dimethanonaphthalene-2t,3t,6c,7c- tetracarboxylic 2,3:6,7-Dianhydride' MACROMOLECULES vol. 27, 1994, ISSN 0024-9297 pages 1117 - 1123, XP000433176

## Description

### [Technical Field]

The present invention relates to a tetracarboxylic dianhydride, a carbonyl compound, a polyamic acid, a polyimide, methods for producing the same, a solution of the polyamic acid, and a film using the polyimide.

### [Background Art]

In general, tetracarboxylic dianhydrides are useful as raw materials for producing polyimide resins, as epoxy curing agents, and as the like. Of the tetracarboxylic dianhydrides, aromatic tetracarboxylic dianhydrides such as pyromellitic dianhydride have been mainly used as raw materials for polyimide resins used in the field of electronic devices or the like. Then, among polyimides obtained by using such aromatic tetracarboxylic dianhydrides, for example, a polyimide (trade name: "Kapton") marketed by DU PONT-TORAY CO., LTD. has been conventionally widely known as a material necessary for cutting-edge industries for aerospace and aviation applications and the like. Conventional polyimides obtained by using aromatic tetracarboxylic dianhydrides have excellent physical properties in terms of heat resistance; however, such polyimides are colored (yellow to brown), and cannot be used in the optical and other applications where transparency is necessary.

Under such circumstances, in order to produce a polyimide which can be used in optical and other applications, research has been conducted on various tetracarboxylic dianhydrides. In recent years, tetracarboxylic dianhydrides have been reported which make it possible to produce polyimides having a sufficiently high light transmittance and also having a sufficiently high heat resistance. For example, WO 2011/099518 A1 discloses a norbornane-2-spiro-α-cycloalkanone-α'-spiro-2"-norbor nane-5,5",6,6"-tetracarboxylic dianhydride having a specific structure. WO 2013/021942 A1 provides for a similar disclosure in that it describes polyimides containing a repeating unit derived from such bis-norbonane-tetracarboxylic acid derivatives, wherein the two norbonane units are linked by spiro fused cyclic groups. Such bis-norbonane-tetracarboxylic acid derivatives are also mentioned in WO 2015/083649 A1. JP H11 60732 A discloses bis-norbonane-tetracarboxylic acid derivatives wherein two norbonane units are linked by functional groups, namely sulfonyl groups. This Japanese patent application, and moreover CN 104 804 188 A, JP 2014 133887 A and WO 2011/033751 A1 disclose 5,5' directly linked dicyclohexyl tetracarboxylic acid derivatives. All of the above patent applications generally relate to polyimide resins for the preparation of films, wherein the tetracarboxylic acid derivatives constitute monomer units. Kusama, M. et al. in Macromolecules, vol. 27, 1994, 1117-1123 use directly fused bis-norbonane-tetracarboxylic acid derivatives to prepare polyimides.

### [Summary of Invention]

### [Technical Problem]

The tetracarboxylic dianhydride as described in WO 2011/099518 A1 above can be used for producing polyimides having a sufficiently high light transmittance and a sufficiently high heat resistance. However, some of applications and the like of such polyimides require further enhancement of heat resistance while sufficiently maintaining an optical characteristic (light transmittance). For this reason, in the field of polyimides, there is a demand for the advent of an polyimide which can sufficiently maintain an optical characteristic (light transmittance) while achieving has an even higher heat resistance than polyimides which can be obtained by using the tetracarboxylic dianhydride described in WO 2011/099518 A1 above, and which have a sufficiently high light transmittance and a sufficiently high heat resistance.

The present invention has been made in view of the above-described problems of the conventional techniques, and an object thereof is to provide a tetracarboxylic dianhydride which is usable as a raw material monomer for producing a polyimide having a sufficient light transmittance and a heat resistance at a higher level, as well as a method for producing a tetracarboxylic dianhydride which makes it possible to produce the tetracarboxylic dianhydride efficiently and surely. In addition, another object of the present invention is to provide a carbonyl compound which can be used for efficiently producing the tetracarboxylic dianhydride as well as a method for producing a carbonyl compound which makes it possible to produce the carbonyl compound efficiently and surely.

Moreover, still another object of the present invention is to provide a polyimide which can have a sufficient light transmittance and a heat resistance at a higher level and a method for producing a polyimide which makes it possible to produce the polyimide efficiently and surely, as well as to provide a film using the polyimide. Furthermore, yet another object of the present invention is to provide a polyamic acid which can be preferably utilized for producing the polyimide and which can be efficiently produced by using the tetracarboxylic dianhydride and a method for producing a polyamic acid which makes it possible to produce the polyamic acid efficiently and surely, as well as to provide a polyamic acid solution containing the polyamic acid.

### [Solution to Problem]

The present inventors have conducted intensive study to achieve the above-described objects, and consequently have found that a method including: first preparing, as a tetracarboxylic dianhydride, a compound represented by the following general formula (1); and then producing a polyimide by using the compound makes it possible to produce a polyimide which can have a sufficient light transmittance and a heat resistance at a higher level. This finding has led to the completion of the present invention.

Specifically, first, a tetracarboxylic dianhydride of the present invention is a compound represented by the following general formula (1): 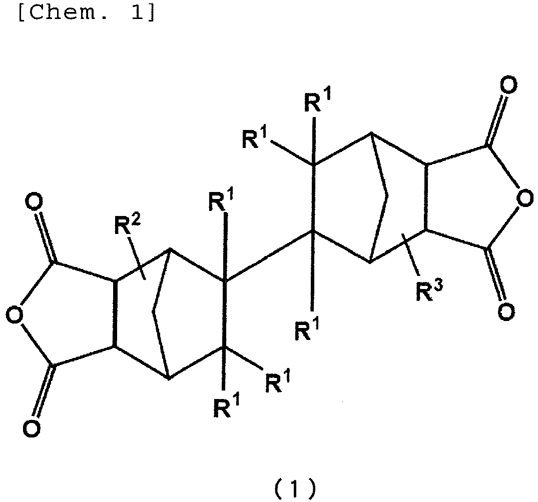
[in the formula (1), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group, and
R² and R³ each independently represent one selected from the group consisting of a hydrogen atom and alkyl groups having 1 to 10 carbon atoms].

In the above-described tetracarboxylic dianhydride of the present invention, each of multiple R¹s, R², and R³ in the general formula (1) is preferably a hydrogen atom.

Meanwhile, a carbonyl compound of the present invention is a compound represented by the following general formula (2): 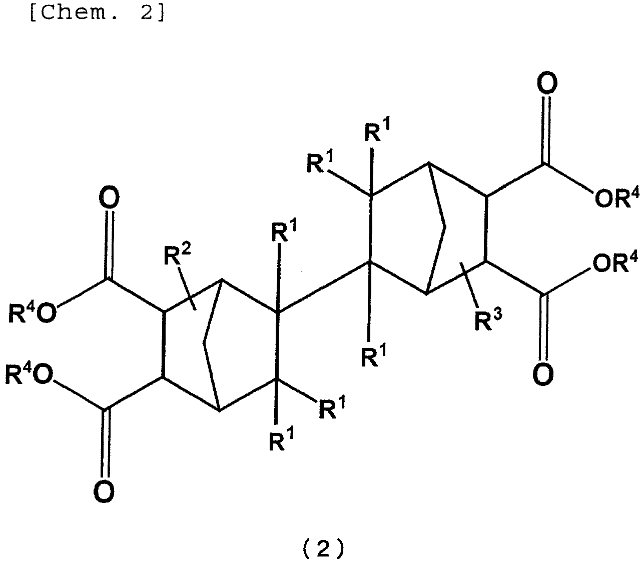
[in the formula (2), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group,
R² and R³ each independently represent one selected from the group consisting of a hydrogen atom and alkyl groups having 1 to 10 carbon atoms, and
multiple R⁴s each independently represent one selected from the group consisting of a hydrogen atom, alkyl groups having 1 to 10 carbon atoms , cycloalkyl groups having 3 to 10 carbon atoms, alkenyl groups having 2 to 10 carbon atoms, aryl groups having 6 to 20 carbon atoms, and aralkyl groups having 7 to 20 carbon atoms].

In the above-described carbonyl compound of the present invention, each of multiple R¹s, R², and R³ in the general formula (2) is preferably a hydrogen atom.

A method for producing a tetracarboxylic dianhydride of the present invention comprises
heating a carbonyl compound represented by the following general formula (2): 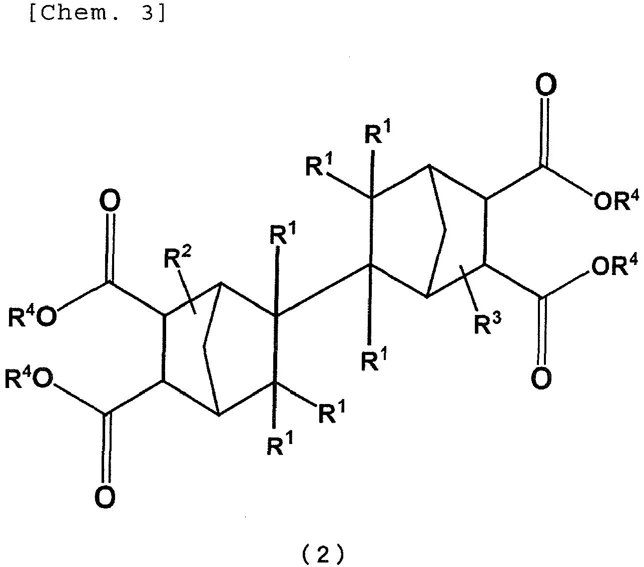
[in the formula (2), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group,
R² and R³ each independently represent one selected from the group consist ing of a hydrogen atom and alkyl groups having 1 to 10 carbon atoms, and
multiple R⁴s each independently represent one selected from the group consisting of a hydrogen atom, alkyl groups having 1 to 10 carbon atoms, cycloalkyl groups having 3 to 10 carbon atoms, alkenyl groups having 2 to 10 carbon atoms, aryl groups having 6 to 20 carbon atoms, and aralkyl groups having 7 to 20 carbon atoms] in a carboxylic acid having 1 to 5 carbon atoms with an acid catalyst being used, to thereby obtain a tetracarboxylic dianhydride represented by the following general formula (1):

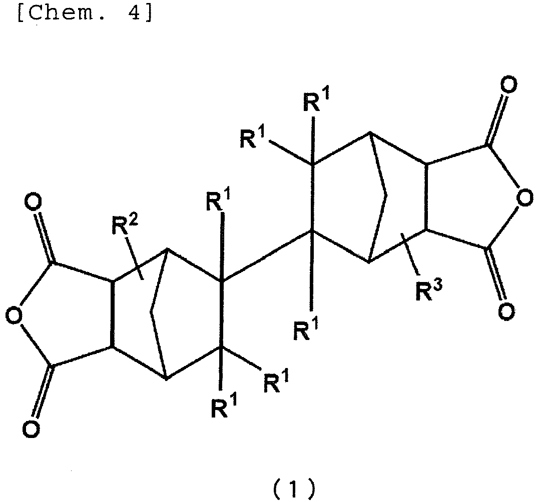

[in the formula (1), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group, and
R² and R³ each independently represent one selected from the group consisting of a hydrogen atom and alkyl groups having 1 to 10 carbon atoms].

In the above-described method for producing a tetracarboxylic dianhydride of the present invention, it is preferable that the heating further uses acetic anhydride.

In addition, a method for producing a carbonyl compound of the present invention comprises
reacting a norbornene-based compound represented by the following general formula (3): 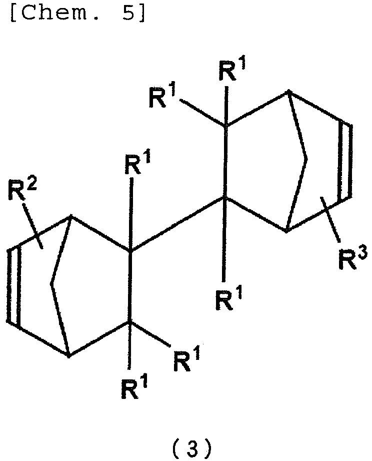
[in the formula (3), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group, and
R² and R³ each independently represent one selected from the group consisting of a hydrogen atom and alkyl groups having 1 to 10 carbon atoms] with an alcohol and carbon monoxide in the presence of a palladium catalyst and an oxidant, to thereby obtain a carbonyl compound represented by the following general formula (2):

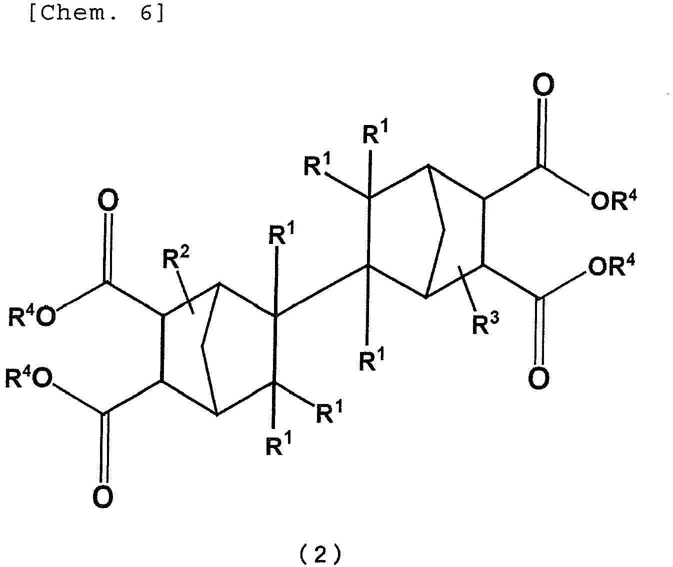

[in the formula (2), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group,
R² and R³ each independently represent one selected from the group consisting of a hydrogen atom and alkyl groups having 1 to 10 carbon atoms, and
multiple R⁴s each independently represent one selected from the group consisting of a hydrogen atom, alkyl groups having 1 to 10 carbon atoms , cycloalkyl groups having 3 to 10 carbon atoms, alkenyl groups having 2 to 10 carbon atoms, aryl groups having 6 to 20 carbon atoms, and aralkyl groups having 7 to 20 carbon atoms].

A polyimide of the present invention comprises a repeating unit represented by the following general formula (4) : 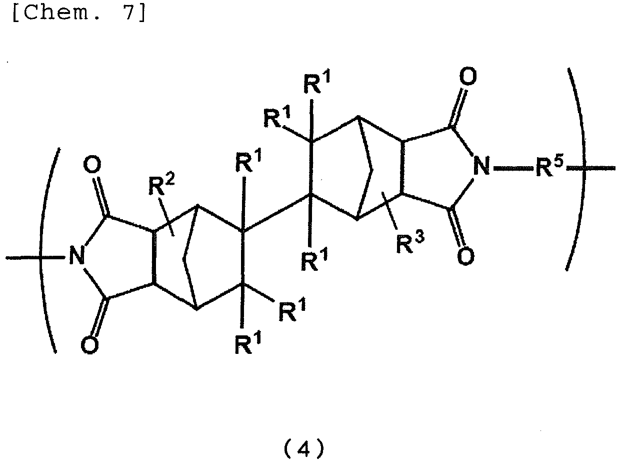
[in the formula (4), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group,
R² and R³ each independently represent one selected from the group consisting of a hydrogen atom and alkyl groups having 1 to 10 carbon atoms, and
R⁵ represents an arylene group having 6 to 40 carbon atoms].

A polyamic acid of the present invention comprises a repeating unit represented by the following general formula (5): 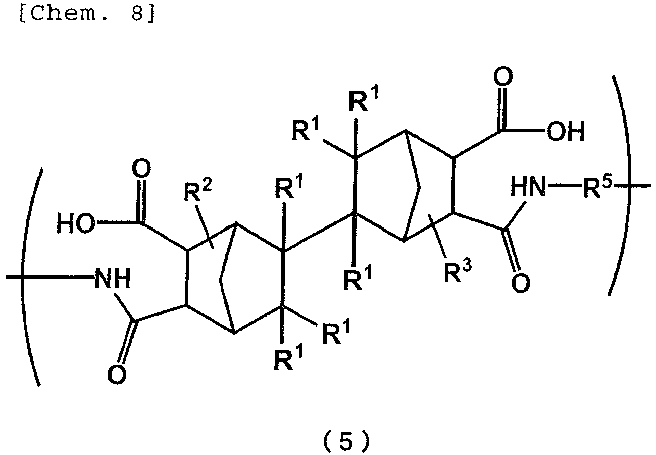
[in the formula (5), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group,
R² and R³ each independently represent one selected from the group consisting of a hydrogen atom and alkyl groups having 1 to 10 carbon atoms, and
R⁵ represents an arylene group having 6 to 40 carbon atoms]. Note that the polyamic acid can be obtained as a reaction intermediate when the above-described polyimide of the present invention is produced.

In addition, a method for producing a polyamic acid of the present invention comprises
reacting a tetracarboxylic dianhydride represented by the following general formula (1): 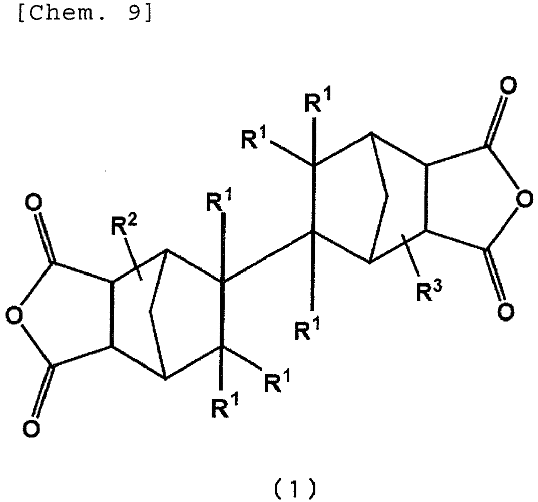
[in the formula (1), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group, and
R² and R³ each independently represent one selected from the group consisting of a hydrogen atom and alkyl groups having 1 to 10 carbon atoms] with an aromatic diamine represented by the following general formula (6):
[Chem. 10]

H₂N-R⁵-NH₂ (6)

[in the formula (6), R⁵ represents an arylene group having 6 to 40 carbon atoms] in the presence of an organic solvent, to thereby obtain a polyamic acid having a repeating unit represented by the following general formula (5): 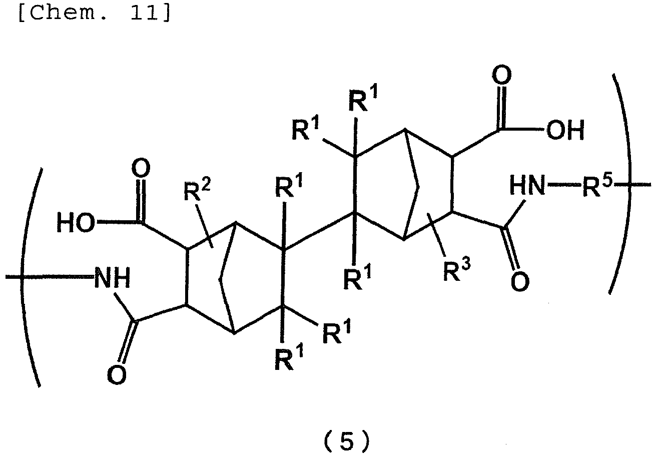
[in the formula (5), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group,
R² and R³ each independently represent one selected from the group consisting of a hydrogen atom and alkyl groups having 1 to 10 carbon atoms, and
R⁵ represents an arylene group having 6 to 40 carbon atoms].

In addition, a method for producing a polyimide of the present invention comprises
imidizing a polyamic acid having a repeating unit represented by the following general formula (5): 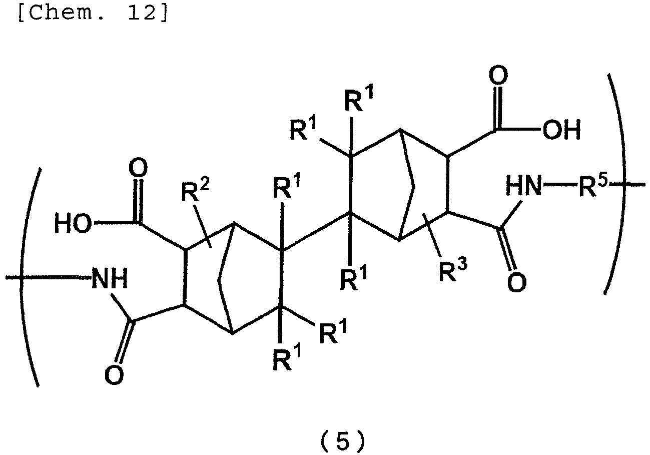
[in the formula (5), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group,
R² and R³ each independently represent one selected from the group consisting of a hydrogen atom and alkyl groups having 1 to 10 carbon atoms, and
R⁵ represents an arylene group having 6 to 40 carbon atoms], to thereby obtain a polyimide having a repeating unit represented by the following general formula (4):

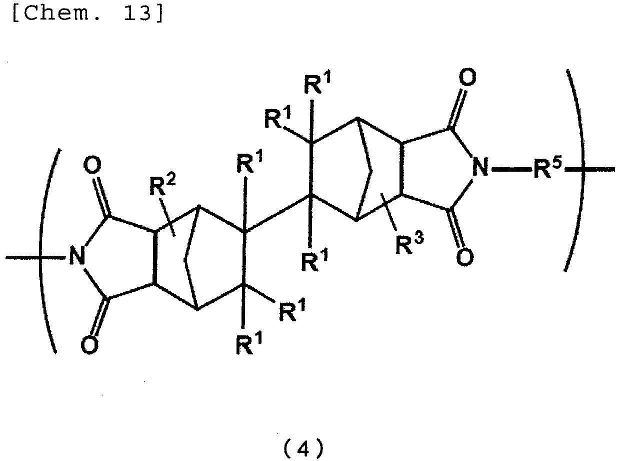

[in the formula (4), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group,
R² and R³ each independently represent one selected from the group consisting of a hydrogen atom and alkyl groups having 1 to 10 carbon atoms, and
R⁵ represents an arylene group having 6 to 40 carbon atoms].

In addition, the above-described method for producing a polyimide of the present invention preferably comprises the step of
reacting a tetracarboxylic dianhydride represented by the following general formula (1): 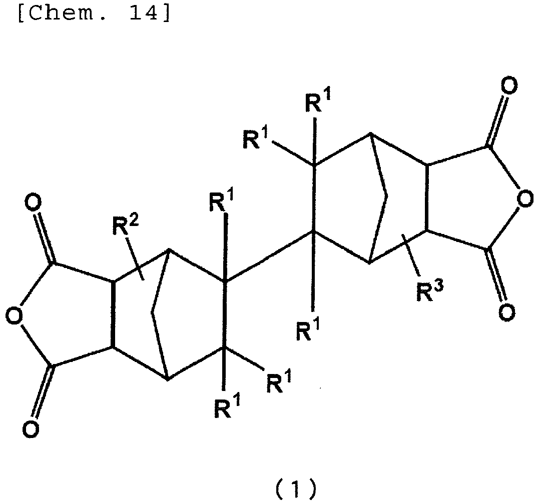
[in the formula (1), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group, and
R² and R³ each independently represent one selected from the group consisting of a hydrogen atom and alkyl groups having 1 to 10 carbon atoms] with an aromatic diamine represented by the following general formula (6):
[Chem. 15]

H₂N-R⁵-NH₂ (6)

[in the formula (6), R⁵ represents an arylene group having 6 to 40 carbon atoms] in the presence of an organic solvent, to thereby obtain a polyamic acid having a repeating unit represented by the following general formula (5): 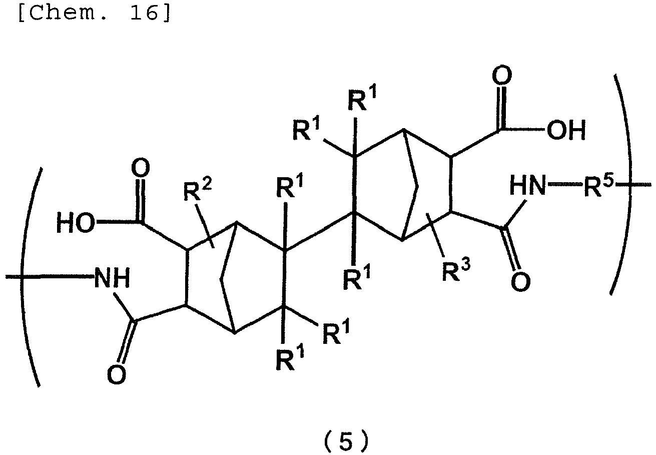
[in the formula (5), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group,
R² and R³ each independently represent one selected from the group consisting of a hydrogen atom and alkyl groups having 1 to 10 carbon atoms, and
R⁵ represents an arylene group having 6 to 40 carbon atoms]. In this case, the above-described method for producing a polyimide of the present invention may be a method comprising the steps of: reacting the tetracarboxylic dianhydride represented by the general formula (1) with the aromatic diamine represented by the general formula (6) in the presence of the organic solvent, to thereby obtain a polyamic acid comprising the repeating unit represented by the general formula (5) ; and imidizing the polyamic acid, to thereby obtain a polyimide comprising the repeating unit represented by the general formula (4), and hence it is also possible to more efficiently produce the polyimide by continuous steps.

In addition, a polyamic acid solution of the present invention comprises: the above-described polyamic acid of the present invention; and an organic solvent. The polyamic acid solution (resin solution: varnish) makes it possible to efficiently produce a polyimide in various shapes. In addition, a film of the present invention comprises the above-described polyimide of the present invention. Note that since the film of the present invention comprises the above-described polyimide of the present invention, the film has not only a sufficient light transmittance (transparency) but also a sufficiently high heat resistance. Thus, it is possible to suppress the deterioration and the like of the film at a higher level even when exposed under high- temperature conditions. For example, a step of laminating a polyimide film with a metal oxide or the like is a high-temperature process. The present inventors presume that the polyimide of the present invention is also sufficiently applicable to such a step.

### [Advantageous Effects of Invention]

According to the present invention, it is possible to provide a tetracarboxylic dianhydride which is usable as a raw material monomer for producing a polyimide having a sufficient light transmittance and a heat resistance at a higher level, as well as a method for producing a tetracarboxylic dianhydride which makes it possible to produce the tetracarboxylic dianhydride efficiently and surely.

In addition, according to the present invention, it is possible to provide a carbonyl compound which can be used for efficiently producing the tetracarboxylic dianhydride as well as a method for producing a carbonyl compound which makes it possible to produce the carbonyl compound efficiently and surely.

Moreover, according to the present invention, it is possible to provide a polyimide which can have a sufficient light transmittance and a heat resistance at a higher level and a method for producing a polyimide which makes it possible to produce the polyimide efficiently and surely, as well as a film using the polyimide.

Furthermore, according to the present invention, it is possible to produce a polyamic acid which can be preferably utilized for producing the polyimide and which can be efficiently produced by using the tetracarboxylic dianhydride and a method for producing a polyamic acid which makes it possible to produce the polyamic acid efficiently and surely, as well as a polyamic acid solution containing the polyamic acid.

### [Brief Description of Drawings]

[Fig. 1] Fig. 1 is a graph showing an IR spectrum of a tetraester compound obtained in Example 1.
[Fig. 2] Fig. 2 is a graph showing a ¹H-NMR (CDCl₃) spectrum of the tetraester compound obtained in Example 1.
[Fig. 3] Fig. 3 is a graph showing a ¹³C-NMR (CDCl₃) spectrum of the tetraester compound obtained in Example 1.
[Fig. 4] Fig. 4 is a graph showing an IR spectrum of a tetracarboxylic dianhydride obtained in Example 2.
[Fig. 5] Fig. 5 is a graph showing a ¹H-NMR (DMSO-d₆) spectrum of the tetracarboxylic dianhydride obtained in Example 2.
[Fig. 6] Fig. 6 is a graph showing a ¹³C-NMR (DMSO-de) spectrum of the tetracarboxylic dianhydride obtained in Example 2.
[Fig. 7] Fig. 7 is a graph showing an IR spectrum of a polyimide obtained in Example 3.
[Fig. 8] Fig. 8 is a graph showing an IR spectrum of a polyimide obtained in Example 4.
[Fig. 9] Fig. 9 is a graph showing an IR spectrum of a polyimide obtained in Example 5.
[Fig. 10] Fig. 10 is a graph showing an IR spectrum of a tetracarboxylic dianhydride obtained in Example 6.
[Fig. 11] Fig. 11 is a graph showing a ¹H-NMR (DMSO-d₆) spectrum of the tetracarboxylic dianhydride obtained in Example 6.
[Fig. 12] Fig. 12 is a graph showing a ¹³C-NMR (DMSO-d₆) spectrum of the tetracarboxylic dianhydride obtained in Example 6.
[Fig. 13] Fig. 13 is a graph showing an IR spectrum of a polyimide obtained in Example 7.
[Fig. 14] Fig. 14 is a graph showing an IR spectrum of a polyimide obtained in Example 8.

### [Description of Embodiments]

Hereinafter, the present invention will be described in detail based on preferred embodiments thereof.

### [Tetracarboxylic Dianhydride]

A tetracarboxylic dianhydride of the present invention is a compound represented by the following general formula (1): 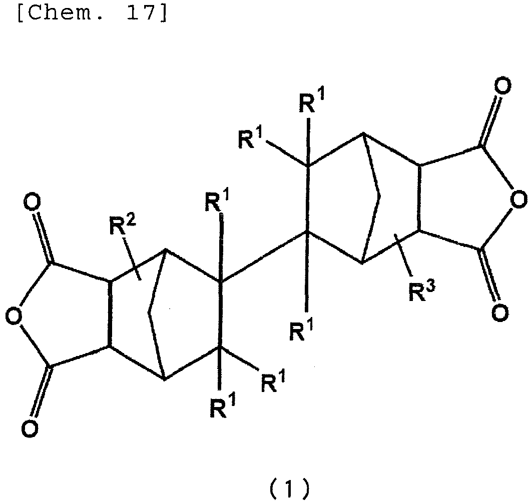
[in the formula (1), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group, and
R² and R³ each independently represent one selected from the group consisting of a hydrogen atom and alkyl groups having 1 to 10 carbon atoms].

An alkyl group which maybe selected as R¹ in the general formula (1) is an alkyl group having 1 to 10 carbon atoms. If the number of carbon atoms exceeds 10, the heat resistance of a polyimide obtained in the use as a monomer for the polyimide is lowered. In addition, the number of carbon atoms of such an alkyl group which may be selected as R¹ is preferably 1 to 6, more preferably 1 to 5, further preferably 1 to 4, and particularly preferably 1 to 3 from the viewpoint that a higher heat resistance can be obtained when a polyimide is produced. In addition, such an alkyl group which may be selected as R¹ may be linear or branched.

In addition, of multiple R¹s in the general formula (1), two R¹s connected to a common carbon atom may together form a methylidene group (= CH₂). To be more specific, two R¹s connected to a common carbon atom in the general formula (1) may together be connected to the carbon atom (of the carbon atoms forming a norbornane ring structure, the carbon atom connected with two R¹s) as a methylidene group (methylene group) via double bond.

Multiple R¹s in the general formula (1) are each independently more preferably a hydrogen atom, a methyl group, an ethyl group, an n-propyl group, and an isopropyl group, and particularly preferably a hydrogen atom and a methyl group, for example, from the viewpoint that a higher heat resistance can be obtained when a polyimide is produced, that the raw material is readily available, and that the purification is easier. In addition, multiple R¹s in the formula (1) may be the same as one another or different from one another, and are preferably the same from the viewpoints of ease of purification and the like.

R² and R³ in the general formula (1) are each independently one selected from the group consisting of a hydrogen atom and alkyl groups having 1 to 10 carbon atoms . If the number of carbon atoms of such an alkyl group which may be selected as R² and R³ exceeds 10, the heat resistance of a polyimide obtained in the use as a monomer for the polyimide is lowered. In addition, such an alkyl group which may be selected as R² and R³ is preferably 1 to 6, more preferably 1 to 5, further preferably 1 to 4, and particularly preferably 1 to 3 from the viewpoint that a higher heat resistance can be obtained when a polyimide is produced. In addition, such an alkyl group which may be selected as R² and R³ may be linear or branched.

R² and R³ in the general formula (1) are each independently more preferably a hydrogen atom, a methyl group, an ethyl group, an n-propyl group, and an isopropyl group, and particularly preferably a hydrogen atom and a methyl group, for example, from the viewpoint that a higher heat resistance can be obtained when a polyimide is produced, that the raw material is readily available, and that the purification is easier. In addition, the R² and R³ in the formula (1) may be the same as each other or different from each other, and are preferably the same from the viewpoints of ease of purification and the like.

In addition, each of multiple R¹s, R², and R³ in the general formula (1) is particularly preferably a hydrogen atom. As described above, in the compound represented by the general formula (1), if each of the substituents represented by R¹s, R², and R³ is a hydrogen atom, the yield of the compound tends to increase. In addition, when a polyimide containing the compound as a monomer is produced, a higher heat resistance tends to be obtained.

In addition, when a polyimide is produced by using such a tetracarboxylic dianhydride of the present invention as a monomer, it is possible to reduce the loss tangent (tanδ) to a lower value compared to the case where a conventional alicyclic tetracarboxylic dianhydride is utilized. For this reason, it is possible to sufficiently reduce transmission loss when a polyimide containing the tetracarboxylic dianhydride of the present invention as a monomer is produced and the resultant is utilized in interlayer insulating film material for semiconductor, a board film for a flexible printed circuit board (FPC), and the like. For this reason, the tetracarboxylic dianhydride of the present invention is also applicable to a material and the like for producing a high frequency band material (for example, a large-scale integration (LSI), an electronic circuit, and the like) and can be preferably utilized in these applications.

Although a method for producing a tetracarboxylic dianhydride of the present invention is not particularly limited, it is preferable to employ the method for producing a tetracarboxylic dianhydride of the present invention because the tetracarboxylic dianhydride of the present invention can be produced more efficiently. Note that the method for producing a tetracarboxylic dianhydride of the present invention is described later.

In the foregoing, the tetracarboxylic dianhydride of the present invention has been described. Next, a carbonyl compound of the present invention is described which can be preferably utilized when the tetracarboxylic dianhydride of the present invention is produced.

### [Carbonyl Compound]

A carbonyl compound of the present invention is a compound represented by the following general formula (2) : 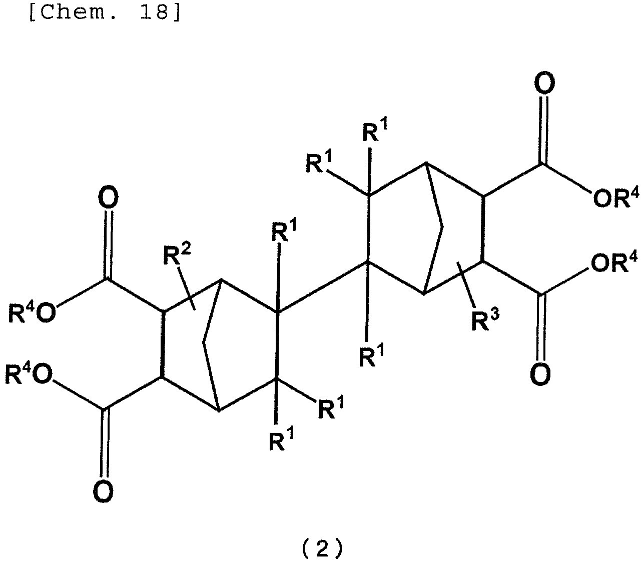
[in the formula (2), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group,
R² and R³ each independently represent one selected from the group consisting of a hydrogen atom and alkyl groups having 1 to 10 carbon atoms, and
multiple R⁴s each independently represent one selected from the group consisting of a hydrogen atom, alkyl groups having 1 to 10 carbon atoms, cycloalkyl groups having 3 to 10 carbon atoms, alkenyl groups having 2 to 10 carbon atoms, aryl groups having 6 to 20 carbon atoms, and aralkyl groups having 7 to 20 carbon atoms]. As described above, the carbonyl compound of the present invention may include a tetracarboxylic acid which is represented by the general formula (2) and in which all of R⁴s in the formula are hydrogen atoms, and an ester compound which is represented by the general formula (2) and in which any of R⁴s in the formula is a group other than a hydrogen atom (compound containing an ester group: a tetraester compound if none of R⁴s in the formula (2) is a hydrogen atom).
R¹s, R², and R³ in the general formula (2) are the same as R¹s, R², and R³ in the general formula (1), and preferred ones thereof are also the same as R¹s, R², and R³ in the general formula (1). Note that multiple R¹s in the general formula (2) may be the same as one another or different from one another, and are preferably the same from the viewpoint of ease of purification and the like. Moreover, R² and R³ in the general formula (2) may be the same as each other or different from each other, and are preferably the same from the viewpoint of ease of purification and the like.

In addition, each of multiple R¹s, R², and R³ in the general formula (2) is particularly preferably a hydrogen atom. As described above, in the compound represented by the general formula (2), if each of the substituents represented by R¹s, R², and R³ is a hydrogen atom, the yield of the compound tends to increase. In addition, when an acid dianhydride is formed from the compound and a polyimide containing the obtained acid dianhydride as a monomer is produced, a higher heat resistance tends to be obtained.

In addition, an alkyl group which may be selected as R⁴ in the general formula (2) is an alkyl group having 1 to 10 carbon atoms. If the number of carbon atoms of such an alkyl group exceeds 10, purification is difficult. In addition, the number of carbon atoms of such an alkyl group which may be selected as multiple R⁴s is more preferably 1 to 5 and further preferably 1 to 3 from the viewpoint that the purification is easier. In addition, such an alkyl group which may be selected as multiple R⁴s may be linear or branched.

In addition, a cycloalkyl group which may be selected as R⁴ in the general formula (2) is a cycloalkyl group having 3 to 10 carbon atoms. If the number of carbon atoms of such a cycloalkyl group exceeds 10, purification is difficult. In addition, the number of carbon atoms of such a cycloalkyl group which may be selected as multiple R⁴s is more preferably 3 to 8 and further preferably 5 and 6 from the viewpoint that the purification is easier.

Moreover, an alkenyl group which may be selected as R⁴ in the general formula (2) is an alkenyl group having 2 to 10 carbon atoms. If the number of carbon atoms of such an alkenyl group exceeds 10, purification is difficult. In addition, the number of carbon atoms of such an alkenyl group which may be selected as multiple R⁴s is more preferably 2 to 5 and further preferably 2 and 3 from the viewpoint that the purification is easier.

In addition, an aryl group which may be selected as R⁴ in the general formula (2) is an aryl group having 6 to 20 carbon atoms. If the number of carbon atoms of such an aryl group exceeds 20, purification is difficult. In addition, the number of carbon atoms of such an aryl group which may be selected as multiple R⁴s is more preferably 6 to 10 and further preferably 6 to 8 from the viewpoint that the purification is easier.

In addition, an aralkyl group which may be selected as R⁴ in the general formula (2) is an aralkyl group having 7 to 20 carbon atoms. If the number of carbon atoms of such an aralkyl group exceeds 20, purification is difficult . In addition, the number of carbon atoms of such an aralkyl group which may be selected as multiple R⁴s is more preferably 7 to 10 and further preferably 7 to 9 from the viewpoint that the purification is easier.

Moreover, multiple R⁴s in the general formula (2) are each independently preferably a methyl group, an ethyl group, an n-propyl group, an isopropyl group, an n-butyl group, an isobutyl group, a sec-butyl, a t-butyl, a cyclohexyl group, an allyl group, a phenyl group, or a benzyl group, more preferably a methyl group, an ethyl group, or an n-propyl group, further preferably a methyl group or an ethyl group, and particularly preferably a methyl group from the viewpoint that the purification is easier. Note that multiple R⁴s in the general formula (2) may be the same as one another or different from one another, and are more preferably the same from the viewpoint of synthesis.

Although a method for producing a carbonyl compound of the present invention is not particularly limited, it is preferable to employ the method for producing a carbonyl compound of the present invention because the carbonyl compound of the present invention can be produced more efficiently. Note that the method for producing a carbonyl compound of the present invention is described later.

In the foregoing, the carbonyl compound of the present invention has been described. Next, a method for producing a tetracarboxylic dianhydride of the present invention is described.

### [Method for Producing Tetracarboxylic Dianhydride]

A method for producing a tetracarboxylic dianhydride of the present invention comprises
heating a carbonyl compound represented by the following general formula (2): 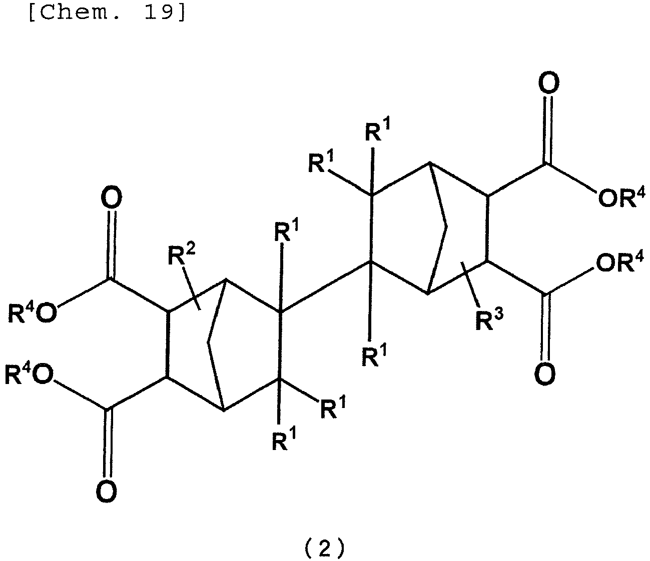
[in the formula (2), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group,
R² and R³ each independently represent one selected from the group consisting of a hydrogen atom and alkyl groups having 1 to 10 carbon atoms, and
multiple R⁴s each independently represent one selected from the group consisting of a hydrogen atom, alkyl groups having 1 to 10 carbon atoms, cycloalkyl groups having 3 to 10 carbon atoms, alkenyl groups having 2 to 10 carbon atoms, aryl groups having 6 to 20 carbon atoms, and aralkyl groups having 7 to 20 carbon atoms] in a carboxylic acid having 1 to 5 carbon atoms with an acid catalyst being used, to thereby obtain a tetracarboxylic dianhydride represented by the following general formula (1):

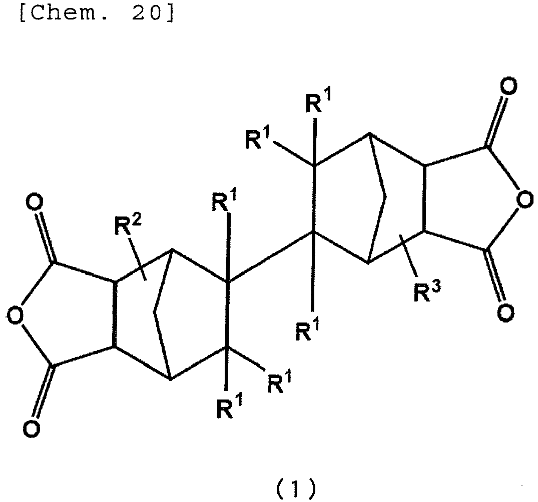

[in the formula (1), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group, and
R² and R³ each independently represent one selected from the group consisting of a hydrogen atom and alkyl groups having 1 to 10 carbon atoms].

The carbonyl compound represented by the general formula (2), which is used in the method for producing a tetracarboxylic dianhydride of the present invention, is the same as the corresponding one described for the above-described carbonyl compound of the present invention, and preferred ones thereof are also the same.

An acid catalyst used in the method for producing a tetracarboxylic dianhydride of the present invention is not particularly limited, and may be a homogeneous acid catalyst or an inhomogeneous acid catalyst (solid catalyst) . Among such acid catalysts, the homogeneous acid catalyst is preferable from the viewpoint of ease of purification.

Such a homogeneous acid catalyst is not particularly limited, and it is possible to appropriately utilize a known homogeneous acid catalyst which can be used in reacting a carboxylic acid into an anhydride and in reacting an ester compound into an acid anhydride. Examples of such a homogeneous acid catalyst include trifluoromethanesulfonic acid, tetrafluoroethanesulfonic acid, pentafluoroethanesulfonic acid, heptafluoropropanesulfonic acid, heptafluoroisopropanesulfonic acid, nonafluorobutanesulfonic acid, heptafluorodecanesulfonic acid, bis(nonafluorobutanesulfonyl)imide, N,N-bis(trifluoromethanesulfonyl)imide, and chlorodifluoroacetic acid.

In addition, such a homogeneous acid catalyst is more preferably trifluoromethanesulfonic acid, tetrafluoroethanesulfonic acid, nonafluorobutanesulfonic acid, and chlorodifluoroacetic acid and further preferably trifluoromethanesulfonic acid and tetrafluoroethanesulfonic acid from the viewpoint of improvement in reaction yield. Note that one of these homogeneous acid catalysts can be used alone, or two or more thereof can be used in combination.

In addition, the amount of the acid catalyst (more preferably a homogeneous acid catalyst) used is not particularly limited, and is preferably such that the amount of moles of the acid of the acid catalyst is 0.001 to 2.00 mole equivalents (more preferably 0.01 to 1.00 mole equivalents) relative to the amount of the carbonyl compound (raw material compound of the tetracarboxylic dianhydride) represented by the general formula (2) used (amount of moles) . If the amount of such an acid catalyst used is less than the lower limit, the reaction rate tends to be lowered. Meanwhile, if the amount of such an acid catalyst used exceeds the upper limit, purification is rather difficult and the purity of the product tends to be lowered. Note that the amount of moles of the acid of the acid catalyst herein is the amount of moles in terms of the functional groups (for example, a sulfonic acid group (sulfo group) and a carboxylic acid group (carboxy group)) in the acid catalyst.

In addition, the amount of the acid catalyst (more preferably a homogeneous acid catalyst) used is preferably 0.1 to 100 parts by mass and more preferably 1 to 20 parts by mass relative to 100 parts by mass of the carbonyl compound represented by the general formula (2). If the amount of such an acid catalyst used is less than the lower limit, the reaction rate tends to be lowered. Meanwhile, if the amount of such an acid catalyst used exceeds the upper limit, by-products tend to increase.

Moreover, the present invention uses a carboxylic acid having 1 to 5 carbon atoms (hereinafter, sometimes simply referred to as "lower carboxylic acid"). If the number of carbon atoms of such a lower carboxylic acid exceeds the upper limit, production and purification are difficult. In addition, examples of such a lower carboxylic acid include formic acid, acetic acid, propionic acid, butyric acid, and the like, of which formic acid, acetic acid, and propionic acid are preferable, and formic acid and acetic acid are more preferable from the viewpoint of ease of production and purification. One of these lower carboxylic acids can be used alone, or two or more thereof can be used in combination.

In addition, the amount of such a lower carboxylic acid (for example, formic acid, acetic acid, and propionic acid) used is not particularly limited, and is preferably such that the amount of moles of the lower carboxylic acid is 4 to 100 times the amount of moles of the carbonyl compound represented by the general formula (2). If the amount of such a lower carboxylic acid (for example, formic acid, acetic acid, and propionic acid) used is less than the lower limit, the yield tends to be lowered. Meanwhile, if the amount of such a lower carboxylic acid exceeds the upper limit, the reaction rate tends to be lowered.

In addition, since the present invention heats the carbonyl compound in the lower carboxylic acid, it is preferable that the carbonyl compound be contained in the lower carboxylic acid. The content of the carbonyl compound represented by the general formula (2) in such a lower carboxylic acid is preferably 1 to 40% by mass and more preferably 2 to 30% by mass. If the content of such a carbonyl compound is less than the lower limit, the yield tends to be lowered. Meanwhile, if the content of such a carbonyl compound exceeds the upper limit, the reaction rate tends to be lowered.

In the foregoing, the carbonyl compound represented by the general formula (2), the acid catalyst, and the carboxylic acid having 1 to 5 carbon atoms, which are used in the method for producing a tetracarboxylic dianhydride of the present invention, have been described. Next, a heating step (step of heating the carbonyl compound in a carboxylic acid having 1 to 5 carbon atoms with an acid catalyst being used) using these is described.

Note that in the present invention, if the carbonyl compound is a compound (tetracarboxylic acid) which is represented by the general formula (2) and in which all of R⁴s in the formula are hydrogen atoms, the heating step causes a reaction (forward reaction) to proceed in which the tetracarboxylic dianhydride and water are produced from the carbonyl compound (tetracarboxylic acid) . Meanwhile, such a forward reaction and the reverse reaction in which the carbonyl compound (tetracarboxylic acid) is produced from the tetracarboxylic dianhydride and water are equilibrium reactions. In addition, in the present invention, if the carbonyl compound is a compound which is represented by the general formula (2) and in which any of R⁴s in the formula is a group other than a hydrogen atom, the heating step causes a reaction (forward reaction) to proceed in which the tetracarboxylic dianhydride, an ester compound of the lower carboxylic acid, and water are produced from the carbonyl compound and the lower carboxylic acid. Meanwhile, such a forward reaction and the reverse reaction in which the carbonyl compound and the lower carboxylic acid are produced from the carboxylic anhydride, the ester compound of the lower carboxylic acid, and water are equilibrium reactions. For this reason, in such a heating step, it is also possible to cause the reaction (forward reaction) to proceed efficiently by changing the concentrations and the like of the components in the system, as appropriate.

In addition, the conditions which may be employed in such a heating step (including conditions such as the heating temperature and an atmosphere) are not particularly limited, and if the method (conditions) makes it possible to heat the carbonyl compound in the lower carboxylic acid by using the acid catalyst, to thereby convert an ester group and/or a carboxy group (carboxylic acid group) in the carbonyl compound to an acid anhydride group, those conditions can be employed as appropriate. For example, it is possible to appropriately utilize such a condition that is employed for a known reaction which enables formation of an acid anhydride group.

In addition, in such a heating step, for the purpose of enabling heating in the lower carboxylic acid, it is preferable to first prepare a mixture of the lower carboxylic acid, the carbonyl compound, and the acid catalyst. A method for preparing such a mixture is not particularly limited. Preparation may be performed as appropriate depending on an apparatus and the like for utilization in the heating step. For example, preparation may be performed by adding (introducing) them into the same container.

In addition, in such a heating step, another solvent may be further utilized by being added to the lower carboxylic acid. Examples of the solvent (another solvent) include aromatic solvents such as benzene, toluene, xylene, and chlorobenzene; ether-based solvent such as ether, THF, and dioxane; ester-based solvents such as ethyl acetate; hydrocarbon-based solvents such as hexane, cyclohexane, heptane, and pentane; nitrile-based solvents such as acetonitrile and benzonitrile; halogen-containing solvents such as methylene chloride and chloroform; ketone-based solvents such as acetone and MEK; and amide-based solvents such as DMF, NMP, DMI, and DMAc.

In addition, the temperature condition under which the carbonyl compound represented by the general formula (2) is heated in the lower carboxylic acid is not particularly limited, and the upper limit of the heating temperature is preferably 180°C (more preferably 150°C, further preferably 140°C, and particularly preferably 130°C), while the lower limit of the heating temperature is preferably 80°C (more preferably 100°C, and further preferably 110°C). The temperature range (temperature condition) for the heating is preferably 80 to 180°C, more preferably 80 to 150°C, further preferably 100 to 140°C, and particularly preferably 110 to 130°C. If the temperature condition is lower than the lower limit, the reaction tends to proceed so insufficiently that the target tetracarboxylic dianhydride cannot be produced sufficiently efficiently. Meanwhile, if the temperature condition exceeds the upper limit, the catalytic activity tends to be lowered. In addition, the heating temperature is preferably set to a temperature lower than the boiling point of the homogeneous acid catalyst within the range of the above-described temperature condition. By setting the heating temperature as described above, the product can be obtained more efficiently.

In addition, the heating step may include the step of refluxing the mixture (mixture of the lower carboxylic acid, the carbonyl compound, and the acid catalyst) by heating from the viewpoint of producing the carboxylic anhydride more efficiently. As described above, if the heating step includes the refluxing step, it is possible to produce the carboxylic anhydride more efficiently. To be more specific, in the heating step, since the reaction has not sufficiently proceeded at the first stage of heating, by-products such as water are produced in little amount. Thus, by-products (such as water) do not have a very strong influence even if a distillation component (vapor) is not removed until the reaction proceeds to some extent (at the first stage of heating), making it possible to cause the forward reaction for producing the carboxylic dianhydride to proceed efficiently. For this reason, at the first stage of heating in particular, refluxing makes it possible to cause the forward reaction to efficiently proceed by utilizing the lower carboxylic acid more efficiently. This makes it possible to more efficiently produce the carboxylic anhydride.

Here, the progress of the forward reaction can be determined by checking e.g. the amount of by-product (for example, water and an ester compound of the lower carboxylic acid) contained in the vapor. For this reason, the refluxing step may be carried out by appropriately setting the reflux time so that the reaction proceeds efficiently while checking e.g. the amount of by-product (for example, an ester compound of the lower carboxylic acid) in the vapor. Thereafter, the distillation component removal step may be carried out while performing heating. If the distillation component removal step is carried out as described above, it is possible to remove by-products (for example, an ester compound of the lower carboxylic acid and water) from the reaction system and to cause the forward reaction to proceed more efficiently. In addition, during the distillation component removal step, if the lower carboxylic acid is reduced when the distillation component (vapor) is removed by distillation as appropriate (for example, when an ester compound of the lower carboxylic acid and water are produced as by-products, the carboxylic acid is consumed, the vapor is removed by distillation, and as a result the carboxylic acid is reduced), it is preferable to perform heating by appropriately adding (sometimes continuously adding) the lower carboxylic acid in an amount reduced by the removal by distillation. As described above, by adding (sometimes continuously adding) the lower carboxylic acid, it is possible to cause the forward reaction to proceed further efficiently if, for example, the carbonyl compound is e.g. a compound which is represented by the general formula (2) and in which any of R⁴s in the formula is a group other than a hydrogen atom.

In addition, if such a heating step includes the step of refluxing the mixture, the condition for the reflux is not particularly limited, can employ as appropriate a known condition, and can be changed as appropriate to a preferable condition depending on the type and the like of the carbonyl compound (raw material compound) to be used.

In addition, the pressure condition for heating the carbonyl compound (raw material compound) represented by the general formula (2) in the lower carboxylic acid (the pressure condition during the reaction) is not particularly limited. The condition may be normal pressure, a pressurized condition, or a reduced pressure condition, and the reaction can be caused to proceed under any one of these conditions. For this reason, when, for example, the aforementioned refluxing step is employed without particularly controlling the pressure in the heating step, for example, the reaction may be conducted under a pressurized condition by the vapor of the lower carboxylic acid serving as the solvent, or the like. In addition, the pressure condition is preferably 0.001 to 10 MPa, and further preferably 0.1 to 1.0 MPa. If the pressure condition is lower than the lower limit, the lower carboxylic acid tends to be gasified. Meanwhile, if the pressure condition exceeds the upper limit, an ester compound of the lower carboxylic acid produced by the reaction by heating tends not to evaporate, so that it is difficult to cause the forward reaction to proceed.

In addition, an atmospheric gas in which the carbonyl compound represented by the general formula (2) is heated in the lower carboxylic acid is not particularly limited, and may be, for example, air or an inert gas (nitrogen, argon, or the like) . Note that, to cause the reaction to proceed more efficiently (to shift the transesterification equilibrium reaction more to the product side) by efficiently evaporating by-products (an ester compound of the lower carboxylic acid and water) formed by the reaction, the gas (desirably, an inert gas such as nitrogen or argon) may be bubbled, or stirring may be conducted, while the gas is being passed through the gas phase portion of a reactor (reaction vessel).

In addition, the heating time for which the carbonyl compound represented by the general formula (2) is heated in the lower carboxylic acid is not particularly limited, and is preferably 0.5 to 100 hours, and more preferably 1 to 50 hours. If the heating time is less than the lower limit, the reaction tends to proceed so insufficiently that a sufficient amount of the carboxylic anhydride cannot be produced. Meanwhile, if the heating time exceeds the upper limit, the reaction tends not to proceed any further, so that the production efficiency is lowered, and the economical efficiency and the like are lowered.

In addition, when the carbonyl compound represented by the general formula (2) is heated in the lower carboxylic acid, the reaction may be caused to proceed while the lower carboxylic acid into which the carbonyl compound is introduced (more preferably the mixture of the lower carboxylic acid, the carbonyl compound, and the acid catalyst) is being stirred from the viewpoint that the reaction is caused to proceed uniformly.

Moreover, in the step of heating the carbonyl compound represented by the general formula (2) in the lower carboxylic acid (heating step), it is preferable to utilize an acetic anhydride together with the lower carboxylic acid. To be more specific, in the present invention, it is preferable to utilize an acetic anhydride during the heating. The use of acetic anhydride as described above makes it possible to form acetic acid by a reaction of water produced during the reaction with the acetic anhydride, so that water produced during the reaction can be removed efficiently, making it possible to cause the forward reaction to proceed more efficiently. In addition, when acetic anhydride is used as described above, the amount of the acetic anhydride used is not particularly limited, and is preferably such that the amount of moles of the acetic anhydride used is 4 to 100 times that of the carbonyl compound represented by the general formula (2). If the amount of the acetic anhydride used is less than the lower limit, the reaction rate tends to be lowered. Meanwhile, if the amount of the acetic anhydride exceeds the upper limit, the yield tends to decrease.

In addition, also when an acetic anhydride is utilized as described above, it is preferable to employ the conditions described in the aforementioned heating step as conditions and the like such as the temperature condition, the pressure condition, the atmospheric gas condition, and the heating time during heating. In addition, the use of acetic anhydride as described above makes it possible not only to form acetic acid by a reaction of water produced during the reaction with the acetic anhydride, so that water produced during the reaction can be removed efficiently without e.g. removing the vapor by distillation, but also to cause a reaction in which acetic acid is formed from acetic anhydride and water to produce the tetracarboxylic dianhydride (forward reaction) to proceed more efficiently. For this reason, if an acetic anhydride is utilized as described above, it is possible to cause the reaction to proceed efficiently by employing the refluxing step in the heatingstep. From such a viewpoint, if an acetic anhydride is utilized, the heating step is preferably the step of refluxing the mixture. As described above, if the reflux is carried out by utilizing an acetic anhydride, it is possible to cause the reaction to proceed sufficiently only by carrying out the refluxing step without carrying out a step such as removing the vapor by distillation or adding the lower carboxylic acid depending on the amount used, making it possible to produce the tetracarboxylic dianhydride more efficiently.

In the present invention, by carrying out the heating step described above, it is possible to efficiently obtain the tetracarboxylic dianhydride represented by the general formula (1) from the carbonyl compound represented by the general formula (2). Note that the tetracarboxylic dianhydride represented by the general formula (1) is the same as the above-described tetracarboxylic dianhydride of the present invention, and preferred ones thereof are also the same.

In the foregoing, the method for producing a tetracarboxylic dianhydride of the present invention has been described. Next, a method for producing a carbonyl compound of the present invention is described.

### [Method for Producing Carbonyl Compound]

A method for producing a carbonyl compound of the present invention comprises
reacting a norbornene -based compound represented by the following general formula (3): 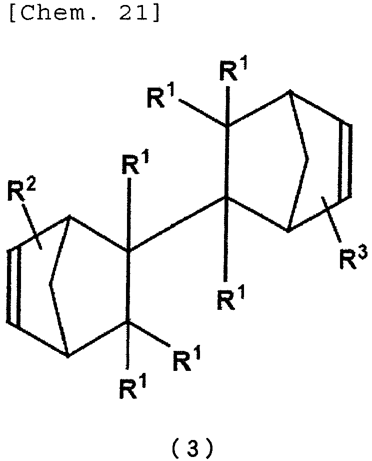
[in the formula (3), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group, and
R² and R³ each independently represent one selected from the group consisting of a hydrogen atom and alkyl groups having 1 to 10 carbon atoms] with an alcohol and carbon monoxide in the presence of a palladium catalyst and an oxidant, to thereby obtain a carbonyl compound represented by the following general formula (2):

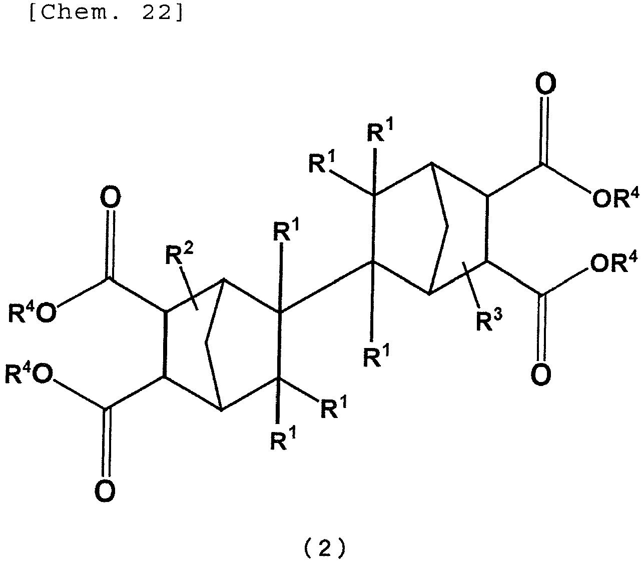

[in the formula (2), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group,
R² and R³ each independently represent one selected from the group consisting of a hydrogen atom and alkyl groups having 1 to 10 carbon atoms, and
multiple R⁴s each independently represent one selected from the group consisting of a hydrogen atom, alkyl groups having 1 to 10 carbon atoms, cycloalkyl groups having 3 to 10 carbon atoms, alkenyl groups having 2 to 10 carbon atoms, aryl groups having 6 to 20 carbon atoms, and aralkyl groups having 7 to 20 carbon atoms].

In the present invention, the production of the carbonyl compound utilizes the norbornene-based compound represented by the general formula (3) as a raw material compound. In the norbornene-based compound represented by the general formula (3), R¹s, R², and R³ in the formula (3) are the same as R¹s, R², and R³ in the general formula (1), and preferred ones thereof are also the same as R¹s, R², and R³ in the general formula (1). Note that multiple R¹s in the general formula (3) may be the same as one another or different from one another, and are preferably the same from the viewpoint of ease of purification and the like. Moreover, R² and R³ in the general formula (3) may be the same as each other or different from each other, and are preferably the same from the viewpoint of ease of purification and the like.

In addition, each of multiple R¹s, R², and R³ in the general formula (3) is particularly preferably a hydrogen atom. As described above, in the compound represented by the general formula (3), if each of the substituents represented by R¹s, R², and R³ is a hydrogen atom, the yield of the compound tends to increase. In addition, when a polyimide containing the compound as a monomer is produced, a higher heat resistance tends to be obtained.

Note that examples of the compound represented by the general formula (3) include 5,5'-bibicyclo[2.2.1]hept-2-ene (also referred to as: 5,5'-bi-2-norbornene) (CAS number: 36806-67-4), 3-methyl-3'-methylene-2,2'-bis(bicyclo[2.2.1]heptene-5 ,5'-diene) (CAS number: 5212-61-3), 5,5'-bisbicyclo[2.2.1]hept-5-ene-2,2'-diol (CAS number: 15971-85-4), and the like. The method for producing the compound represented by the general formula (3) is not particularly limited, and may employ as appropriate a known method.

In addition, in the present invention, an alcohol is used to perform reaction with the norbornene-based compound. Such an alcohol is not particularly limited, and is preferably an alcohol represented by the following general formula (7):

R^{a}OH (7)

[in the formula (7), R^{a} is an atom or a group other than a hydrogen atom which may be selected as R⁴ in the general formula (2)] from the viewpoint of ease of purification. To be more specific, it is preferable to use, as such an alcohol, alkyl alcohol having 1 to 10 carbon atoms, cycloalkyl alcohol having 3 to 10 carbon atoms, alkenyl alcohol having 2 to 10 carbon atoms, aryl alcohol having 6 to 20 carbon atoms, and aralkyl alcohol having 7 to 20 carbon atoms.

Such an alcohol includes, specifically, methanol, ethanol, butanol, allyl alcohol, cyclohexanol, benzyl alcohol, and the like, of which methanol and ethanol are more preferable and methanol is particularly preferable from the viewpoint that the purification of the obtained compound is easier. Note that one of these alcohols can be used alone, or two or more thereof can be used in combination.

In addition, in the present invention, the reaction of the alcohol (preferably R^{a}OH), carbon monoxide (CO), and the norbornene-based compound represented by the general formula (3) makes it possible to introduce an ester group (regarding such an ester group, R⁴s at positions for introduction may be the same as one another or different from one another) represented by the following general formula (8):

-COOR^{a} (8)

[in the formula (8), R^{a} is an atom or a group other than a hydrogen atom which may be selected as R⁴ in the general formula (2)] into each of the carbon atoms at the olefin portions in the norbornene-based compound represented by the general formula (3) in the presence of a palladium catalyst and an oxidant. This makes it possible to obtain the carbonyl compound represented by the general formula (2). As described above, the present invention makes it possible to obtain the carbonyl compound represented by the general formula (2) by using an alcohol (preferably R^{a}OH) and carbon monoxide (CO) and by utilizing a reaction (hereinafter, such a reaction is sometimes simply referred to as "esterification reaction") to introduce an ester group into each of the carbon atoms at the olefin portions in the carbonyl compound in the presence of a palladium catalyst and an oxidant.

The palladium catalyst used in such an esterification reaction is not particularly limited, and a known catalyst containing palladium can be used as appropriate. Examples include a palladium salt of an inorganic acid, a palladium salt of an organic acid, a catalyst whose support supports palladium, and the like. In addition, preferable examples of such a palladium catalyst include palladium chloride, palladium nitrate, palladium sulfate, palladium acetate, palladium propionate, palladium on carbon, palladium on alumina, palladium black, palladium acetate having nitrite ligand (formula: Pd₃(CH₃COO)₅(NO₂), and the like.

In addition, as such a palladium catalyst, it is preferable to use a palladium catalyst containing palladium acetate having nitrite ligand (catalyst represented by the formula: Pd₃(CH₃COO)₅(NO₂)) (hereinafter, sometimes simply referred to as "Pd₃(OAc)₅(NO₂)") from the viewpoint that it is possible to more sufficiently suppress the production of by-products and to produce the carbonyl compound represented by the general formula (2) with a higher selectivity.

In addition, in the palladium catalyst containing palladium acetate having such a nitrite ligand (Pd₃(OAc)₅(NO₂)), the content of palladium acetate having the nitrite ligand (Pd₃(OAc)₅(NO₂)) is preferably 10% by mole or more in terms of metal (relative to the total amount of palladium in the palladium catalyst). If the content ratio of palladium acetate having such a nitrite ligand is less than the lower limit, it tends to be difficult to sufficiently suppress the production of by-products and to produce the carbonyl compound represented by the general formula (2) with a sufficiently high selectivity. In addition, as the palladium catalyst, the content ratio of palladium acetate having the nitrite ligand (Pd₃(OAc)₅(NO₂)) is more preferably 30% by mole or more, further preferably 40% by mole or more, particularly preferably 50% by mole or more, and most preferably 70% by mole to 100% by mole in terms of metal (relative to the total amount of palladium in the palladium catalyst) from the viewpoint that it is possible to suppress the production of by-products at a higher level and to produce an ester compound with a higher selectivity.

In addition, if the palladium catalyst used in the esterification reaction is one which contains palladium acetate having the nitrite ligand (Pd₃(OAc)₅(NO₂)), another catalyst (another palladium catalyst component) which can be contained other than Pd₃(OAc)₅(NO₂) is not particularly limited, and it is possible to use as appropriate a known palladium-based catalyst component (for example, palladium chloride, palladium nitrate, palladium sulfate, palladium acetate, palladium propionate, palladium on carbon, palladium on alumina, palladium black, and the like) usable when reacting the olefin portions with carbon monoxide and an alcohol (during esterification).

In addition, asacomponent (palladium-based catalyst component) other than palladium acetate having the nitrite ligand which may be contained in such a palladium catalyst, it is preferable to use palladium acetate from the viewpoint of suppressing the production of by-products such as polymerization products and improving selectivity. In addition, as the palladium catalyst, it is possible to more preferably utilize a mixture catalyst of palladium acetate having the nitrite ligand (Pd₃(OAc)₅(NO₂)) and palladium acetate, and a catalyst made only of palladium acetate having the nitrite ligand (Pd₃(OAc)₅(NO₂)) from the viewpoint of suppressing the production of by-products such as polymerization products and improving selectivity.

Note that a method for producing palladium acetate having such a nitrite ligand (Pd₃(OAc)₅(NO₂)) is not particularly limited, and a known method can be used as appropriate. For example, one may use e.g. a method described in pages 1989 to 1992 of Dalton Trans (vol. 11) published on June 7, 2005 (author: Vladimir I, Bakhmutov, et al.).

In addition, the oxidant used in the esterification reaction may be one which can oxidize Pd⁰ to Pd²⁺, where Pd⁰ is created by reduction of Pd²⁺ in the palladium catalyst in the esterification reaction. Such an oxidant is not particularly limited, and examples thereof include a copper compound, an iron compound, and the like. In addition, such an oxidant includes, specifically, copper(II) chloride, copper(II) nitrate, copper(II) sulfate, copper(II) acetate, iron(II) chloride, iron(II) nitrate, iron(II) sulfate, iron(II) acetate, and the like.

Moreover, the amount of alcohol used in such an esterification reaction may be an amount which makes it possible to obtain the compound represented by the general formula (2) and is not particularly limited. For example, in order to obtain the compound represented by the general formula (2), the alcohol may be added in an amount equal to or more than theoretically necessary (theoretical value) and surplus alcohol may be used as a solvent as it is.

In addition, in the esterification reaction, it suffices that the carbon monoxide can be supplied to the reaction system in a necessary amount. For this reason, it is unnecessary to use a high-purity gas of carbon monoxide as the carbon monoxide, and a mixture gas in which a gas inert to the esterification reaction (for example, nitrogen) and carbon monoxide are mixed may be used. In addition, the pressure of such a carbon monoxide is not particularly limited, and is preferably normal pressure (approximately 0.1 MPa [1 atm]) or more and 10 MPa or less.

Moreover, the method for supplying the carbon monoxide to the reaction system is not particularly limited, and a known method can be used as appropriate. Examples of the method include a method for supplying by bubbling the carbon monoxide into a mixture liquid of the alcohol, the compound represented by the general formula (3), and the palladium catalyst, and in the case of using a reaction vessel, a method for supplying the carbon monoxide to the reaction system by introducing the carbon monoxide into the atmospheric gas in the vessel, and the like.

In addition, in the case of supplying the carbon monoxide into the mixture liquid containing the alcohol, the compound represented by the general formula (3), and the palladium catalyst, it is preferable to supply the carbon monoxide at a rate (supply rate) of 0.002 to 0.2 mole equivalents/min (more preferably 0.005 to 0.1 mole equivalents/min and further preferably 0.005 to 0.05 mole equivalents/min) to the compound represented by the general formula (3). If the supply rate of carbon monoxide is less than the lower limit, the reaction rate is slow and by-products such as polymerization products tend to be produced. Meanwhile, if the supply rate of carbon monoxide exceeds the upper limit, it tends to be difficult to control the reaction because the reaction rate improves and the reaction rapidly proceeds. Note that theoretically, 4 mole equivalents of carbon monoxide react with 1 mole of the compound represented by the general formula (3) being a raw material, which means that if the rate (supply rate) is 0.1 mole equivalents/min, for example, it takes 40 minutes (4 [mole equivalents]/0.1 [mole equivalents/min] = 40 minutes) in order to introduce theoretical value 4 mole equivalents to 1 mole of the compound of the general formula (1). In addition, as a method for supplying carbon monoxide at such a supply rate, it is preferable to employ a method for supplying by bubbling carbon monoxide into a mixture liquid of the alcohol, the compound represented by the general formula (3), and the palladium catalyst.

In addition, in the case of supplying by bubbling the carbon monoxide, a specific method for the bubbling is not particularly limited, and a known bubbling method can be employed as appropriate. For example, carbon monoxide may be supplied by bubbling into a mixture liquid using what is called a bubbling nozzle, a tube provided with numerous pores, or the like as appropriate.

Moreover, a method for controlling the supply rate of the carbon monoxide is not particularly limited, and a known control method may be employed as appropriate. For example, in the case of supplying carbon monoxide by bubbling, a method for controlling the supply rate of carbon monoxide at the rate by using a known apparatus which can supply a gas at a certain rate to the bubbling nozzle, the tube provided with numerous pores, or the like. In addition, in the case of supplying carbon monoxide by bubbling, if a reaction vessel is used, it is preferable to adjust the bubbling nozzle, the tube, or the like to near a bottom portion of the vessel. The purpose of this is to promote contact between the compound represented by the general formula (3) present at the bottom portion and carbon monoxide supplied from the bubbling nozzle or the like.

In addition, in the esterification reaction, the amount of the palladium catalyst used is preferably such an amount that the amount of moles of palladium in the palladium catalyst is 0.001 to 0.1 times (more preferably 0.001 to 0.01 times) the amount of moles of the norbornene-based compound represented by the general formula (3). If the amount of such a palladium catalyst used is less than the lower limit, the reaction rate is lowered and thus the yield tends to be lowered. Meanwhile, if the amount of such a palladium catalyst used exceeds the upper limit, it tends to be difficult to remove palladium from the product and the purity of the product tends to be lowered.

The amount of such an oxidant used is preferably 2 to 16 times (more preferably 2 to 8 times and further preferably 2 to 6 times) the amount of moles of the norbornene-based compound represented by the general formula (3). If the amount of such an oxidant used is less than the lower limit, it is impossible to sufficiently promote the oxidization reaction of palladium and as a result by-products tend to be produced in a large amount. Meanwhile, if the amount of such an oxidant used exceeds the upper limit, purification is difficult and the purity of the products tends to be lowered.

In addition, a solvent may be used in the reaction (esterification reaction) among the norbornene-based compound represented by the general formula (3), the alcohol, and carbon monoxide. Such a solvent is not particularly limited, and a known solvent usable in the esterification reaction can be used as appropriate. Examples thereof include hydrocarbon-based solvents such as n-hexane, cyclohexane, benzene, toluene, and the like.

Moreover, in the esterification reaction, since an acid is produced as a by-product from the oxidant and the like, a base may be added in order to remove such an acid. Such a base is preferably a fatty acid salt such as sodium acetate, sodium propionate, sodium butyrate, or the like. In addition, the amount of such a base used may be adjusted as appropriate depending on e.g. the amount of acid produced.

In addition, the reaction temperature condition in the esterification reaction is not particularly limited, and is preferably 0°C to 200°C {more preferably a temperature of 0°C to 100°C, further preferably about 10 to 60°C, and particularly preferably about 20 to 50°C}. If such a reaction temperature exceeds the upper limit, the yield tends to be lowered. Meanwhile, if such a reaction temperature is less than the lower limit, the reaction rate tends to be lowered. In addition, the reaction time of the esterification reaction is not particularly limited, and is preferably about 30 minutes to 24 hours.

In addition, the atmospheric gas in the esterification reaction is not particularly limited, and a gas usable in the esterification reaction can be used as appropriate. Examples include a gas inert to the esterification reaction (nitrogen, argon, or the like), carbon monoxide, and a mixture gas of carbon monoxide and another gas (nitrogen, air, oxygen, hydrogen, carbon dioxide, argon, or the like), and preferably carbon monoxide, a gas inert to the esterification reaction, and a mixture gas of carbon monoxide and a gas inert to the esterification reaction from the viewpoint that they do not affect the catalyst or the oxidant. Note that in the case of employing the method for introducing carbon monoxide by bubbling as a method for supplying carbon monoxide into the mixture liquid, the reaction may be caused to proceed such that before the reaction, the atmospheric gas is a gas inert to the esterification reaction, the reaction is started by the aforementioned bubbling, and as a result the atmospheric gas becomes a mixture gas of carbon monoxide and the gas inert to the esterification reaction, for example.

Moreover, the pressure condition in the esterification reaction (pressure condition of atmospheric gas: pressure condition of the gas in the reaction vessel if the reaction is caused to proceed in the vessel) is not particularly limited, and is preferably 0.05 MPa to 15 MPa, more preferably normal pressure (0.1 MPa [1 atm]) to 15 MPa, further preferably 0.1 MPa to 10 MPa, and particularly preferably 0.11 MPa to 5 MPa. If such a pressure condition is less than the lower limit, the reaction rate is lowered and the yield of the target product tends to be lowered. Meanwhile, if such a pressure condition exceeds the upper limit, it tends to be difficult to control the reaction because the reaction rate improves and the reaction rapidly proceeds, and the facility capable of carrying out the reaction tends to be limited.

If the esterification reaction is caused to proceed as described above, it is possible to obtain the carbonyl compound (tetraester compound) represented by the general formula (2) in which each of R⁴s in the formula (2) is a group other than a hydrogen atom. In addition, in the case of producing the carbonyl compound represented by the general formula (2) in which each of R⁴s in the formula (2) is a hydrogen atom, one may introduce a group represented by the formula: -COOR^{a} by the esterification reaction and then, in order to convert such a group to a group represented by the formula: -COOH having a hydrogen atom in place of R^{a}, carry out a hydrolysis process or a transesterification reaction with a carboxylic acid. A method for such a reaction is not particularly limited, and a known method can be employed as appropriate which makes it possible to convert the group represented by the formula -COOR^{a} (ester group) to the formula: -COOH (carboxy group).

As described above, it is possible to obtain the carbonyl compound represented by the general formula (2) having the target structure. Note that the carbonyl compound represented by the general formula (2), which is obtained as described above, is the same as the corresponding one described for the above-described carbonyl compound of the present invention, and preferred ones thereof are also the same.

In the foregoing, the method for producing a carbonyl compound of the present invention has been described. Next, a polyimide of the present invention is described.

### [Polyimide]

A polyimide of the present invention comprises a repeating unit represented by the following general formula (4) : 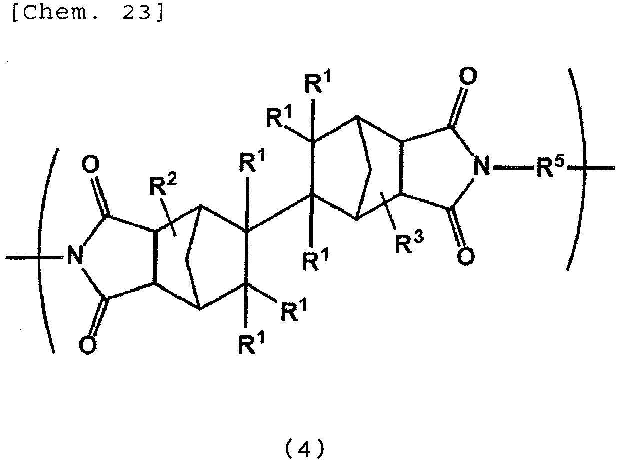
[in the formula (4), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group,
R² and R³ each independently represent one selected from the group consisting of a hydrogen atom and alkyl groups having 1 to 10 carbon atoms, and
R⁵ represents an arylene group having 6 to 40 carbon atoms].

An alkyl group which may be selected as R¹ in the general formula (4) is an alkyl group having 1 to 10 carbon atoms. If the number of carbon atoms exceeds 10, it is impossible to achieve a sufficiently high heat resistance. In addition, the number of carbon atoms of the alkyl group which may be selected as R¹ is preferably 1 to 6, more preferably 1 to 5, further preferably 1 to 4, and particularly preferably 1 to 3 from the viewpoint that the purification is easier. In addition, the alkyl group which maybe selected as R¹ maybe linear or branched. Furthermore, such an alkyl group is more preferably a methyl group and an ethyl group from the viewpoint of ease of purification.

In addition, of multiple R¹s in the general formula (4), two R¹s connected to a common carbon atom may together form a methylidene group (= CH₂). To be more specific, two R¹s connected to a common carbon atom in the general formula (4) may together be connected to the carbon atom (of the carbon atoms forming a norbornane ring structure, the carbon atom connected with two R¹s) as a methylidene group (methylene group) via double bond.

Multiple R¹s in the general formula (4) are each independently more preferably a hydrogen atom, a methyl group, an ethyl group, an n-propyl group, or an isopropyl group, and particularly preferably a hydrogen atom or a methyl group, for example, from the viewpoint that a higher heat resistance can be obtained when a polyimide is produced, that the raw material is more readily available (prepared), and that the purification is easier. In addition, multiple R¹s in the formula may be the same as one another or different from one another, and are preferably the same from the viewpoints of ease of purification and the like.

R² and R³ in the general formula (4) are each independently one selected from the group consisting of a hydrogen atom and alkyl groups having 1 to 10 carbon atoms . If the number of carbon atoms of such an alkyl group which may be selected as R² and R³ exceeds 10, the heat resistance of a polyimide is lowered. In addition, such an alkyl group which may be selected as R² and R³ is preferably 1 to 6, more preferably 1 to 5, further preferably 1 to 4, and particularly preferably 1 to 3 from the viewpoint that a higher heat resistance can be obtained. In addition, such an alkyl group which may be selected as R² and R³ may be linear or branched.

R² and R³ in the general formula (4) are each independently more preferably a hydrogen atom, a methyl group, an ethyl group, an n-propyl group, and an isopropyl group, and particularly preferably a hydrogen atom and a methyl group, for example, from the viewpoint that a higher heat resistance can be obtained when a polyimide is produced, that the raw material is readily available, and that the purification is easier. In addition, the R² and R³ in the formula (4) may be the same as each other or different from each other, and are preferably the same from the viewpoints of ease of purification and the like.

In addition, each of multiple R¹s, R², and R³ in the general formula (4) is particularly preferably a hydrogen atom. As described above, in the repeating unit represented by the general formula (4), if each of the substituents represented by R¹s, R², and R³ is a hydrogen atom, the yield of the compound tends to increase and a higher heat resistance tends to be obtained.

In addition, an arylene group which may be selected as R⁵ in the general formula (4) is an arylene group having 6 to 40 carbon atoms. In addition, the number of carbon atoms of such an arylene group is preferably 6 to 30 and more preferably 12 to 20. If the number of carbon atoms is less than the lower limit, the heat resistance of a polyimide tends to be lowered. Meanwhile, if the number of carbon atoms exceeds the upper limit, the solubility of the obtained polyimide to the solvent is lowered, and formability to a film and the like tend to be lowered.

In addition, R⁵ in the general formula (4) is preferably at least one of the groups represented by the following general formulae (9) to (12): 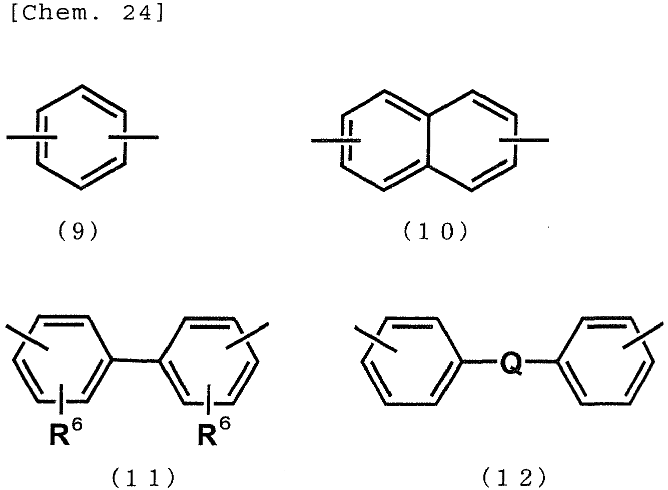 [in the formula (11), R⁶ is one selected form the group consisting of a hydrogen atom, a fluorine atom, methyl groups, ethyl groups, and trifluoromethyl groups, and in the formula (12), Q represents one selected from the group consisting of the groups represented by the formulae : -C₆H₄-, -CONH-C₆H₄-NHCO-, -NHCO-C₆H₄-CONH-, -O-C₆H₄-CO-C₆H₄-O-, -OCO-C₆H₄-COO-, -OCO-C₆H₄-C₆H₄-COO-, -OCO-, -NC₆H₅-, -CO-C₄H₈N₂-CO-, -C₁₃H₁₀-, -(CH₂)₅-, -O-, -S-, -CO-, -CONH-, -SO₂-, -C(CF₃)₂-, -C(CH₃)₂-, -CH₂-, -(CH₂)₂-, -(CH₂)₃-, -(CH₂)₄, -(CH₂)₅-, -O-C₆H₄-C(CH₃)₂-C₆H₄-O-, -O-C₆H₄-SO₂-C₆H₄-O-, -C(CH₃)₂-C₆H₄-C(CH₃)₂-, -O-C₆H₄-C₆H₄-O-, and -O-C₆H₄-O-] from the viewpoint of the balance between the heat resistance and the solubility.

R⁶ in the general formula (11) is more preferably a hydrogen atom, a fluorine atom, a methyl group, or an ethyl group, and particularly preferably a hydrogen atom from the viewpoint of the heat resistance of the obtained polyimide.

In addition, Q in the general formula (12) is preferably a group represented by the formula: -CONH-, -O-C₆H₄-O-, -O-C₆H₄-C₆H₄-O-, -O-, -C(CH₃)₂-, -CH₂-, or -O-C₆H₄-C(CH₃)₂-C₆H₄-O-, particularly preferably a group represented by the formula: -CONH-, -O-C₆H₄-C(CH₃)₂-C₆H₄-O-, or -O-, and most preferably a group represented by the formula: -O-C₆H₄-C(CH₃)₂-C₆H₄-O- or -O- from the viewpoint of the balance between the heat resistance and the solubility.

In addition, a group, which may be selected as R⁵ in the general formula (4) and is represented by the general formulae (9) to (12), is more preferably a group represented by the general formula (11) or (12) from the viewpoint that they can provide a sufficiently high temperature of glass transition temperature and a sufficiently low value of linear expansion coefficient, and thus the balance between these characteristics are improved and higher heat resistance can be obtained. Among these, R⁵ is preferably at least one of a group represented by the general formula (11) and a group represented by the general formula (12) in which the Q is represented by -CONH-, -COO-, -CO-, or -C₆H₄- (more preferably a group represented by -CONH- or -COO- and particularly preferably a group represented by -CONH-) from the viewpoint that they can provide a lower linear expansion coefficient and an even higher heat resistance. Furthermore, R⁵ in the general formula (4) is preferably a group represented by the general formula (9) or a group represented by the general formula (12) in which the Q is at least one of the groups represented by -O-, -S-, -CH₂-, and -O-C₆H₄-O- (more preferably one of the groups represented by -O- and -CH₂- and further preferably a group represented by -O-) from the viewpoint that they can provide the obtained polyimide with a higher flexible property (flexibility).

Moreover, the polyimide preferably contains several types (two or more types) of repeating unit with different types of R⁵ in the general formula (4) from the viewpoint that they have a sufficiently high glass transition temperature, a sufficiently low linear expansion coefficient, and a sufficient flexible property (flexibility) in balance at an even higher level. In addition, from the same viewpoint, since a higher effect can be obtained, the polyimide containing the several types of repeating unit is more preferably one which contains a repeating unit (A) which is represented by the general formula (4) in which R⁵ in the formula is one selected from the group consisting of a group represented by the general formula (11) ; and a group represented by the general formula (12) in which the Q is one of the groups represented by -CONH-, -COO-, -CO-, and -C₆H₄- (more preferably the groups represented by -CONH- and -COO- and particularly preferably the group represented by -CONH-) and a repeating unit (B) which is represented by the general formula (4) in which R⁵ in the formula is one selected from the group consisting of a group represented by the general formula (9); and a group represented by the general formula (12) in which the Q is one of the groups represented by -O-, -S-, -CH₂-, and -O-C₆H₄-O- (more preferably one of the groups represented by -O- and -CH₂- and further preferably the group represented by -O-).

In addition, such a repeating unit (B) is more preferably one in which R⁵ in the general formula (4) is a group represented by the general formula (12) and Q in the formula (12) is one of the groups represented by -O-, -CH₂-, and -O-C₆H₄-O- (more preferably one of the groups represented by -O- and -CH₂- and further preferably the group represented by -O-) from the viewpoint of how easily the monomer is obtained in the production.

If such repeating units (A) and (B) are contained, the content ratio between the repeating unit (A) and the repeating unit (B) is preferably 9:1 to 6:4 (more preferably 8:2 to 7:3) in a mole ratio ((A) : (B)). If the content ratio of the repeating unit (A) is less than the lower limit, it tends to be difficult to obtain a polyimide with a lower linear expansion coefficient. Meanwhile, if the content ratio of the repeating unit (A) exceeds the upper limit, the flexible property of the obtained board film tends to be lowered. In addition, if the repeating units (A) and (B) are contained, it is preferable that the configurations of the substituents other than R⁴ in the general formula (1) be the same from the viewpoint that it is possible to prepare the polyimide more efficiently.

In addition, the polyimide is preferably one which mainly contains the repeating unit represented by the general formula (4) (more preferably, the content of the repeating unit represented by the general formula (4) is 50 to 100% by mole (further preferably 70 to 100% by mole, particularly preferably 80 to 100% by mole, and most preferably 90 to 100% by mole) relative to the entire repeating unit). Note that the polyimide may contain a different repeating unit as long as the effects of the present invention are not impaired. The different repeating unit is not particularly limited, and includes a known repeating unit and the like which can be used as a repeating unit of the polyimide.

In addition, a preferable example of the different repeating unit is a repeating unit represented by the following general formula (13): 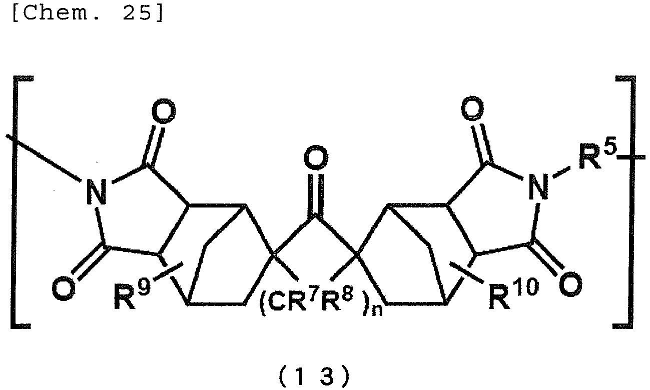 [in the formula (13), R⁷, R⁸, R⁹, R¹⁰ each independently represent one selected from the group consisting of a hydrogen atom, alkyl groups having 1 to 10 carbon atoms, and a fluorine atom, R⁵ represents an aryl group having 6 to 40 carbon atoms, and n represents an integer from 0 to 12].

Note that R⁷, R⁸, R⁹, and R¹⁰ in the general formula (13) each independently are one selected from the group consisting of a hydrogen atom, alkyl groups having 1 to 10 carbon atoms, and a fluorine atom. The alkyl groups having 1 to 10 carbon atoms which may be selected as R⁷, R⁸, R⁹, and R¹⁰ are the same as the alkyl group which may be selected as R¹ in the general formula (4) (preferred ones thereof are also the same) . R⁷, R⁸, R⁹, and R¹⁰ in the formula (13) are each independently more preferably a hydrogen atom or an alkyl group having 1 to 10 carbon atoms from the viewpoint of the adhesiveness of the obtained polyimide. Among these, R⁷, R⁸, R⁹, and R¹⁰ in the formula (13) are each independently more preferably a hydrogen atom, a methyl group, an ethyl group, an n-propyl group, or an isopropyl group, and particularly preferably a hydrogen atom or a methyl group from the viewpoint that the raw material is readily available and that the purification is easier. In addition, R⁷, R⁸, R⁹, and R¹⁰ in the formula are particularly preferably the same as one another from the viewpoint of ease of purification and the like. In addition, R⁵ in the general formula (13) is the same as R⁵ in the general formula (4) (preferred ones thereof are also the same). In addition, n in the general formula (13) represents an integer from 0 to 12. If the value of n exceeds the upper limit, purification is difficult. In addition, the upper limit value of the numerical range of n in the general formula (13) is more preferably 5 and particularly preferably 3 from the viewpoint that the purification is easier. In addition, the lower limit value of the numerical range of n in the general formula (13) is more preferably 1 and particularly preferably 2 from the viewpoint of stability of the raw material compound. As described above, n in the general formula (13) is particularly preferably an integer of 2 or 3.

In addition, the different repeating unit (including the repeating unit represented by the general formula (13) and the like) can be easily introduced by e.g. using the tetracarboxylic dianhydride represented by the general formula (1) as well as another tetracarboxylic dianhydride in the production of the polyimide. In this case, the different repeating unit is derived from the other tetracarboxylic dianhydride other than the tetracarboxylic dianhydride represented by the general formula (1). Note that the other tetracarboxylic dianhydride is described later. In addition, if the different repeating unit is contained, the mole ratio ([repeating unit represented by the general formula (4)]:[different repeating unit]) may be 99.9:0.1 to 0.1:99.9. Furthermore, in the case where the different repeating unit is contained, the content ratio between the repeating unit represented by the general formula (4) and the different repeating unit is preferably 9:1 to 5:5 (more preferably 9:1 to 7:3) in a mole ratio ([repeating unit represented by the general formula (4)]: [different repeating unit]) from the viewpoint of the balance between the heat resistance and the transparency.

The polyimide of the present invention is one having a 5% weight-loss temperature of preferably 350°C or higher, and more preferably 450 to 550 °C. If the 5% weight-loss temperature is lower than the lower limit, it tends to be difficult to achieve a sufficient heat resistance. Meanwhile, if the 5% weight-loss temperature exceeds the upper limit, it tends to be difficult to produce a polyimide having such a property. Note that the 5% weight-loss temperature can be determined by measuring the temperature at which the weight loss of a sample used reaches 5% when the sample is gradually heated from room temperature (25 °C) under a nitrogen gas atmosphere in a nitrogen gas stream. Note that in the measurement, the mass of the sample used is preferably 1.0 mg to 10 mg (more preferably 1.5 mg to 4.0 mg). If the mass of the sample is within the aforementioned range, it is possible to measure the same value for the same polyimide even if the measurement is carried out for a different mass of the sample.

In addition, the polyimide is one having a glass transition temperature (Tg) of preferably 200°C or higher, more preferably 230 to 500°C, and particularly preferably 250 to 500°C. If the glass transition temperature (Tg) is lower than the lower limit, it tends to be difficult to achieve a sufficient heat resistance. Meanwhile, if the glass transition temperature (Tg) exceeds the upper limit, it tends to be difficult to produce a polyimide having such a property. Note that the glass transition temperature (Tg) can be determined by using a thermomechanical analyzer (manufactured by Rigaku Corporation under the trade name of "TMA 8310").

Moreover, the polyimide has a softening temperature of preferably 300°C or higher, and more preferably 350 to 550°C. If the softening temperature is lower than the lower limit, it tends to be difficult to achieve a sufficient heat resistance. Meanwhile, if the softening temperature exceeds the upper limit, it tends to be difficult to produce a polyimide having such a property. Note that the softening temperature can be determined by using a thermomechanical analyzer (manufactured by Rigaku Corporation under the trade name of "TMA 8310") in a penetration mode. In addition, in the measurement, since the size (length, width, thickness, and the like) of the sample does not affect the measurement value, the size of the sample may be adjusted as appropriate to a size attachable to a jig of the thermomechanical analyzer to be used (manufactured by Rigaku Corporation under the trade name of "TMA 8310") . Note that the softening temperature and the glass transition temperature can be measured at the same time under the same conditions employed by using the thermomechanical analyzer (manufactured by Rigaku Corporation under the trade name of "TMA 8310").

In addition, the polyimide has a thermal decomposition temperature (Td) of preferably 400°C or higher, and more preferably 450 to 600°C. If the thermal decomposition temperature (Td) is lower than the lower limit, it tends to be difficult to achieve a sufficient heat resistance. Meanwhile, if the thermal decomposition temperature (Td) exceeds the upper limit, it tends to be difficult to produce a polyimide having such a property. Note that the thermal decomposition temperature (Td) can be determined by measuring the temperature at an intersection of tangent lines drawn to decomposition curves before and after thermal decomposition using a TG/DTA220 thermogravimetric analyzer (manufactured by SII NanoTechnology Inc.) under a nitrogen atmosphere under a condition of a rate of temperature rise of 10°C /min. Note that in the measurement, the mass of the sample used is preferably 1.0 to 10 mg (more preferably 5 mg to 10 mg). If the mass of the sample is within the aforementioned range, it is possible to measure the same value for the same polyimide even if the measurement is carried out for a different mass of the sample. Furthermore, the thermal decomposition temperature (Td) can be measured at the same time under the same conditions (under a nitrogen atmosphere under a condition of a rate of temperature rise of 10°C /min) by using the same apparatus as in the measurement of the 5% weight-loss temperature.

Moreover, the polyimide preferably has a number average molecular weight (Mn) of 1000 to 1000000 in terms of polystyrene. If the number average molecular weight is less than the lower limit, it tends to be difficult to achieve a sufficient heat resistance. Meanwhile, if the number average molecular weight exceeds the upper limit, the polyimide tends to be difficult to process.

In addition, the polyimide preferably has a weight average molecular weight (Mw) of 1000 to 5000000 in terms of polystyrene. If the weight average molecular weight is less than the lower limit, it tends to be difficult to achieve a sufficient heat resistance. Meanwhile, if the weight average molecular weight exceeds the upper limit, the polyimide tends to be difficult to process.

Moreover, the polyimide preferably has a molecular weight distribution (Mw/Mn) of 1.1 to 5.0. If the molecular weight distribution is less than the lower limit, the polyimide tends to be difficult to produce. Meanwhile, if the molecular weight distribution exceeds the upper limit, it tends to be difficult to produce a uniform film. Note that the molecular weights (Mw and Mn) of the polyimide and the distribution (Mw/Mn) of the molecular weights can be determined by using a gel permeation chromatograph as a measuring apparatus and converting the measured data to that of polystyrene.

Note that when the molecular weight of a polyimide is difficult to measure, a polyimide may be selected and used according to the application or the like by estimating the molecular weight and the like on the basis of the viscosity of a polyamic acid used for producing the polyimide.

In addition, the polyimide is one having a total luminous transmittance of more preferably 80% or higher (further preferably 85% or higher and particularly preferably 87% or higher) from the viewpoint of obtaining a higher transparency. In addition, the polyimide is one having a haze (turbidity) of more preferably 5 to 0 (further preferably 4 to 0 and particularly preferably 3 to 0) from the viewpoint of obtaining a higher transparency. Furthermore, the polyimide is one having a yellowness index (YI) of more preferably 5 to 0 (further preferably 4 to 0 and particularly preferably 3 to 0) from the viewpoint of obtaining a higher transparency. Such a total luminous transmittance, haze (turbidity), and yellowness index (YI) can be achieved easily by selecting, as appropriate, the type of the polyimide and the like. Note that values measured as follows can be employed as the total luminous transmittance and the haze (turbidity). Specifically, a measuring apparatus manufactured by NIPPON DENSHOKU INDUSTRIES CO., LTD. under the trade name of "Haze Meter NDH-5000" was used to measure a film comprising a polyimide having a thickness of 5 to 20 µm prepared as a sample for measurement. In addition, a value measured as follows can be employed as the yellowness index. Specifically, a measuring apparatus manufactured by NIPPON DENSHOKU INDUSTRIES CO., LTD. under the trade name of "Spectrophotometer SD6000" was used to measure a film comprising a polyimide having a thickness of 5 to 20 µm prepared as a sample for measurement. Note that it is possible to measure the same value for each of the total luminous transmittance, the haze (turbidity), and the yellowness index (YI) for the same polyimide if the film comprises a polyimide having a thickness of 5 to 20 µm because the thickness is sufficiently thin and thus does not affect the measurement values. For this reason, in the measurement of the total luminous transmittance, the haze (turbidity), and the yellowness index (YI), it suffices to use a film having a thickness within the range. In addition, the sizes in length and width of the measurement sample maybe such sizes that can be disposed at a measurement position of the measuring apparatus. The sizes in length and width may be changed as appropriate. Note that the total luminous transmittance is obtained by performing measurement in accordance with JIS K7361-1 (issued in 1997), the haze (turbidity) is obtained by performing measurement in accordance with JIS K7136 (issued in 2000), and the yellowness index (YI) is obtained by performing measurement in accordance with ASTM E313-05(issued in 2005).

In addition, the polyimide has a linear expansion coefficient of preferably 0 to 100 ppm/K, and more preferably 10 to 80 ppm/K. If the linear expansion coefficient exceeds the upper limit, the polyimide tends to be easily peeled off because of thermal history when a composite material is formed by combining the polyimide with a metal or an inorganic material having a linear expansion coefficient in a range from 5 to 20 ppm/K. Meanwhile, if the linear expansion coefficient is lower than the lower limit, the solubility tends to be lowered, and film characteristics tend to deteriorate.

A method for measuring the linear expansion coefficient of the polyimide is as follows. Specifically, a measurement sample is prepared by forming a polyimide film in a size of 20 mm in length and 5 mm in width (although the thickness of the film is not particularly limited because it does not affect the measurement value, it is preferably 10 to 30 pm). Then, the change in length of the sample in the longitudinal direction is measured from 50°C to 200°C by using a thermomechanical analyzer (manufactured by Rigaku Corporation under the trade name of "TMA 8310") as a measuring apparatus and by employing a condition of a rate of temperature rise of 5°C/minute under a nitrogen atmosphere in a tensile mode (49 mN) . The average value of changes in length per Celsius degree is determined for the temperature range from 100°C to 200°C. The thus obtained value is employed as the linear expansion coefficient.

The shape of the polyimide is not particularly limited, and may be, for example, the shape of a film, the form of powder, moreover the shape of a pellet by extrusion molding, and the like. As described above, the polyimide of the present invention can be formed into various shapes as appropriate by a known method such as in the shape of a film and in the shape of a pellet by extrusion molding.

In addition, a polyimide having the repeating unit represented by the general formula (4) has a sufficiently high transparency and a higher heat resistance . Note that, although it is not exactly clear why the polyimide comprising a repeating unit represented by the general formula (4) exhibits a sufficiently high heat resistance, the present inventors speculate that the sufficiently high heat resistance is achieved because the repeating unit has a rigid alicyclic structure, and hence the polyimide has a chemically sufficiently stable structure.

In addition, such a polyimide is especially useful as a material for producing films for flexible wiring boards, heat-resistant insulating tapes, enameled wires, protective coating agents for semiconductors, liquid crystal orientation films, transparent electrically conductive films for organic ELs, flexible substrate films, flexible transparent electrically conductive films, transparent electrically conductive films for organic thin film-type solar cells, transparent electrically conductive films for dye-sensitized-type solar cells, flexible gas barrier films, films for touch panels, seamless polyimide belts (so-called transfer belts) for copiers, transparent electrode substrates (transparent electrode substrates for organic ELs, transparent electrode substrates for solar cells, transparent electrode substrates for electronic paper, and the like), interlayer insulating films, sensor substrates, substrates for image sensors, reflectors for light-emitting diodes (LED) (reflectors for LED illumination: LED reflectors), covers for LED illumination, covers for LED reflector illumination, coverlay films, highly extensible composite substrates, resists for semiconductors, lithium-ion batteries, substrates for organic memories, substrates for organic transistors, substrates for organic semiconductors, color filter base materials, and the like because the polyimide has a sufficiently high transparency and a higher heat resistance. In addition, if the sufficiently high heat resistance is used, the polyimide can be used as appropriate in, for example, parts for automobiles, aerospace parts, bearing parts, sealing materials, bearing parts, gearwheels, valve parts, and the like in addition to the aforementioned applications by e.g. forming the shape into the form of powder and into various formed bodies.

Note that the polyimide can have a lower value of loss tangent (tanδ) depending on the structure. For this reason, it is possible to sufficiently reduce transmission loss when the polyimide of the present invention is utilized in, for example, interlayer insulating film material for semiconductor, a board film for a flexible printed circuit board (FPC), and the like. For this reason, the polyimide of the present invention can also be preferably utilized in a high frequency band material (for example, a large-scale integration (LSI), an electronic circuit, and the like) and the like.

Although a method for producing a polyimide of the present invention is not particularly limited, it is preferable to employ the method for producing a polyimide of the present invention because the polyimide of the present invention can be produced more efficiently. Note that the method for producing a polyimide of the present invention is described later.

In the foregoing, the polyimide of the present invention has been described. Next, a polyamic acid of the present invention is described.

### [Polyamic Acid]

A polyamic acid of the present invention comprises a repeating unit represented by the following general formula (5): 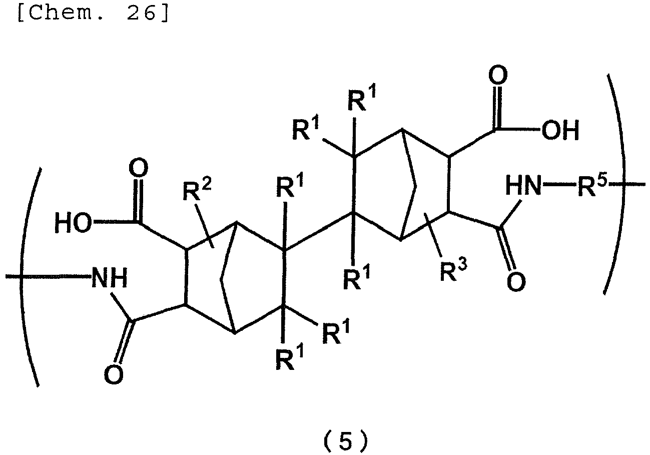
[in the formula (5), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group,
R² and R³ each independently represent one selected from the group consisting of a hydrogen atom and alkyl groups having 1 to 10 carbon atoms, and
R⁵ represents an arylene group having 6 to 40 carbon atoms].

The polyamic acid is one which can be preferably utilized when the polyimide of the present invention is produced (one which can be obtained as a reaction intermediate (precursor) when the polyimide of the present invention is produced) . R¹s, R², R³, and R⁵ in the general formula (5) are the same as R¹s , R², R³, and R⁵ in the general formula (4), and preferred ones thereof are also the same as R¹s , R², R³, and R⁵ in the general formula (4). Note that multiple R¹s in the general formula (5) may be the same as one another or different from one another, and are preferably the same from the viewpoint of ease of purification and the like. Moreover, R² and R³ in the general formula (5) maybe the same as each other or different from each other, and are preferably the same from the viewpoint of ease of purification and the like.

The polyamic acid has an intrinsic viscosity [η] of preferably 0.05 to 3.0 dL/g, and more preferably 0.1 to 2.0 dL/g. If the intrinsic viscosity [η] is lower than 0.05 dL/g, the obtained film tends to be brittle, when a polyimide in the form of a film is produced by using this polyamic acid. Meanwhile, if the intrinsic viscosity [η] exceeds 3.0 dL/g, the viscosity is so high that the processability decreases, for example, making it difficult to form a uniform film when a film is produced. In addition, the intrinsic viscosity [η] can be determined as follows. Specifically, first, N,N-dimethylacetamide is used as a solvent, and the polyamic acid is dissolved in the N,N-dimethylacetamide at a concentration of 0.5 g/dL to obtain a measurement sample (solution). Next, by using the measurement sample, the viscosity of the measurement sample is measured by using a kinematic viscometer under a temperature condition of 30°C, and the determined value is employed as the intrinsic viscosity [η] . Note that, as the kinematic viscometer, an automatic viscometer manufactured by RIGO CO. , LTD. (trade name: "VMC-252") is used.

In addition, the polyamic acid is more preferably mainly comprising a repeating unit represented by the general formula (5) (further preferably having a content of the repeating unit represented by the general formula (5) of 50 to 100% by mole relative to all the repeating units). Note that the polyamic acid may comprise one or more other repeating units within a range not impairing an effect of the present invention. The other repeating units are not particularly limited, and a known repeating unit usable in polyamic acids can be used as appropriate. Examples thereof include repeating units derived from other tetracarboxylic dianhydrides other than the tetracarboxylic dianhydride represented by the general formula (1), and the like. Note that the other tetracarboxylic dianhydrides are described later. In addition, a preferable example of the different repeating unit which may be contained in the polyamic acid is a repeating unit represented by the following general formula (14) : 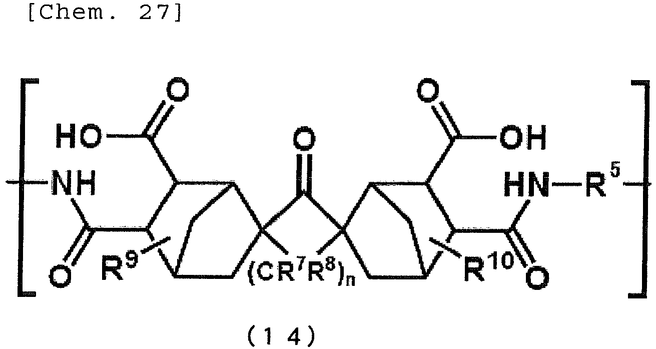 [R⁵, R⁷, R⁸, R⁹, R¹⁰, and n in the general formula (14) have the same meanings as those of R⁵, R⁷, R⁸, R⁹, R¹⁰, and n in the general formula (13), respectively (preferred ones thereof are also the same)].

In addition, if the different repeating unit is contained, the mole ratio ([repeating unit represented by the general formula (5)]: [different repeating unit]) may be 99.9:0.1 to 0.1:99.9. Furthermore, in the case where the different repeating unit is contained, the content ratio between the repeating unit represented by the general formula (5) and the different repeating unit is preferably 9:1 to 5:5 (more preferably 9:1 to 7:3) in a mole ratio ([repeating unit represented by the general formula (5)]:[different repeating unit]) from the viewpoint of the heat resistance and the transparency of the obtained polyimide.

Although a method for producing a polyamic acid of the present invention is not particularly limited, it is preferable to employ the method for producing a polyamic acid of the present invention to be described later because the polyamic acid of the present invention can be produced more efficiently.

### [Method for Producing Polyamic Acid]

A method for producing a polyamic acid of the present invention comprises
reacting a tetracarboxylic dianhydride represented by the following general formula (1): 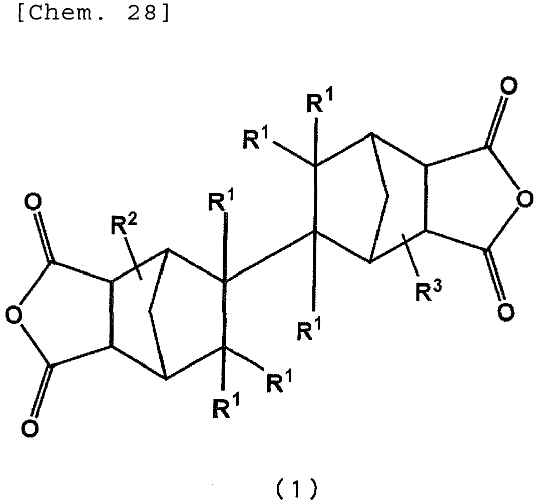
[in the formula (1), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group, and
R² and R³ each independently represent one selected from the group consisting of a hydrogen atom and alkyl groups having 1 to 10 carbon atoms] with an aromatic diamine represented by the following general formula (6):
[Chem. 29]

H₂N-R⁵-NH₂ (6)

[in the formula (6), R⁵ represents an arylene group having 6 to 40 carbon atoms] in the presence of an organic solvent, to thereby obtain a polyamic acid having a repeating unit represented by the following general formula (5): 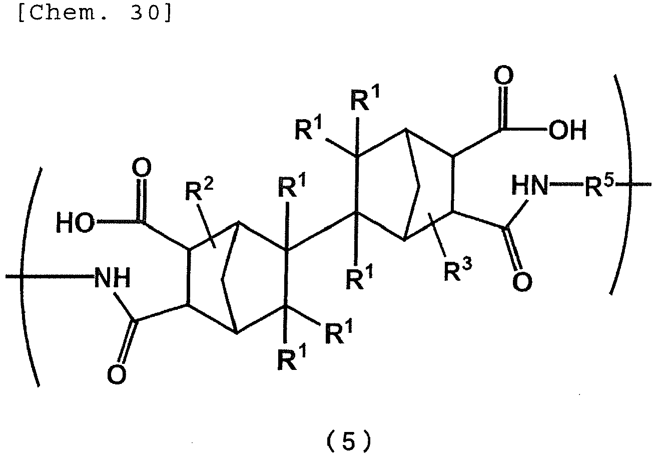
[in the formula (5), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group,
R² and R³ each independently represent one selected from the group consisting of a hydrogen atom and alkyl groups having 1 to 10 carbon atoms, and
R⁵ represents an arylene group having 6 to 40 carbon atoms]. To be more specific, the method for producing a polyamic acid of the present invention is a method comprising the step of reacting the tetracarboxylic dianhydride represented by the general formula (1) with the aromatic diamine represented by the general formula (6) in the presence of an organic solvent, to thereby obtain the repeating unit represented by the general formula (5) .

The tetracarboxylic dianhydride represented by the general formula (1) and used in the method for producing a polyamic acid is the same as the above-described tetracarboxylic dianhydride of the present invention (R¹s, R², and R³ in the tetracarboxylic dianhydride represented by the general formula (1) are the same as those described for the above-described tetracarboxylic dianhydride of the present invention, and preferred ones thereof are also the same). Note that R¹s, R², and R³ in the general formula (1) used for the reaction are preferably the same as R¹s, R², and R³ in the general formula (4). Note that, as a method for producing the tetracarboxylic dianhydride represented by the general formula (1), the above-described method for producing a tetracarboxylic dianhydride of the present invention can be used preferably. In addition, one of the tetracarboxylic dianhydrides represented by the general formula (1) may be used alone, or two or more thereof may be used in combination.

In addition, in the aromatic diamine represented by the general formula (6), R⁵ in the formula (6) is the same as R⁵ in the general formula (4) described for the above-described polyimide of the present invention, and preferred ones thereof are also the same as those of R⁵ in the general formula (4).

Examples of the aromatic diamine represented by the general formula (6) include 4,4'-diaminodiphenylmethane, 3,3'-diaminodiphenylmethane, 4,4'-diaminodiphenylethane, 3,3'-diaminodiphenylethane, 3,3'-diaminobiphenyl, 4,4'-diaminodiphenyl ether, 3,3'-diaminodiphenyl ether, 2,2-bis(4-aminophenoxyphenyl)propane, 1,3-bis(4-aminophenoxy)benzene, 1,3-bis(3-aminophenoxy)benzene, bis[4-(4-aminophenoxy)phenyl]sulfone, bis[4-(3-aminophenoxy)phenyl]sulfone, 2,2'-bis(trifluoromethyl)-4,4'-diaminobiphenyl, 3,4'-diaminodiphenyl ether, 4,4'-diaminobenzophenone, 3,3'-diaminobenzophenone, 9,9-bis(4-aminophenyl)fluorene, p-diaminobenzene, m-diaminobenzene, o-diaminobenzene, 4,4'-diaminobiphenyl, 4,4'-diamino-2,2'-dimethylbiphenyl, 4,4'-diamino-3,3'-dimethylbiphenyl, 3,3'-diaminobiphenyl, 2,2'-diaminobiphenyl, 3,4'-diaminobiphenyl, 2,6-diaminonaphthalene, 1,4-diaminonaphthalene, 1,5-diaminonaphthalene, 4,4'-[1,3-phenylenebis(1-methyl-ethylidene)]bisaniline, 4,4'-[1,4-phenylenebis(1-methyl-ethylidene)]bisaniline, 3,3'-diaminodiphenyl sulfone, 4,4'-diaminodiphenyl sulfone, 4,4'-diaminodiphenyl sulfide, 1,4-bis(4-aminophenoxy)benzene, 4,4'-bis(4-aminophenoxy)biphenyl, 4,4'-diaminobenzanilide, o-tolidine sulfone, 2,3,5,6-tetramethyl-1,4-phenylenediamine, 3,3',5,5'-tetramethylbenzidine, 1,5-bis(4-aminophenoxy)pentane, 4,4'-diaminotriphenylamine, 1,4-bis(4-aminobenzoyl) piperazine, 2-phenoxy-1,4-diaminobenzene, bis(4-aminophenyl)terephthalate, N¹,N⁴-bis(4-aminophenyl)terephthalamide, bis(4-aminophenyl)[1,1'-biphenyl]-4,4'-dicarboxylate, 4,4''-diamino-p-terphenyl, N,N'-bis(4-aminobenzoyl)-p-phenylenediamine, bis[4-(4-aminophenoxy)phenyl]ketone, 4-aminophenyl-4-aminobenzoate, [1,1'-biphenyl]-4,4'-diyl bis(4-aminobenzoate), and the like.

A method for producing the aromatic diamine is not particularly limited, and a known method can be employed, as appropriate. In addition, as the aromatic diamine, commercially available one may be used, as appropriate. In addition, one of these aromatic diamines represented by the general formula (6) may be used alone, or two or more thereof may be used in combination.

In addition, the organic solvent used in the step is preferably an organic solvent capable of dissolving both the tetracarboxylic dianhydride represented by the general formula (1) and the aromatic diamine represented by the general formula (6). Examples of the organic solvent include aprotic polar solvents such as N-methyl-2-pyrrolidone, N,N-dimethylacetamide, N,N-dimethylformamide, dimethyl sulfoxide, γ-butyrolactone, propylene carbonate, tetramethylurea, 1,3-dimethyl-2-imidazolidinone, hexamethylphosphoric triamide, and pyridine; phenol-based solvents such as m-cresol, xylenol, phenol, and halogenated phenols; ether-based solvents such as tetrahydrofuran, dioxane, Cellosolve, and glyme; aromatic solvents such as benzene, toluene, and xylene; and the like. One of these organic solvents may be used alone, or two or more thereof may be used as a mixture.

In addition, the ratio of the tetracarboxylic dianhydride represented by the general formula (1) and the aromatic diamine represented by the general formula (6) used is not particularly limited, and the acid anhydride groups of the tetracarboxylic dianhydride represented by the general formula (1) are pref erably 0.2 to 2 equivalents, and more preferably 0.3 to 1.2 equivalents per equivalent of the amino groups of the aromatic diamine represented by the general formula (6). If the preferred ratio of the tetracarboxylic dianhydride represented by the general formula (1) and the aromatic diamine represented by the general formula (6) used is lower than the lower limit, the polymerization reaction tends not to proceed efficiently, so that a polyamic acid having a high molecular weight cannot be obtained. Meanwhile, if the ratio exceeds the upper limit, a polyamic acid having a high molecular weight tends not to be obtained as in the above described case.

Moreover, the amount of the organic solvent used is preferably such that the total amount of the tetracarboxylic dianhydride represented by the general formula (1) and the aromatic diamine represented by the general formula (6) can be 1 to 80% by mass (more preferably 5 to 50% by mass) relative to the total amount of the reaction solution. If the amount of the organic solvent used is less than the lower limit, the polyamic acid tends not to be obtained efficiently. Meanwhile, if the amount of the organic solvent used exceeds the upper limit, the viscosity tends to increase, making the stirring difficult, so that a polymer having a high molecular weight cannot be obtained .

In addition, when the tetracarboxylic dianhydride represented by the general formula (1) and the aromatic diamine represented by the general formula (6) are reacted with each other, a basic compound may be further added to the organic solvent, from the viewpoints of improving the reaction rate and obtaining a polyamic acid with a high degree of polymerization. The basic compound is not particularly limited, and examples thereof include triethylamine, tetrabutylamine, tetrahexylamine, 1,8-diazabicyclo[5.4.0]-undecene-7, pyridine, isoquinoline, α-picoline, and the like. In addition, the amount of the basic compound used is preferably 0.001 to 10 equivalents, and more preferably 0.01 to 0.1 equivalents per equivalent of the tetracarboxylic dianhydride represented by the general formula (1). If the amount of the basic compound used is less than the lower limit, the effect achieved by the addition tends not to be exhibited. Meanwhile, if the amount of the basic compound used exceeds the upper limit, the basic compound tends to cause color development or the like.

In addition, the reaction temperature for the reaction between the tetracarboxylic dianhydride represented by the general formula (1) and the aromatic diamine represented by the general formula (6) is not particularly limited, as long as the temperature is adjusted, as appropriate, to a temperature at which these compounds can be reacted with each other. The reaction temperature is preferably 15 to 100°C. In addition, a method for reacting the tetracarboxylic dianhydride represented by the general formula (1) with the aromatic diamine represented by the general formula (6) is not particularly limited, and it is possible to use, as appropriate, a method by which a polymerization reaction between a tetracarboxylic dianhydride and an aromatic diamine can be conducted. For example, a method may be employed in which the aromatic diamine is dissolved in the solvent under atmospheric pressure in an inert atmosphere of nitrogen, helium, argon, or the like, then the tetracarboxylic dianhydride represented by the general formula (1) is added at the reaction temperature, and then the reaction is allowed to proceed for 10 to 48 hours. If the reaction temperature or the reaction time is lower or less than the lower limit, it tends to be difficult to cause the reaction to proceed sufficiently. Meanwhile, if the reaction temperature or the reaction time exceeds the upper limit, the possibility of contamination with a substance (such as oxygen) that degrades the polymerization product tends to increase, so that the molecular weight decreases .

By reacting the tetracarboxylic dianhydride represented by the general formula (1) with the aromatic diamine represented by the general formula (6) in the presence of the organic solvent as described above, a polyamic acid comprising a repeating unit represented by the general formula (5) can be obtained. The thus obtained polyamic acid comprising a repeating unit represented by the general formula (5) is the same as that described for the above-described polyamic acid of the present invention (note that R¹s, R², R³, and R⁵ in the general formula (5) are the same as R¹s, R², R³, and R⁵ described for the above-described polyamic acid of the present invention, and preferred ones thereof are also the same). For this reason, the method for producing a polyamic acid of the present invention can be used preferably as a method for producing the above-described polyamic acid of the present invention.

In addition, to obtain a polyamic acid comprising another repeating unit together with the repeating unit represented by the general formula (5) by the present invention, a method may be employed in which another tetracarboxylic dianhydride is used together with the tetracarboxylic dianhydride represented by the general formula (1) in the production of the polyamic acid, and these tetracarboxylic dianhydrides are reacted with the aromatic diamine.

Examples of the other tetracarboxylic dianhydride other than the tetracarboxylic dianhydride represented by the general formula (1) include aliphatic or alicyclic tetracarboxylic dianhydrides such as butanetetracarboxylic dianhydride, 1,2,3,4-cyclobutanetetracarboxylic dianhydride, 1,2,3,4-cyclopentanetetracarboxylic dianhydride, 2,3,5-tricarboxycyclopentylacetic dianhydride, 3,5,6-tricarboxynorbornane-2-acetic dianhydride, 2,3,4,5-tetrahydrofurantetracarboxylic dianhydride, 1,3,3a,4,5,9b-hexahydro-5-(tetrahydro-2,5-dioxo-3-fura nyl)-naphtho[1,2-c]-furan-1,3-dione, 1,3,3a,4,5,9b-hexahydro-5-methyl-5-(tetrahydro-2,5-dio xo-3-furanyl)-naphtho[1,2-c]-furan-1,3-dione, 1,3,3a,4,5,9b-hexahydro-8-methyl-5-(tetrahydro-2,5-dio xo-3-furanyl)-naphtho[1,2-c]-furan-1,3-dione, 5-(2,5-dioxotetrahydrofural)-3-methyl-3-cyclohexene-1, 2-dicarboxylic dianhydride, bicyclo[2,2,2]-oct-7-ene-2,3,5,6-tetracarboxylic dianhydride, norbornane-2-spiro-α-cyclopentanone-α'-spiro-2''-norbo rnane-5,5'',6,6''-tetracarboxylic dianhydride, and 5,5'-(1,4-phenylene)bis(hexahydro-4,7-methanoisobenzof uran-1,3-dione); aromatic tetracarboxylic dianhydrides such as pyromellitic dianhydride, 3,3',4,4'-benzophenonetetracarboxylic dianhydride, 3,3',4,4'-biphenyl sulfonetetracarboxylic dianhydride, 1,4,5,8-naphthalenetetracarboxylic dianhydride, 2,3,6,7-naphthalenetetracarboxylic dianhydride, 3,3',4,4'-biphenyl ether tetracarboxylic dianhydride, 3,3',4,4'-dimethyldiphenylsilanetetracarboxylic dianhydride, 3,3',4,4'-tetraphenylsilanetetracarboxylic dianhydride, 1,2,3,4-furantetracarboxylic dianhydride, 4,4'-bis(3,4-dicarboxyphenoxy)diphenyl sulfide dianhydride, 4,4'-bis(3,4-dicarboxyphenoxy)diphenyl sulfone dianhydride, 4,4'-bis(3,4-dicarboxyphenoxy)diphenylpropane dianhydride, 3,3',4,4'-perfluoroisopropylidenediphthalic dianhydride, 4,4'-(2,2-hexafluoroisopropylidene)diphthalic dianhydride, 3,3',4,4'-biphenyltetracarboxylic dianhydride, 2,3,3',4'-biphenyltetracarboxylic dianhydride, bis(phthalic acid)phenylphosphine oxide dianhydride, p-phenylene-bis(triphenylphthalic) dianhydride, m-phenylene-bis(triphenylphthalic) dianhydride, bis(triphenylphthalic acid)-4,4'-diphenyl ether dianhydride, and bis(triphenylphthalic acid)-4,4'-diphenylmethane dianhydride; and the like.

In addition, as the other tetracarboxylic dianhydride other than the tetracarboxylic dianhydride represented by the general formula (1), it is preferable to use the compound represented by following general formula (15): 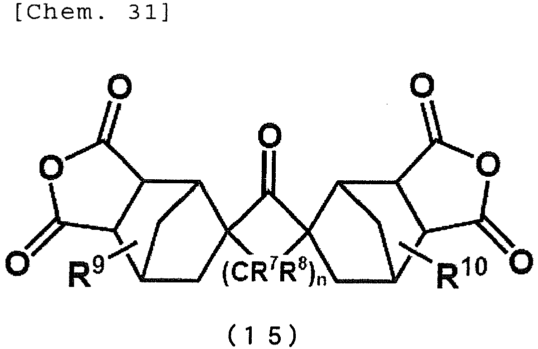 [R⁷, R⁸, R⁹, R¹⁰, and n in the general formula (15) have the same meanings as those of R⁷, R⁸, R⁹, R¹⁰, and n in the general formula (13), respectively (preferred ones thereof are also the same)] in the case of, for example, introducing the repeating unit represented by the general formula (14) into the polyamic acid as the other repeating unit (in the case of introducing the repeating unit represented by the general formula (13) into the polyimide obtained by using the polyamic acid) . Note that a method for producing the other tetracarboxylic dianhydride represented by the general formula (15) is not particularly limited, and a known method (for example, a method described in International Publication No. WO2011/099517 and a method described in International Publication No. WO2011/099518) can be employed as appropriate.

The compounds and the like listed as examples above can be used as appropriate as the other tetracarboxylic dianhydride other than the tetracarboxylic dianhydride represented by the general formula (1). Note that in a case where an aromatic tetracarboxylic acid is used as the other tetracarboxylic dianhydride, the amount of the aromatic tetracarboxylic acid used is preferably changed, as appropriate, within a range where the obtained polyimide can have a sufficient transparency from the viewpoint of preventing color development due to intramolecular CT.

In addition, when the other tetracarboxylic dianhydride as described above is used in the production of the polyimide, the total amount of acid anhydride groups in the tetracarboxylic dianhydride represented by the general formula (1) and the other tetracarboxylic dianhydride (all tetracarboxylic dianhydrides present in the reaction system) is preferably 0.2 to 2 equivalents (more preferably 0.3 to 1.2 equivalents) per equivalent of the amino groups of the aromatic diamine represented by the general formula (6).

In addition, if the other tetracarboxylic dianhydride is used together with the tetracarboxylic dianhydride represented by the general formula (1), the ratio of these used is preferably 9:1 to 5:5 (more preferably 9:1 to 7:3) in a mole ratio ([tetracarboxylic dianhydride represented by the general formula (1)]:[other tetracarboxylic dianhydride]). If the ratio of the tetracarboxylic dianhydride represented by the general formula (1) used (mole ratio) is less than the lower limit, the heat resistance of the obtained polyimide tends to be lowered. Meanwhile, if the ratio of the tetracarboxylic dianhydride represented by the general formula (1) used exceeds upper limit, an effect of physical property of the polyimide tends not to be exhibited when the other tetracarboxylic dianhydride is used.

In addition, when the polyamic acid comprising the repeating unit represented by the general formula (5) is isolated from the organic solvent after the above-described step is conducted, a method for the isolation is not particularly limited, and a known method capable of isolating a polyamic acid can be employed, as appropriate. For example, a method in which the polyamic acid is isolated as a reprecipitation product or the like may be employed.

In the foregoing, the method for producing a polyamic acid of the present invention has been described. Next, a polyamic acid solution of the present invention is described.

### [Polyamic Acid Solution]

A polyamic acid solution of the present invention comprises: the above -described polyamic acid of the present invention; and an organic solvent. As the organic solvent used for the polyamic acid solution (resin solution: varnish), the same organic solvents used in the above-described method for producing a polyamic acid of the present invention can be used preferably. For this reason, the polyamic acid solution of the present invention may be prepared by conducting the above-described method for producing a polyamic acid of the present invention and employing the reaction liquid obtained after the reaction directly as the polyamic acid solution. In other words, the polyamic acid solution of the present invention may be produced by preparing a polyamic acid comprising a repeating unit represented by the general formula (5) by reacting the tetracarboxylic dianhydride represented by the general formula (1) with the aromatic diamine represented by the general formula (6) in the presence of the organic solvent, to thereby obtain a solution comprising the polyamic acid and the organic solvent.

The content of the polyamic acid in the polyamic acid solution is not particularly limited, and is preferably 1 to 80% by mass, and more preferably 5 to 50% by mass. If the content is less than the lower limit, it tends to be difficult to produce a polyimide film. Meanwhile, if the content exceeds the upper limit, it likewise tends to be difficult to produce a polyimide film. Note that the polyamic acid solution can be used preferably for producing the above-described polyimide of the present invention and can be used preferably for producing polyimides with various shapes. For example, a polyimide with the shape of a film is easily produced by applying the polyamic acid solution onto various substrates and curing the resultant by imidization.

### [Method for Producing Polyimide]

A method for producing a polyimide of the present invention comprises
imidizing a polyamic acid having a repeating unit represented by the following general formula (5): 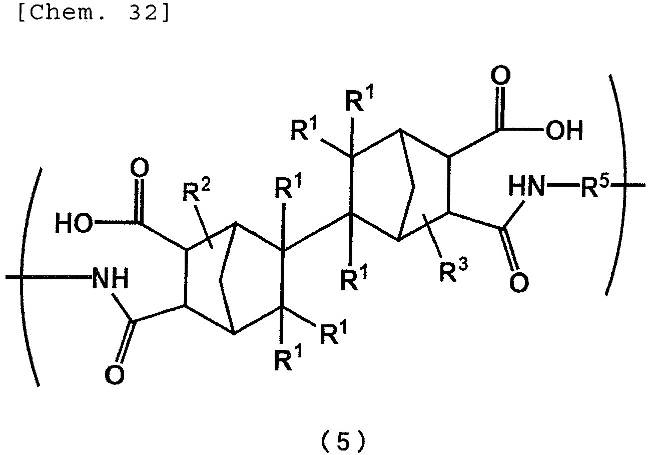
[in the formula (5), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group,
R² and R³ each independently represent one selected from the group consisting of a hydrogen atom and alkyl groups having 1 to 10 carbon atoms, and
R⁵ represents an arylene group having 6 to 40 carbon atoms], to thereby obtain a polyimide having a repeating unit represented by the following general formula (4):

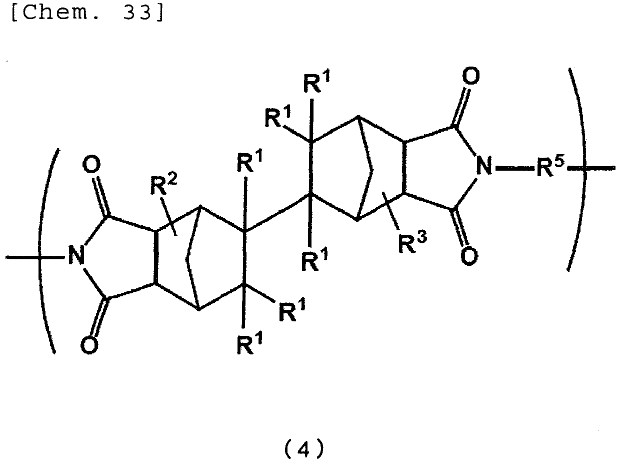

[in the formula (4), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group,
R² and R³ each independently represent one selected from the group consisting of a hydrogen atom and alkyl groups having 1 to 10 carbon atoms, and
R⁵ represents an arylene group having 6 to 40 carbon atoms].

The polyamic acid comprising a repeating unit represented by the general formula (5) and used in the method for producing a polyimide is the same as that described for the above-described polyamic acid of the present invention (preferred ones thereof are also the same).

A method for the imidization is not particularly limited, as long as imidization of a polyamic acid can be performed by the method. A known method can be employed, as appropriate, and it is preferable to employ, for example, a method in which the imidization is conducted by subjecting the polyamic acid comprising a repeating unit represented by the general formula (5) to a heat treatment under a temperature condition of 60 to 400°C (more preferably 150 to 350°C) or a method in which the imidization is conducted by using a so-called "imidization agent."

In the case where the method in which the imidization is conducted by a heat treatment is employed, if the heating temperature is lower than 60 °C, the progress of the reaction tends to be slow, while if the heating temperature exceeds the upper limit, color development, molecular weight reduction due to thermal decomposition, or the like tends to occur. Meanwhile, when the method in which the imidization is conducted by a heat treatment is employed, the reaction time (heating time) is preferably 0.5 to 5 hours. If the reaction time is less than the lower limit, it tends to be difficult to conduct the imidization sufficiently, while if the reaction time exceeds the upper limit, color development, molecular weight reduction due to thermal decomposition, or the like tends to occur.

On the other hand, when the method in which the imidization is conducted by utilizing a so-called "imidization agent" is employed, it is preferable to perform the imidization of the polyamic acid comprising a repeating unit represented by the general formula (5) in a solvent in the presence of an imidization agent. As the solvent, the same solvent as the organic solvent used for the above-described method for producing a polyimidic acid of the present invention can be used preferably.

As the imidization agent, a known imidization agent can be used, as appropriate, and examples thereof include acid anhydrides such as acetic anhydride, propionic anhydride, and trifluoroacetic anhydride; tertiary amines such as pyridine, collidine, lutidine, triethylamine, and N-methylpiperidine; and the like. In addition, when the imidization is conducted by adding the imidization agent, the reaction temperature for the imidization is preferably -40°C to 200°C, more preferably 0 to 180°C, and further preferably 30 to 150°C. Meanwhile, the reaction time is preferably 0.1 to 48 hours. If the reaction temperature or time is lower or less than the lower limit, it tends to be difficult to conduct the imidization sufficiently. Meanwhile, if the reaction temperature or time exceeds the upper limit, the possibility of contamination with a substance (oxygen or the like) that degrades the polymerization product tends to increase, so that the molecular weight decreases. In addition, the amount of the imidization agent used is not particularly limited, and may be several millimoles to several moles (preferably about 0.05 to 4.0 moles) per mole of the repeating unit represented by the general formula (5) in the polyamic acid.

In addition, for the chemical imidization using the imidization agent, it is preferable to employ, as the imidization agent, a combination (combined use) of a condensation agent (such as a carboxylic anhydride, a carbodiimide, an acid azide, or an active ester-forming agent) with a reaction accelerator (such as tertiary amine) . The combined use of a condensation agent (a so-called dehydration condensation agent such as a carboxylic anhydride, a carbodiimide, an acid azide, or an active ester-forming agent) with a reaction accelerator (such as tertiary amine) as described above makes it possible to perform the imidization by more efficient dehydration ring-closure of the polyamic acid under a low-temperature condition (more preferably under a temperature condition of about 100°C or below).

The condensation agent is not particularly limited, and examples thereof include carboxylic anhydrides such as acetic anhydride, propionic anhydride, and trifluoroacetic anhydride; carbodiimides such as N,N'-dicyclohexylcarbodiimide (DCC); acid azides such as diphenylphosphoryl azide (DPPA); active ester-forming agents such as Castro's reagent; and dehydration condensation agents such as 2-chloro-4,6-dimethoxytriazine (CDMT). Of these condensation agents, acetic anhydride, propionic anhydride, and trifluoroacetic anhydride are preferable, acetic anhydride and propionic anhydride are more preferable, and acetic anhydride is further preferable from the viewpoints of reactivity, availability, and practicability. One of these condensation agents may be used alone or two or more thereof may be used in combination.

In addition, the reaction accelerator may be any, as long as the reaction accelerator can be used for conversion of the polyamic acid to a polyimide by condensation, and a known compound can be used, as appropriate. The reaction accelerator can also function as an acid scavenger that captures the acid by-produced during the reaction. For this reason, the use of the reaction accelerator accelerates the reaction and suppresses the reverse reaction due to the by-produced acid, so that the reaction can be caused to proceed more efficiently. The reaction accelerator is not particularly limited, and is more preferably one also having a function of an acid scavenger. Examples of the reaction accelerator include tertiary amines such as triethylamine, diisopropylethylamine, N-methylpiperidine, pyridine, collidine, lutidine, 2-hydroxypyridine, 4-dimethylaminopyridine (DMAP), 1,4-diazabicyclo[2.2.2]octane (DABCO), diazabicyclononene (DBN), and diazabicycloundecene (DBU), and the like. Of these reaction accelerators, triethylamine, diisopropylethylamine, N-methylpiperidine, and pyridine are preferable, triethylamine, pyridine, and N-methylpiperidine are more preferable, and triethylamine and N-methylpiperidine are further preferable from the viewpoints of reactivity, availability, and practicability. One of those reaction accelerators may be used alone or two or more thereof may be used in combination.

In addition, for the chemical imidization using the imidization agent, the chemical imidization may be conducted by, for example, adding a catalytic amount of a reaction accelerator (such as DMAP) and an azeotropic dehydration agent (such as benzene, toluene, or xylene), and removing water produced when the polyamic acid is converted to the imide by azeotropic dehydration. For the chemical imidization (imidization using an imidization agent), the azeotropic dehydration agent may be used, as appropriate, together with the reaction accelerator as described above. The azeotropic dehydration agent is not particularly limited, and an azeotropic dehydration agent may be selected from known azeotropic dehydration agents and used, as appropriate, according to the type of the material used for the reaction and the like.

In addition, in the method for producing a polyimide of the present invention, the polyamic acid comprising a repeating unit represented by the general formula (5) is preferably obtained by the above-described method for producing a polyamic acid of the present invention.

Moreover, the method for producing a polyimide of the present invention preferably further comprises the step of reacting the tetracarboxylic dianhydride represented by the general formula (1) with the aromatic diamine represented by the general formula (6) in the presence of an organic solvent, to thereby obtain a polyamic acid comprising a repeating unit represented by the general formula (5). Note that this step is the same as the step of obtaining the polyamic acid described for the above-described method for producing a polyamic acid of the present invention (the organic solvent, the tetracarboxylic dianhydride, and the aromatic diamine used, the reaction conditions, and the like are also the same as those described for the above-described method for producing a polyamic acid of the present invention). As described above, the method for producing a polyimide of the present invention preferably comprises: a step (I) of reacting a tetracarboxylic dianhydride represented by the general formula (1) with an aromatic diamine represented by the general formula (6) in the presence of an organic solvent, to thereby obtain a polyamic acid comprising a repeating unit represented by the general formula (5) ; and a step (II) of imidizing the polyamic acid, to thereby obtain a polyimide comprising a repeating unit represented by the general formula (4). When the method for producing a polyimide of the present invention comprises the steps (I) and (II) as described above, a polyimide can be produced more efficiently by the continuous steps.

Note that when the method in which the imidization is conducted by a heat treatment is employed for the imidization in a case where the method comprising these steps (I) and (II) is used, the following method may be employed. Specifically, after the step (I) is conducted, the reaction liquid obtained by reacting the tetracarboxylic dianhydride represented by the general formula (1) with the aromatic diamine represented by the general formula (10) in the organic solvent (the reaction liquid comprising the polyamic acid comprising a repeating unit represented by the general formula (5)) is directly used without isolation of the polyamic acid comprising a repeating unit represented by the general formula (5). The solvent is removed from the reaction liquid by subjecting the reaction liquid to a treatment (solvent removal treatment) for removing the solvent by evaporation, and then the imidization is conducted by the heat treatment. This treatment for removing the solvent by evaporation makes it possible to perform a heat treatment or the like after the polyamic acid comprising a repeating unit represented by the general formula (5) is isolated in the form of a film or the like. A temperature condition in the method for the treatment for removing the solvent by evaporation (solvent removal treatment) is preferably 0 to 180°C, and more preferably 30 to 150°C. If the temperature condition in the drying treatment is lower than the lower limit, it tends to be difficult to sufficiently remove the solvent by evaporation. Meanwhile, if the temperature condition exceeds the upper limit, the solvent tends to boil, resulting in formation of a film containing air bubbles or voids. In this case, for example, When a polyimide in the form of a film is produced, the obtained reaction liquid may be directly applied onto a base material (for example, a glass plate), followed by the treatment for removing the solvent by evaporation and the heat treatment. Thus, a polyimide in the form of a film can be produced by a simple method. Note that a method for applying the reaction liquid is not particularly limited, and a known method (such as a cast method) can be employed, as appropriate. In addition, when the polyamic acid comprising a repeating unit represented by the general formula (5) is used after isolation from the reaction liquid, a method for the isolation is not particularly limited, and a known method capable of isolating a polyamic acid can be employed, as appropriate. For example, a method may be employed in which the polyamic acid is isolated as a reprecipitation product.

In addition, suppose a case where the method comprising the steps (I) and (II) is used and the method in which the imidization is conducted by using the "imidization agent" is employed. In such a case, since the method in which the imidization is conducted by using the "imidization agent" is basically a method in which the imidization is preferably performed in a solvent (more preferably the organic solvent described for the above-described method for producing a polyamic acid of the present invention), it is preferable to employ, for example, a method in which the reaction liquid (the reaction liquid comprising the polyamic acid comprising a repeating unit represented by the general formula (5)) obtained by reacting the tetracarboxylic dianhydride represented by the general formula (1) with the aromatic diamine represented by the general formula (6) in the organic solvent is directly used (the reaction liquid is directly used without isolation of the polyamic acid comprising a repeating unit represented by the general formula (5) from the reaction liquid after the step (I) is conducted), and the imidization is conducted by adding the imidization agent to the reaction liquid.

In addition, the solvent used when the method in which the imidization is conducted by using the "imidization agent (preferably a combination of a condensation agent with a reaction accelerator)" is employed is preferably the organic solvent (the solvent used for the polymerization: the polymerization solvent) described for the above-described method for producing a polyamic acid of the present invention, from the viewpoints as described above (the viewpoints of directly using the reaction liquid and the like). Especially, the solvent is preferably N,N-dimethylacetamide, N-methyl-2-pyrrolidone, N,N-dimethylformamide, 1,3-dimethyl-2-imidazolidinone, dimethyl sulfoxide, or the like, and more preferably N,N-dimethylacetamide. One of these organic solvents (polymerization solvents) may be used alone, or two or more thereof may be used as a mixture.

In addition, when the reaction liquid (the reaction liquid comprising the polyamic acid comprising the repeating unit represented by the general formula (4)) is directly used and the imidization is conducted by adding the imidization agent to the reaction liquid, the organic solvent (polymerization solvent) is preferably one having a boiling point of 20°C or higher, and preferably one having a boiling point of 50 to 250°C. If the boiling point is lower than the lower limit, polymerization under atmospheric pressure at normal temperature tends to be difficult, so that the polymerization has to be carried out under a special condition, namely, under pressure or under a low temperature. Meanwhile, if the boiling point exceeds the upper limit, such an organic solvent tends to be difficult to remove in a step of drying an obtained polyimide in the form of powder after washing, so that the solvent remains in the obtained polyimide.

In addition, when a combination of a condensation agent with a reaction accelerator is used as the imidization agent, a temperature condition for the chemical imidization is preferably -40°C to 200°C, more preferably -20°C to 150°C, further preferably 0 to 150°C, and particularly preferably 50 to 100°C. If the temperature exceeds the upper limit, an undesirable side reaction tends to proceed, so that the polyimide cannot be obtained. Meanwhile, if the temperature is lower than the lower limit, the reaction rate of the chemical imidization tends to be lowered, or the reaction itself tends not to proceed, so that the polyimide cannot be obtained. As described above, when the condensation agent and the reaction accelerator are used in combination, the imidization can be performed in a relatively low-temperature region of from -40°C to 200°C. Hence, it is possible to reduce the load on the environment, and the method can be advantageous in terms of the manufacturing process.

In addition, when a combination of a condensation agent with a reaction accelerator is used as the imidization agent, the amount of the condensation agent used is not particularly limited, and is preferably 0.05 to 10. 0 moles, and further preferably 1 to 5 moles per mole of the repeating unit in the polyamic acid. If the amount of the condensation agent (imidization agent) used is less than the lower limit, the reaction rate of the chemical imidization tends to be lowered or the reaction itself tends not to proceed sufficiently, so that the polyimide cannot be obtained sufficiently. Meanwhile, if the amount of the condensation agent exceeds the upper limit, the polyimide tends not to be obtained efficiently, for example, because an undesirable side reaction proceeds.

In addition, when a combination of a condensation agent with a reaction accelerator is used as the imidization agent, the amount of the reaction accelerator used is not particularly limited, and is preferably 0.05 to 4.0 moles, and further preferably 0.5 to 2 moles per mole of the repeating unit in the polyamic acid. If the amount of the reaction accelerator used is less than the lower limit, the reaction rate of the chemical imidization tends to be lowered or the reaction itself tends not to proceed sufficiently, so that the polyimide cannot be obtained sufficiently. Meanwhile, if the amount of the reaction accelerator used exceeds the upper limit, the polyimide tends not to be obtained efficiently, for example, because an undesirable side reaction proceeds.

In addition, an atmosphere condition for the chemical imidization is preferably an inert gas atmosphere of nitrogen gas or the like or a vacuum condition, from the viewpoint of preventing color development due to oxygen in the air and molecular weight reduction due to water vapor in the air. In addition, a pressure condition for the chemical imidization is not particularly limited, and is preferably 0.01 hPa to 1 MPa, and more preferably 0.1 hPa to 0.3 MPa. If the pressure is lower than the lower limit, the solvent, the condensation agent, and the reaction accelerator tend to be gasified, so that the stoichiometry is disturbed and an adverse influence is exerted on the reaction, making it difficult to cause the reaction to proceed sufficiently. Meanwhile, if the pressure exceeds the upper limit, an undesirable side reaction tends to proceed, or the solubility of the polyamic acid tends to decease, so that precipitation occurs before the imidization.

In addition, when the polyimide obtained by the present invention is obtained in the form of being dissolved in the organic solvent (polymerization solvent), the polyimide may be precipitated by concentration, as appropriate, or the polyimide may be precipitated by dropwise addition to a solvent in which the polyimide is insoluble, and then collected. Note that it is also possible to obtain the polyimide as a precipitate by dropwise addition to a solvent in which the polyimide is insoluble as described above. In this case, it is also possible to obtain a polyimide in the form of powder (particles).

Note that, to obtain a polyimide comprising another repeating unit together with the repeating unit represented by the general formula (4) by the present invention, the polyamic acid used for producing the polyimide may be one comprising another repeating unit together with the repeating unit represented by the general formula (5). For example, when the above-described method for producing a polyimide of the present invention comprises the steps (I) and (II), another tetracarboxylic dianhydride is used together with the tetracarboxylic dianhydride represented by the general formula (1) and these tetracarboxylic dianhydrides are reacted with the aromatic diamine in the step (I), and then the step (II) may be performed. As the other tetracarboxylic dianhydride other than the tetracarboxylic dianhydride represented by the general formula (1), it is possible to use, as appropriate, the same tetracarboxylic dianhydride as described for the above-described method for producing a polyamic acid of the present invention.

The thus obtained polyimide represented by the general formula (4) is the same as that described for the above-described polyimide of the present invention (R¹s, R², R³, and R⁵ in the formula (4) are also the same as R¹s, R², R³, and R⁵ described for the above-described polyimide of the present invention, and preferred ones thereof are also the same). For this reason, the method for producing a polyimide of the present invention is a method also preferably usable as a method for producing the above-described polyimide of the present invention.

Note that the polyimide obtained as described above forms a first-order structure in which an acid dianhydride having a rigid alicyclic structure and an aromatic diamine are bonded to each other, and thus electron transfer between molecular chains of the obtained polyimide is less likely to occur. Consequently, the polyimide has an extremely high transparency and the heat resistance with the softening temperature as a reference is higher. For this reason, the polyimide of the present invention can be used in various applications as appropriate, and can be preferably used as a material for producing, for example, films for flexible wiring boards, heat-resistant insulating tapes, enameled wires, protective coating agents for semiconductors, liquid crystal orientation films, transparent electrically conductive films for organic electroluminescence (organic EL), flexible substrate films, flexible transparent electrically conductive films, transparent electrically conductive films for organic thin film-type solar cells, transparent electrically conductive films for dye-sensitized-type solar cells, flexible gas barrier films, films for touch panels, seamless polyimide belts (so-called transfer belts) for copiers, transparent electrode substrates (transparent electrode substrates for organic ELs, transparent electrode substrates for solar cells, transparent electrode substrates for electronic paper, and the like), interlayer insulating films, sensor substrates, substrates for image sensors, reflectors for light-emitting diodes (LED) (reflectors for LED illumination: LED reflectors), covers for LED illumination, covers for LED reflector illumination, coverlay films, highly extensible composite substrates, resists for semiconductors, lithium-ion batteries, substrates for organic memories, substrates for organic transistors, substrates for organic semiconductors, color filter base materials, and the like.

In the foregoing, the method for producing a polyimide of the present invention has been described. In the following description, a film of the present invention is described.

### [Film]

A film of the present invention comprises the above-described polyimide of the present invention. The film (polyimide film) of the present invention may be a film comprising a polyimide described as the above-described polyimide of the present invention. For this reason, the film of the present invention may be, for example, one obtained by using the above -described polyamic acid solution of the present invention.

The form of the polyimide film is not particularly limited, as long as the form is in a film shape, and the polyimide film may be designed to have any of various shapes (a circular disc shape, a cylindrical shape (a film processed into a tube), or the like), as appropriate. When the polyimide film is produced by using the polyimide solution, it is also possible to change the design of the polyimide film more easily.

In addition, the thickness of the film of the present invention is not particularly limited, and preferably 1 to 500 µm, and more preferably 10 to 200 µm. If the thickness is less than the lower limit, the strength tends to decrease, making the film difficult to handle. Meanwhile, if the thickness exceeds the upper limit, it tends to be necessary to perform application multiple times, or the process tends to be complicated.

Moreover, the film of the present invention has a sufficiently high transparency and a higher heat resistance, and hence can be used, as appropriate, in applications such as, for example, films for flexible wiring boards, films used for liquid crystal orientation, transparent electrically conductive films for organic ELs, films for organic EL lighting devices, flexible substrate films, substrate films for flexible organic ELs, flexible transparent electrically conductive films, transparent electrically conductive films, transparent electrically conductive films for organic thin film-type solar cells, transparent electrically conductive films for dye-sensitized-type solar cells, flexible gas barrier films, films for touch panels, front films for flexible displays, back films for flexible displays, polyimide belts, coating agents, barrier films, sealants, interlayer insulating materials, passivation films, TAB (Tape Automated Bonding) tapes, optical waveguides, color filter base materials, semiconductor coating agents, heat-resistant insulating tapes, enameled wires, and the like.

### [Examples]

Hereinafter, the present invention will be described more specifically on the basis of Examples; however, the present invention is not limited to Examples below.

First, chemical formulae of the aromatic diamines used in Examples and Comparative Examples, and abbreviated names (abbreviations) of the compounds are shown below.

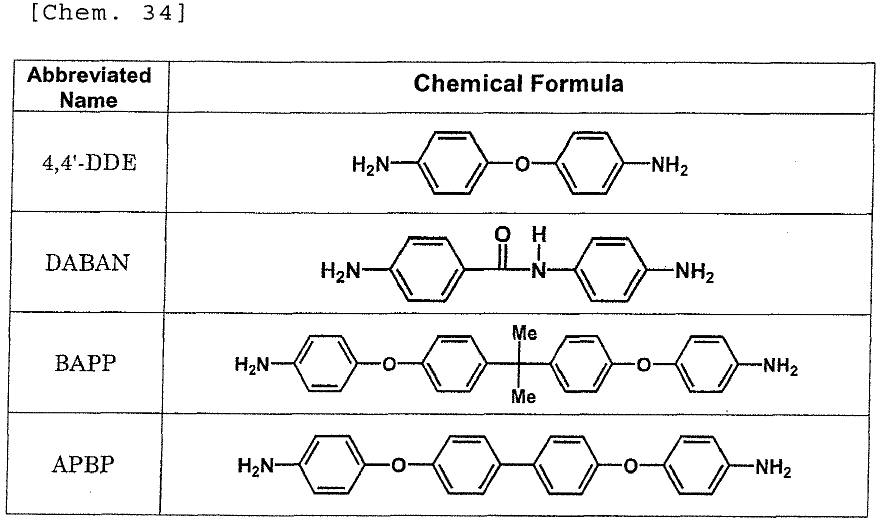

Note that each of the above aromatic diamines used is a commercial product (4,4'-DDE: manufactured by Tokyo Chemical Industry Co., Ltd., DABAN: manufactured by Nippon Junryo Chemicals Co Ltd., BAPP: manufactured by Tokyo Chemical Industry Co., Ltd., and APBP: manufactured by Nippon Junryo Chemicals Co Ltd.).

Subsequently, methods for evaluating characteristics of compounds and the like obtained in Examples and Comparative Examples are described.

### <Identification of Molecular Structures>

The molecular structures of compounds obtained in Examples and Comparative Examples were identified by employing as appropriate measurements such as infrared absorption spectrum measurement (IR measurement), nuclear magnetic resonance spectrum measurement (NMR measurement), and the like depending on the compound. Note that the IR measurement and the NMR measurement used, as measuring apparatuses, an IR measuring apparatus (manufactured by Thermo Scientific under the trade name of Nicolet 380 FT-IR Spectrometer) and an NMR measuring apparatus (manufactured by VARIAN under the trade name of UNITY INOVA-600), respectively.

### <Measurement of Glass Transition Temperature (Tg)>

The values (unit: °C) of the glass transition temperatures (Tg) of the polyimides obtained in Example 3, Examples 7 and 8, Comparative Example 1, and Comparative Examples 5 and 6 were measured by using a thermomechanical analyzer (manufactured by Rigaku Corporation under the trade name "TMA 8311") as a measuring apparatus, using samples cut from films comprising polyimides obtained in above-described Examples and Comparative Examples in sizes attachable to a jig of the measuring apparatus (sample sizes do not affect the measurement values), and employing the same method (the same conditions) as a method for measuring the softening temperature described below.

### <Measurement of Softening Temperature>

The softening temperatures of the polyimides obtained in Examples and Comparative Examples were measured by using a thermomechanical analyzer (manufactured by Rigaku Corporation under the trade name "TMA 8311") as a measuring apparatus, using samples cut from films comprising polyimides obtained in Examples and Comparative Examples in sizes attachable to a jig of the measuring apparatus (sample sizes do not affect the measurement values), and penetrating the film with a transparent quartz pin (diameter of the tip: 0.5 mm) at a pressure of 500 mN under a nitrogen atmosphere under a condition of a rate of temperature rise of 5°C/min and under a condition of a temperature range (scanning temperature) of 30°C to 550°C (a measurement by a so-called penetration (needle insertion) method). In the measurement, the softening temperatures were calculated based on measurement data in accordance with a method described in JIS K 7196 (1991) except that the measurement samples were used.

### <Measurement of Intrinsic Viscosity [η]>

The intrinsic viscosity [η] of the polyamic acid obtained as an intermediate in producing the film or the like comprising a polyimide in Examples and Comparative Examples was measured as follows. Specifically, a measurement sample of the polyamic acid was prepared at a concentration of 0.5 g/dL by using N,N-dimethylacetamide as a solvent. Then, the intrinsic viscosity [η] was measured by using an automatic viscometer (trade name: "VMC-252") manufactured by RIGO CO., LTD. under a temperature condition of 30°C.

### <Measurement of Total Luminous Transmittance, Haze (Turbidity), and Yellowness Index (YI)>

The values of total luminous transmittance (unit: %), haze (turbidity: HAZE), and yellowness index (YI) were obtained by carrying out measurement by use of the polyimides (polyimides in the shape of a film) produced in Examples and Comparative Examples as samples for measurement as it is, and by use of a measuring apparatus for the total luminous transmittance and the haze manufactured by NIPPON DENSHOKU INDUSTRIES CO., LTD. under the trade name of "Haze Meter NDH-5000" and a measuring apparatus for the yellowness index manufactured by NIPPON DENSHOKU INDUSTRIES CO., LTD. under the trade name of "Spectrophotometer SD6000." Note that the total luminous transmittance was obtained by performing measurement in accordance with JIS K7361-1 (issued in 1997), the haze (turbidity) is obtained by performing measurement in accordance with JIS K7136 (issued in 2000), and the chromaticity (YI) is obtained by performing measurement in accordance with ASTM E313-05(issued in 2005).

### <Measurement of Loss Tangent (tanδ) and Relative Permittivity (εr)>

Sample pieces were prepared by cutting the polyimides (polyimides in the shape of a film) produced in Examples 3, 7, and 8 and Comparative Example 1, 2, 5, and 6 in a size of width: 15 mm and length 80 mm. Then, a cavity resonator perturbation method was employed to measure the values of the loss tangent (tanδ) and the relative permittivity (εr) as follows.

The measurement of each of the values of the loss tangent (tanδ) and the relative permittivity (sr) was carried out using the test pieces (width: 15 mm and length: 80 mm) prepared as described above in a laboratory adjusted under an environment of 23°C and a relative humidity of 50%. In addition, the measuring apparatus used was an apparatus made up of one under the trade name of "PNA-L Network Analyzer N5230A" manufactured by Agilent Technologies, Inc. and one under the trade name of "Cavity Resonator CP431" manufactured by Electronic Application and Development Inc. In addition, in the measurement, the test pieces were set in the cavity resonator (manufactured by Kanto Electronic Application and Development Inc. under the trade name of "Cavity Resonator CP431) of the measuring apparatus. Then, actual measurement values of the loss tangent (tanδ) and the relative permittivity (εr) were obtained by the cavity resonator perturbation method (in accordance with ASTM D2520) at a frequency of 1 GHz. Each of the values of the loss tangent (tanδ) and the relative permittivity (εr) was obtained by measuring the actual measurement values three times in total and obtaining the average value thereof. As described above, the average value of the actual measurement values obtained by three measurements was employed as the value of each of the loss tangent (tanδ) and the relative permittivity (εr).

### (Example 1)

A 1000 mL autoclave vessel (manufactured by Taiatsu Techno Corporation under the trade name of "Hyper Gras Star TEM-V-type") made of glass was added with methanol (410 mL), CuCl₂(II) (40.8 g, 304 mmol), 5,5'-bibicyclo[2.2.1]hept-2-ene (also referred to as: 5,5'-bi-2-norbornene. 13.8 g, 74.1mmol: in the following description, sometimes simply referred to as "raw material compound.") represented by the following general formula (16) : 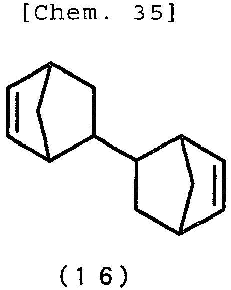 and Pd₃(OAc)₅(NO₂) (83.2 mg, 0.37 mmol in terms of Pd), to thereby obtain a mixture liquid. Note that Pd₃(OAc)₅(NO₂) was produced by employing a method described in page 1991 of Dalton Trans (vol. 11), published in 2005.

Subsequently, a glass tube was provided so that bubbling of gas could be performed through the glass tube to the mixture liquid present inside the vessel. Next, the vessel was tightly closed and the inside atmospheric gas was substituted with nitrogen. After that, a vacuum pump was connected to the vessel to reduce the pressure inside the vessel (pressure inside the vessel: 0.015 MPa). Next, the mixture liquid was stirred for 2.5 hours while supplying, by bubbling, carbon monoxide at a rate (flow rate) of 0.015 mole equivalents/min relative to the raw material compound through the glass tube into the mixture liquid and maintaining the conditions of temperature: 25 to 30°C and pressure: 0.13MPa. Afterthat, the temperature condition was changed and the mixture liquid was further stirred for 2 hours while maintaining the conditions of temperature: 40°C and pressure: 0.13 MPa. Thereby, the reaction liquid was obtained.

Subsequently, the atmospheric gas containing carbon monoxide was removed from the inside of the vessel. Then, methanol was removed (removed by distillation) from the reaction liquid by concentrating the reaction liquid with an evaporator while maintaining the temperature within a range of 30 to 40°C. Thereby, the reaction product was obtained. After that, the reaction product was added with chloroform (200 ml), followed by Celite® filtration and separation of the filtrate by use of 5% hydrochloric acid and saturated sodium hydrogen carbonate. Thereby, organic layers were collected. Next, the organic layers collected as described above were added with 20 g of anhydrous sodium sulfate as a desiccant, followed by stirring for 1 hour. Next, the desiccant was separated by filtration from the organic layers. After the desiccant was separated by filtration, the organic layers were concentrated. Thereby, the product (white to light yellow solid, the yield of 26.8 g, and the yield of 85.6%) was obtained.

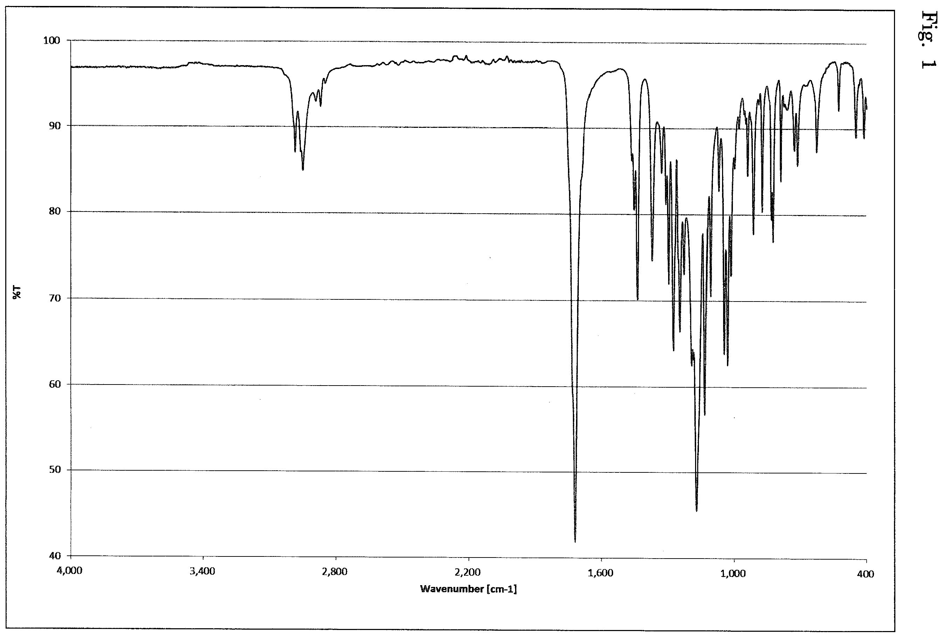

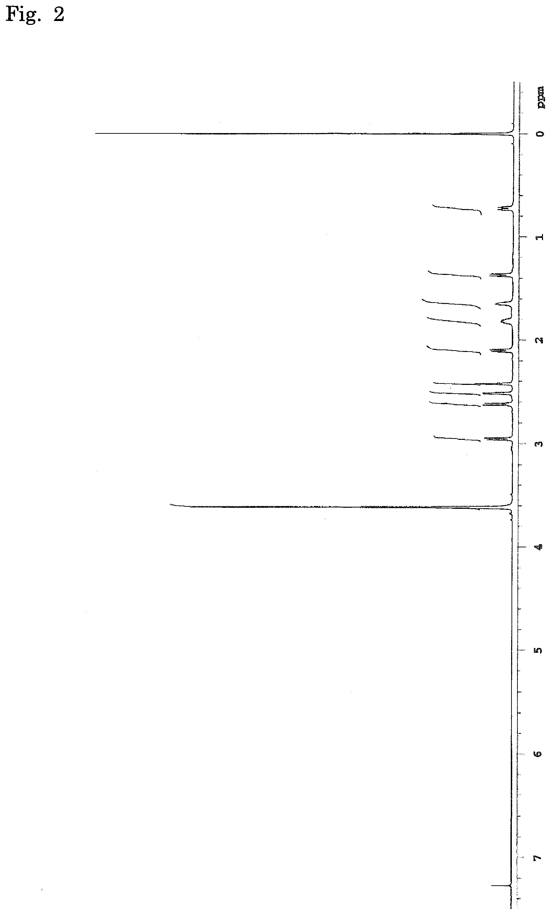

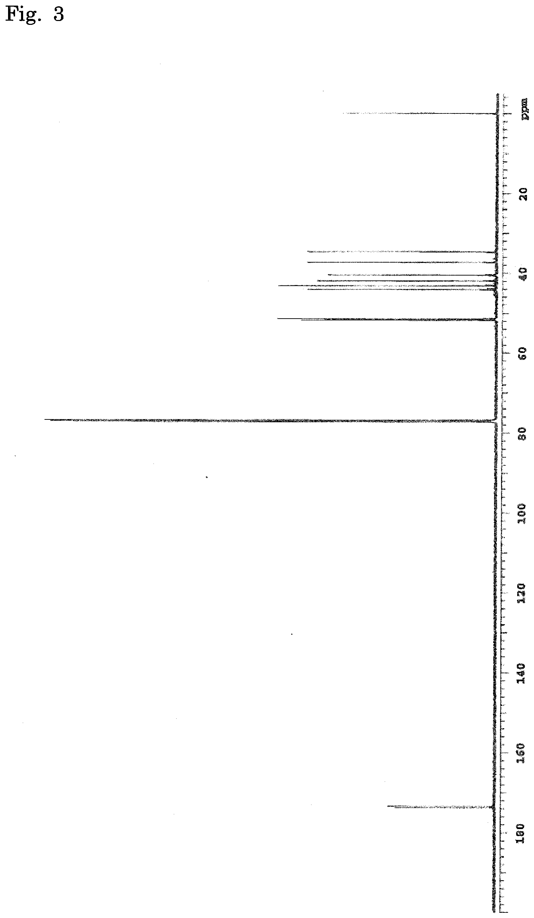

To identify the structure of the thus obtained product, IR measurement and NMR (¹H-NMR, ¹³C-NMR) measurement were carried out. Fig. 1 shows an IR spectrum of the thus obtained product, and Fig. 2 and Fig. 3 show ¹H-NMR and ¹³C-NMR (CDCl₃) spectra, respectively. As is apparent from the results shown in Figs. 1 to 3, the obtained product was identified to be a tetraester compound (5,5'-bi-2-norbornene-5,5',6,6'-tetracarboxylic acid tetramethyl ester) represented by the following general formula (17):

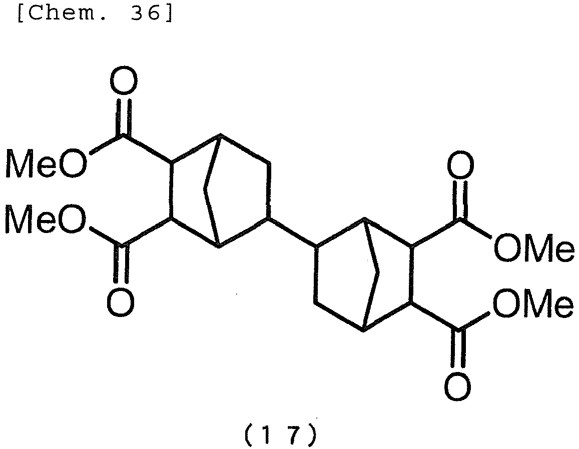

Moreover, when a GPC analysis was conducted on the obtained product, the content of impurities (a polymerization product in which norbornene rings in the raw material compound are addition-polymerized, a polymerization product in which multiple norbornene rings are bonded at keto groups, and the like) was identified to be 0.74%. From these results, it was found that the method employed in Example 1 made it possible to sufficiently efficiently produce the tetraester compound represented by the general formula (17). Note that the GPC analysis was conducted using a gel permeation chromatography measuring apparatus (GPC, manufactured by Tosoh Corporation under the trade name of HLC-8020/4 columns : manufactured by Tosoh Corporation under the trade name of TSK gel GMH_{HR}, and solvent : tetrahydrofuran (THF)).

### (Example 2)

First, a solution was prepared by dissolving 72 g of acetic acid with 5 g of the tetraester compound obtained in Example 1 and represented by the general formula (17) (5,5'-bi-2-norbornene-5,5',6,6'-tetracarboxylic acid tetramethyl ester) . The solution was added into a flask of capacity 200 mL with a refluxing tube. Next, 0.089 g of trifluoromethanesulfonic acid (CF₃SO₃H) as an acid catalyst (homogeneous acid catalyst) was added into the solution. Note that the amount of the acid catalyst used (the amount added into the solution) was such an amount that the mole ratio of the functional group (sulfonic acid) in the acid catalyst ([amount of moles the tetraester compound] : [amount of moles the functional group (sulfonic acid) in the catalyst)]) was 1:0.05 relative to the tetraester compound represented by the general formula (17) (such an amount that the amount of moles of the acid of the catalyst was 0.05 mole equivalents relative to the tetraester compound).

Next, after the atmospheric gas in the flask was substituted with nitrogen, the solution was heated while being stirred with a magnetic stirrer under a nitrogen stream and under a condition of atmospheric pressure. Refluxing was carried out for 0.5 hours at a temperature of 118°C inside the flask (refluxing step). After the refluxing step, a step (hereinafter referred to as "step (i) ") was carried out in which vapor generated by using a Liebig condenser was removed by distillation while continuing the heating so as to maintain the temperature inside the flask at 118 °C and at the same time the amount of liquid in the flask was kept constant by adding acetic acid into the flask by using a dropping funnel. Note that in step (i), 2 hours after the removal by distillation of the vapor was started, a white precipitate produced was observed in the liquid inside the flask (in the reaction solution) . In addition, in step (i), the progress of the reaction was checked every hour by analyzing the distillate removed by distillation to the outside of the system by means of mass measurement and a gas chromatograph. Note that the analysis revealed that acetic acid, methyl acetate, and water were present in the distillate. In step (i), since the distillation of the methyl acetate was stopped 5 hours after the removal by distillation of the vapor was started, the heating was stopped and step (i) was finished. Note that the amount of the methyl acetate distillated (total amount) by the time 5 hours had passed since the removal by distillation was started was 2. 9 g. In addition, the amount of the acetic acid removed by distillation by the time the distillation of the methyl acetate was stopped (by the time the reaction was finished) was 59 g.

As described above, after carrying out step (i), a liquid concentrate was obtained by removing by distillation acetic acid from the solution inside the flask. After that, vacuum filtration using a filter paper was carried out on the liquid concentrate to obtain a white solid. The obtained white solid was cleaned with ethyl acetate and dried. Thereby, 3.1 g of white powder was obtained.

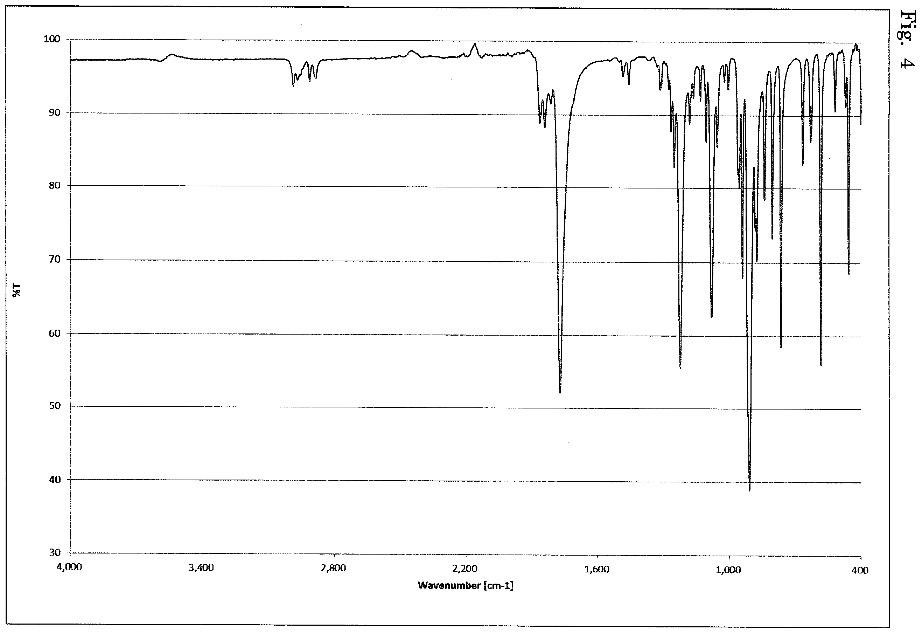

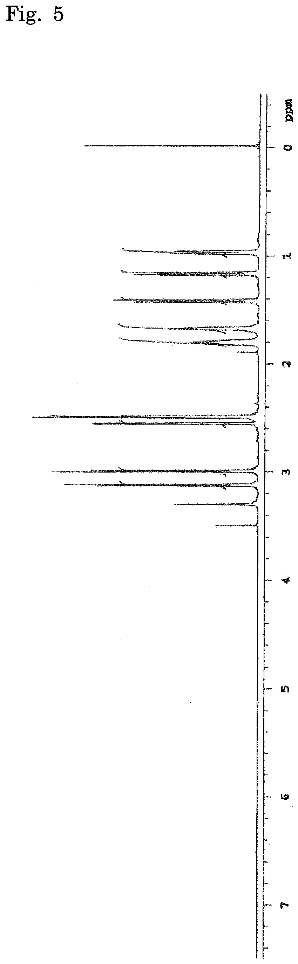

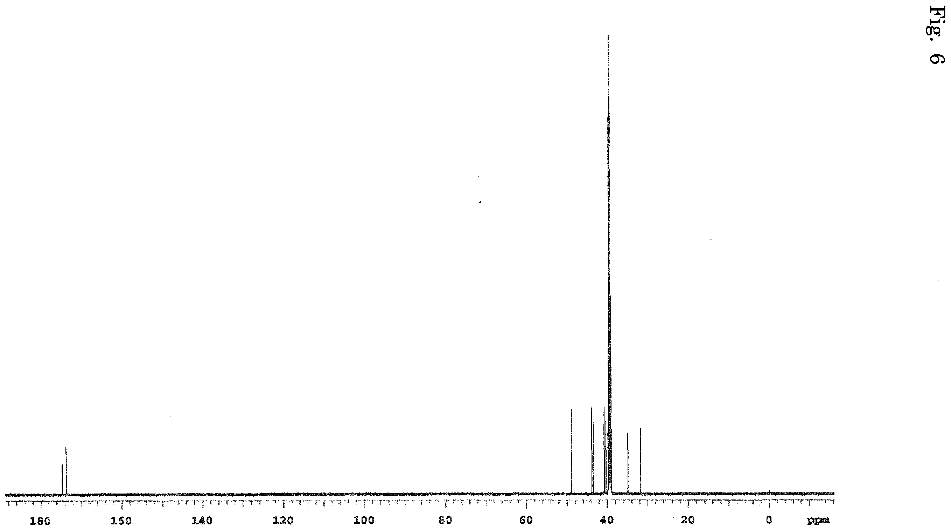

To identify the structure of the thus obtained white powder (product), IR measurement and NMR (¹H-NMR, ¹³C-NMR) measurement were carried out. Fig. 4 shows the thus obtained IR spectrum, and Fig. 5 and Fig. 6 show ¹H-NMR and ¹³C-NMR (DMSO-d₆) spectra, respectively. As is apparent from the results and the like shown in Figs. 4 to 6, the product obtained in Example 2 was identified to be a tetracarboxylic dianhydride (5,5'-bi-2-norbornene-5,5',6,6'-tetracarboxylic acid-5,5',6,6'-dianhydride) represented by the following general formula (18):

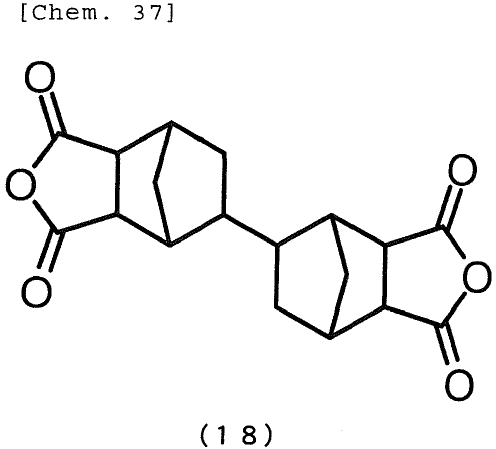

In addition, regarding the thus obtained compound, the yield of the product relative to the theoretical value calculated from the amount of the tetraester compound (raw material) prepared (amount used) represented by the general formula (17) and used in the production was calculated. The yield was calculated to be 79.6%. Moreover, when the obtained product was checked visually, it was white and no color development was observed.

### (Example 3)

### <Step of Preparing Polyamic Acid>

Under a nitrogen atmosphere, 0.601 g (3.00 mmol) of 4,4'-diaminodiphenyl ether (4,4'-DDE) was introduced as an aromatic diamine into a 20 mL screw cap vial, and also 0.9910 g (3.00 mmol) of the tetracarboxylic dianhydride obtained in Example 2 (tetracarboxylic dianhydride represented by the general formula (18)) was introduced into the screw cap vial. Subsequently, 6.01 g of dimethylacetamide (N,N-dimethylacetamide) was added into the screw cap vial to obtain a mixture liquid. Next, the obtained mixture liquid was stirred under a nitrogen atmosphere at room temperature (25°C) for 3 hours to produce a polyamic acid, thereby obtaining a reaction liquid containing the polyamic acid (solution of polyamic acid) . Note that a dimethylacetamide solution containing the polyamic acid at a concentration of 0.5 g/dL was prepared by using the thus obtained reaction liquid [a solution of the polyamic acid (solvent: dimethylacetamide)], and the intrinsic viscosity [η] of the polyamic acid was measured. The result showed that the intrinsic viscosity [η] was 0.579 dL/g.

### <Step of Preparing Polyimide (Thermal Imidization Step) >

The reaction liquid obtained in the step of preparing a polyamic acid (solution of polyamic acid) was spin-coated onto a large scale glass slide (manufactured by Matsunami Glass Ind., Ltd. under the trade name of "S9213, " length: 76 mm, width 52 mm, and thickness 1.3 mm), thereby forming a coating film on the glass plate. After that, the glass plate having the coating film formed was introduced into an oven and was allowed to stand for 4 hours under a temperature condition of 60°Cunder a nitrogen atmosphere. Thereafter, the temperature condition was changed to 350°C (final heating temperature) and the glass plate was allowed to stand for 1 hour to cure the coating film. Thereby, polyimide-coated glass coated with a thin film made of polyimide (film made of polyimide) on the glass substrate was obtained.

Next, the thus obtained polyimide-coated glass was taken out of the oven and was immersed in hot water of 90°C for 0.5 hours, and the film was collected by being peeled off of the glass substrate. Thereby, a film made of polyimide (film having sizes of length: 76 mm, width 52 mm, and thickness 12 µm) was obtained. Note that regarding the obtained film made of polyimide, when the color was checked visually, the color was observed to be transparent.

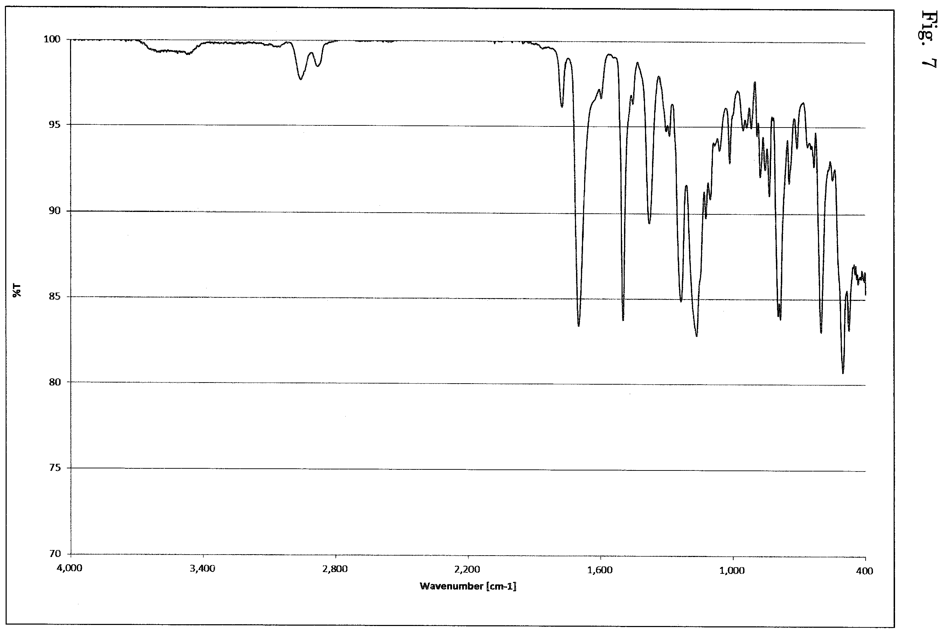

An IR spectrum of the thus obtained film was measured. Fig. 7 shows the IR spectrum of the obtained film. As is apparent from the results shown in Fig. 7, a C=O stretching vibration of imidocarbonyl was observed at 1701.0 cm⁻¹, indicating that the obtained film was made of a polyimide.

Table 1 shows evaluation results of characteristics of the thus obtained polyimide. Note that, since the total luminous transmittance of the obtained film made of polyimide was 89% as shown in Table 1, the light transmittance was found to be sufficiently high. In addition, the softening temperature of the polyimide forming the thus obtained film was measured with a thermomechanical analyzer (manufactured by Rigaku Corporation under the trade name of "TMA 8310"), and the softening temperature thus measured was 499°C and Tg was 356°C. Hence, it was found that the polyimide had a sufficiently high heat resistance.

### (Comparative Example 1)

### <Step of Preparing Tetracarboxylic Dianhydride>

Prepared was a tetracarboxylic dianhydride (norbornane-2-spiro-a-cyclopentanone-α'-spiro-2''-norb ornane-5,5'',6,6''-tetracarboxylic dianhydride) represented by the following general formula (19): 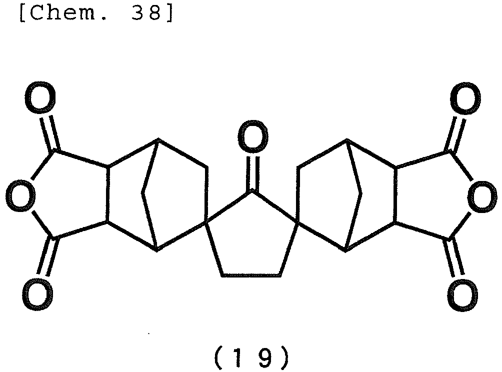 in accordance with a method described in Synthesis Example 1, Example 1, and Example 2 of International Publication No. WO2011/099518.

### <Step of Preparing Polyamic Acid>

A 30 ml three-necked flask was,heated with a heat gun and sufficiently dried. Next, the atmospheric gas inside the sufficiently dried three-necked flask was substituted with nitrogen to fill the three-necked flask with a nitrogen atmosphere. Next, after 0.1802 g of 4,4'-diaminodiphenyl ether (0.90 mmol: 4,4'-DDE) as an aromatic diamine was added into the three-necked flask, 2.7 g of dimethylacetamide (N,N-dimethylacetamide) was further added and stirred. Thereby, the aromatic diamine (4,4'-DDE) was dissolved into the N,N-dimethylacetamide to obtain a dissolution liquid. Next, 0.3459 g (0.90 mmol) of the compound represented by the general formula (19) was added into the three-necked flask containing the dissolution liquid under a nitrogen atmosphere, followed by stirring for 12 hours under a nitrogen atmosphere at room temperature (25°C). Thereby, a reaction liquid (solution of polyamic acid) containing polyamic acid was obtained.

Note that a portion of the reaction liquid (solution of polyamic acid) was used to prepare a solution of N,N-dimethylacetamide having a concentration of polyamic acid of 0.5 g/dL, and the intrinsic viscosity [η] of the polyamic acid being a reaction intermediate was measured as described above. The intrinsic viscosity [η] of the polyamic acid was 1.00 dL/g.

### <Step of Preparing Polyimide (Thermal Imidization Step) >

A large scale glass slide (manufactured by Matsunami Glass Ind., Ltd. under the trade name of "S9213," length: 76 mm, width 52 mm, and thickness 1.3 mm) was prepared as a glass substrate. The reaction liquid (solution of polyamic acid) obtained as described above was spin-coated onto the surface of the glass substrate so that the thickness of the coating film after thermal curing was a thickness of 13 µm. Thereby, a coating film was formed on the glass substrate. After that, the glass substrate having the coating film formed thereon was placed on a hot plate of 60°C and was allowed to stand for 2 hours. Thereby, the solvent was vaporized and removed from the coating film (solvent removal treatment).

After the solvent removal treatment was carried out, the glass substrate having the coating film formed thereon was introduced into an inert oven in which nitrogen was flowing at a flow rate of 3 L/min. Inside the inert oven, the coating film was cured by allowing the glass substrate to stand for 0.5 hours under a nitrogen atmosphere under a temperature condition of 25°C, and then heating for 0.5 hours under a temperature condition of 135°C, followed by further heating for 1 hour under a temperature condition (final heating temperature) of 350°C. Thereby, a polyimide-coated glass coated with a thin film made of polyimide (film made of polyimide) on the glass substrate was obtained.

Next, the thus obtained polyimide-coated glass was immersed in hot water of 90°C to peel the film off of the glass substrate. Thereby, a film (film having sizes of length 76 mm, width 52 mm, and thickness 13 µm) made of polyimide was obtained.

Note that when the molecular structure of the compound forming the obtained film was identified in the same manner as in Example 3, the compound forming the film was identified to be a polyimide. In addition, Table 1 shows evaluation results of characteristics of the thus obtained polyimide. Note that, since the total luminous transmittance of the film made of the obtained polyimide was 88% as shown in Table 1, the light transmittance was found to be sufficiently high. In addition, the softeningtemperature of the polyimide forming the thus obtained film was measured with a thermomechanical analyzer (manufactured by Rigaku Corporation under the trade name of "TMA 8310"), and the softening temperature thus measured was 480°C and Tg was 347°C. Hence, it was found that the polyimide had a sufficiently high heat resistance.

### (Comparative Example 2)

A film made of polyimide (film having sizes of length 76 mm, width 52 mm, and thickness 9 µm) was obtained in the same manner as in Example 3 except that, in the step of preparing a polyamic acid, the amount of 4,4'-diaminodiphenyl ether prepared was changed to 2.0024 g (10.0 mmol: 4,4'-DDE), the amount of dimethylacetamide (N,N-dimethylacetamide) prepared was changed to 22.5 g, 1.9611 g (10.0 mmol) of 1,2,3,4-cyclobutane tetracarboxylic dianhydride (CBDA) manufactured by Tokyo Chemical Industry Co., Ltd. was used as a tetracarboxylic dianhydride instead of adding the compound represented by the general formula (18), and furthermore, in the step of preparing a polyimide (thermal imidization step), the temperature condition of the final heating temperature when curing the coating film was changed from 350°C to 300°C.

Note that when the molecular structure of the compound forming the obtained film was identified in the same manner as in Example 3, the compound forming the film was identified to be a polyimide. In addition, Table 1 shows evaluation results of characteristics of the thus obtained polyimide.

**[Table 1]**

| | Type of Tetracarboxylic Dianhydride | Type of Aromatic Diamine | Condition of Preparation | | Characteristic of Polyimide | | | | | | |
|---|---|---|---|---|---|---|---|---|---|---|---|
| | | | Stirring Temperature in Step of Preparing Polyamic Acid (Unit: °C) | Final Heating Temperature in Step of Preparing Polyimide (Unit: °C) | Tg (Unit: °C) | Softening Temperature (Unit: °C) | Total Luminous Transmittance (Unit: %) | HAZE | YI | εr | tanδ |
| Example 3 | Compound Represented by General Formula (18) | 4,4' -DDE | 25 | 350 | 356 | 499 | 89 | 1.4 | 2.1 | 3.16 | 0.0175 |
| Comparative Example 1 | Compound Represented by General Formula (19) | | 25 | 350 | 347 | 480 | 88 | 1.7 | 2.2 | 2.90 | 0.0190 |
| Comparative Example 2 | CBDA | | 25 | 300 | 350 | 453 | 89 | 2.3 | 2.2 | 3.81 | 0.0364 |

### [Evaluation Results of Characteristics of Polyimides Obtained in Example 3 and Comparative Examples 1 and 2]

From the results shown in Table 1, each of the polyimides obtained in Example 3 and Comparative Examples 1 and 2 was found to have a sufficiently high light transmittance. In addition, each of the polyimides obtained in Example 3 and Comparative Example 1 had a value of softening temperature of 480°C or higher and was found to have an excellent heat resistance. Note that since the polyimide obtained in Comparative Example 2 had a softening temperature of 453°C, the polyimides obtained in Example 3 and Comparative Example 1 were found to be ones having better heat resistances. Here, comparison between the polyimide obtained in Example 3 and the polyimides obtained in Comparative Examples 1 and 2 shows that the softening temperature of the polyimide obtained in Example 3 is a higher temperature. The polyimide obtained in Example 3 makes it possible to achieve a heat resistance at a higher level with the softening temperature as a reference. Note that, from the type of monomer used, the results of the IR spectrum, and the like, the polyimide obtained in Example 3 was found to be a polyimide which contained the repeating unit represented by the general formula (1) (each of R¹, R², and R³ in the formula is a hydrogen atom, and R⁵ is a group represented by the general formula (12) (Q in the formula is -O-)), and the repeating unit derived from a tetracarboxylic dianhydride was found to have a structure different from those of the polyimides obtained in Comparative Examples 1 and 2. From these results, it was found that higher heat resistance could be obtained with a polyimide (polyimide containing the repeating unit represented by the general formula (1)) formed by using the tetracarboxylic dianhydride obtained in Example 2 (tetracarboxylic dianhydride represented by the general formula (18)) in comparison with the polyimide formed by using the tetracarboxylic dianhydride represented by the general formula (19) or a CBDA.

In addition, as is apparent from the results shown in Table 1, the polyimide obtained in Example 3 had a value of loss tangent (tanδ) lower than those of the polyimides obtained in Comparative Examples 1 and 2. As described above, it was found that the polyimide (Example 3: polyimide containing the repeating unit represented by the general formula (1)) formed by using the tetracarboxylic dianhydride obtained in Example 2 (tetracarboxylic dianhydride represented by the general formula (18)) made it possible to achieve a lower loss tangent (tanδ) compared to a polyimide formed by using the tetracarboxylic dianhydride represented by the general formula (19) or CBDA. From these results, it is found that when used in, for example, an interlayer insulating film material for semiconductor, a board film for a flexible printed circuit board, and the like, the polyimide obtained in Example 3 has a lower transmission loss in comparison with the polyimides obtained in Comparative Examples 1 and 2. For this reason, the polyimide obtained in Example 3 is preferably applicable to, for example, a high frequency band material and the like.

### (Example 4)

### <Step of Preparing Polyamic Acid>

First, a reaction liquid (solution of polyamic acid) was obtained by employing the same step as the step of preparing a polyamic acid employed in Example 3 except that 0.682 g (3.00 mmol) of 4, 4-diaminobenzanilide (DABAN) was used instead of using 0.601 g (3.00 mmol) of 4,4'-diaminodiphenyl ether (4,4'-DDE) as an aromatic diamine, and the temperature when stirring the mixture liquid was changed from room temperature (25°C) to 60°C. Next, 6.69 g of dimethylacetamide (N,N-dimethylacetamide) was added to the thus obtained reaction liquid (solution of polyamic acid) to prepare a coating liquid to be used in the step of preparing a polyimide (thermal imidization step) described below.

### <Step of Preparing Polyimide (Thermal Imidization Step) >

A film made of polyimide (film having sizes of length: 76 mm, width 52 mm, and thickness 8 µm) was obtained in the same manner as in Example 3 except that the coating liquid obtained by using 0.682 g (3.00 mmol) of 4,4-diaminobenzanilide (DABAN) instead of the reaction liquid (solution of polyamic acid) was used, and furthermore, in the step of preparing a polyimide (thermal imidization step), the temperature condition of the final heating temperature when curing the coating film was changed from 350°C to 360°C.

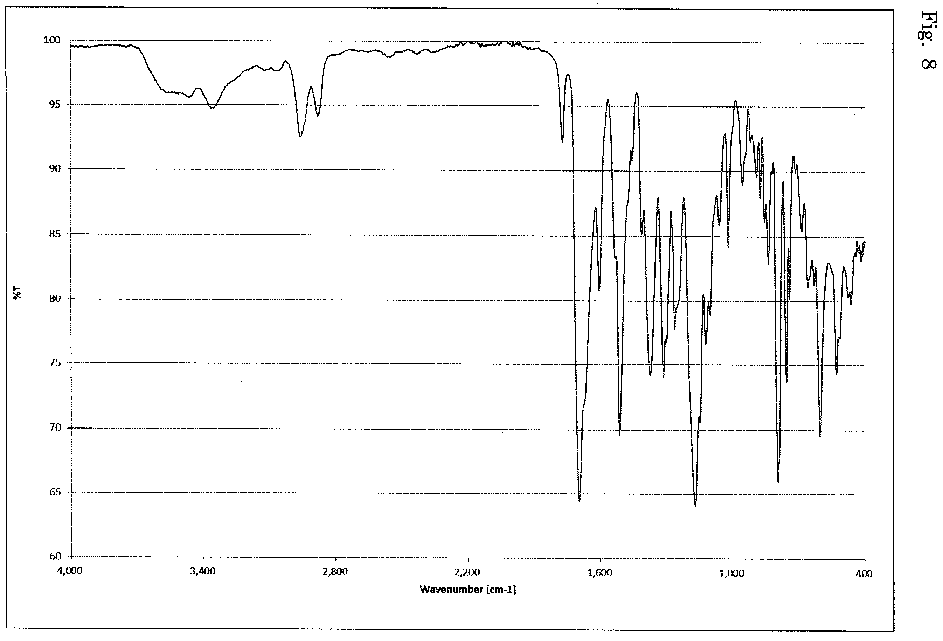

An IR spectrum of the film obtained in Example 4 was measured. Fig. 8 shows the IR spectrum of the obtained film. As is apparent from the results shown in Fig. 8, a C=O stretching vibration of imidocarbonyl was observed at 1697.1 cm⁻¹, indicating that the obtained film was made of a polyimide. Note that, from the type of monomer used, the results of the IR spectrum, and the like, the polyimide obtained in Example 4 was found to be a polyimide which contained the repeating unit represented by the general formula (1) (each of R¹, R², and R³ in the formula is a hydrogen atom, and R⁵ is a group represented by the general formula (12) (Q in the formula is -CONH-)). Table 2 shows evaluation results of characteristics of the thus obtained polyimide.

### (Comparative Example 3)

A film made of polyimide (film having sizes of length 76 mm, width 52 mm, and thickness 13 µm) was obtained by employing the same method as in Comparative Example 1 except that, in the step of preparing a polyamic acid, 0.2045 g (0.90 mmol: DABAN) of 4,4'-diaminobenzanilide was used instead of using 0.1802 g (0.90 mmol) of 4,4'-diaminodiphenyl ether as an aromatic diamine, and furthermore, in the step of preparing a polyimide (thermal imidization step), in the step of preparing a polyimide (thermal imidization step), the temperature condition of the final heating temperature when curing the coating film was changed from 350°C to 380°C.

Note that when the molecular structure of the compound forming the obtained film was identified in the same manner as in Example 3, the compound forming the film obtained in Comparative Example 3 was identified to be a polyimide. In addition, the intrinsic viscosity [η] of the polyamic acid obtained in the step of preparing a polyamic acid was 0.91 dL/g. Furthermore, Table 2 shows evaluation results of characteristics of the thus obtained polyimide.

**[Table 2]**

| | Type of Tetracarboxylic Dianhydride | Type of Aromatic Diamine | Condition of Preparation | | Characteristic of Polyimide | | | |
|---|---|---|---|---|---|---|---|---|
| | | | Stirring Temperature in Step of Preparing Polyamic Acid (Unit: °C) | Final Heating Temperature in Step of Preparing Polyimide (Unit: °C) | Softening Temperature (Unit: °C) | Total Luminous Transmittance (Unit: %) | HAZE | YI |
| Example 4 | Compound Represented by General Formula 18 | DABAN | 60 | 360 | 514 | 88 | 0.5 | 2.5 |
| Comparative Example 3 | Compound Represented by General Formula (19) | | 25 | 380 | 502 | 88 | 0.7 | 2.4 |

### [Evaluation Results of Characteristics of Polyimides] Obtained in Example 4 and Comparative Example 3

From the results shown in Table 2, each of the polyimides obtained in Example 4 and Comparative Example 3 was found to have a sufficiently high light transmittance . In addition, each of the polyimides obtained in Example 4 and Comparative Example 3 had a value of softening temperature of 502°C or higher and was found to have an excellent heat resistance. Here, comparison between the polyimide obtained in Example 4 and the polyimide obtained in Comparative Example 3 shows that the softening temperature of the polyimide obtained in Example 4 is a higher temperature. The polyimide obtained in Example 4 makes it possible to achieve a heat resistance at a higher level with the softening temperature as a reference. Note that the polyimide obtained in Example 4 and the polyimide obtained in Comparative Example 3 have different types of tetracarboxylic dianhydride used in the production and have different structure portions of the repeating unit derived from the tetracarboxylic dianhydride . From these results, the polyimide (polyimide containing the repeating unit represented by the general formula (1)) formed by using the tetracarboxylic dianhydride obtained in Example 2 (tetracarboxylic dianhydride represented by the general formula (18)) was found to have a higher heat resistance compared to the polyimide obtained by using the tetracarboxylic dianhydride represented by the general formula (19).

### (Example 5)

### <Step of Preparing Polyamic Acid>

A reaction liquid (solution of polyamic acid) was obtained by employing the same step as the step of preparing a polyamic acid employed in Example 3 except that a mixture of 0.545 g (2.40 mmol) of 4,4-diaminobenzanilide (DABAN) and 0.120 g (0.60 mmol) of 4,4'-diaminodiphenyl ether (4,4'-DDE) (mole ratio between DABAN and 4,4'-DDE ([DABAN] : [4,4'-DDE]) was 80:20) was used instead of using 0.601 g (3.00 mmol) of 4,4'-diaminodiphenyl ether (4,4'-DDE) as an aromatic diamine, and the temperature when stirring the mixture liquid was changed from room temperature (25°C) to 60°C.

### <Step of Preparing Polyimide (Thermal Imidization Step) >

A film made of polyimide (film having sizes of length 76 mm, width 52 mm, and thickness 8 µm) was obtained in the same manner as in Example 3 except that the solution of polyamic acid obtained by using a mixture of 0.120 g (0.60 mmol) of 4,4'-diaminodiphenyl ether (4,4'-DDE) and 4,4-diaminobenzanilide (DABAN) was used as a reaction liquid (solution of polyamic acid).

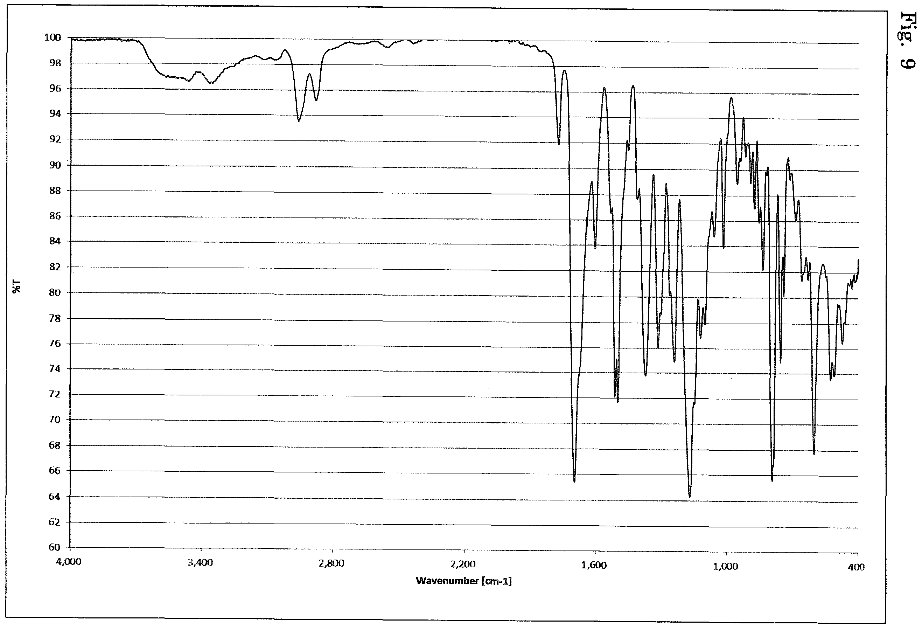

An IR spectrum of the film obtained in Example 5 was measured. Fig. 9 shows the IR spectrum of the obtained film. As is apparent from the results shown in Fig. 9, a C=O stretching vibration of imidocarbonyl was observed at 1697.1 cm⁻¹, indicating that the obtained film was made of a polyimide. Note that, from the type of monomer used, the results of the IR spectrum, and the like, the polyimide obtained in Example 5 was found to be a polyimide which contained the repeating unit represented by the general formula (1) (each of R¹, R², and R³ in the formula is a hydrogen atom, and R⁵ is a group represented by the general formula (12) (including 2 types of groups in which Q in the formula is -O- and -CONH-)). Table 3 shows evaluation results of characteristics of the polyimide.

### (Comparative Example 4)

A film made of polyimide (film having sizes of length 76 mm, width 52 mm, and thickness 13 µm) was obtained by employing the same method as in Comparative Example 1 except that, in the step of preparing a polyamic acid, a mixture of 0.1636 g (0.72 mol: DABAN) of 4,4'-diaminobenzanilide and 0.036 g (0.18 mol: 4,4'-DDE) of 4,4'-diaminodiphenyl ether (mole ratio between DABAN and 4,4'-DDE ([DABAN] : [4,4'-DDE]) was 80:20) was used instead of using 0.1802 g (0.90 mmol) of 4,4'-diaminodiphenyl ether as an aromatic diamine.

Note that when the molecular structure of the compound forming the obtained film was identified in the same manner as in Example 3, the compound forming the film obtained in Comparative Example 4 was identified to be a polyimide. In addition, Table 3 shows evaluation results of characteristics of the thus obtained polyimide.

**[Table 3]**

| | Type of Tetracarboxylic Dianhydride | Type of Aromatic Diamine* | Condition of Preparation | | Characteristic of Polyimide | | | |
|---|---|---|---|---|---|---|---|---|
| | | | Stirring Temperature in Step of Preparing Polyamic Acid (Unit: °C) | Final Heating Temperature in Step of Preparing Polyimide (Unit: °C) | Softening Temperature (Unit: °C) | Total Luminous Transmittance (Unit: %) | HAZE | YI |
| Example 5 | Compound Represented by General Formula (18) | Mixture of DABAN and 4,4' -DDE (Mole Ratio = 80:20) | 60 | 350 | 513 | 88 | 1 | 2.7 |
| Comparative Example 4 | Compound Represented by General Formula (19) | | 25 | 350 | 481 | 87 | 1.5 | 2.0 |

| | | | | | | | | |
|---|---|---|---|---|---|---|---|---|
| The symbol (*) in the table indicates that regarding the aromatic diamine, the mole ratio in the parentheses is the value of [amount of moles of DABAN]:[amount of moles of 4,4' -DDE]. | | | | | | | | |

### [Evaluation Results of Characteristics of Polyimides Obtained in Example 5 and Comparative Example 4]

From the results shown in Table 3, each of the polyimides obtained in Example 5 and Comparative Example 4 was found to have a sufficiently high light transmittance. In addition, each of the polyimides obtained in Example 5 and Comparative Example 4 had a value of softening temperature of 481°C or higher and was found to have an excellent heat resistance. Here, comparison between the polyimide obtained in Example 5 and the polyimide obtained in Comparative Example 4 shows that the softening temperature of the polyimide obtained in Example 5 is a higher temperature. The polyimide obtained in Example 5 makes it possible to achieve a heat resistance at a higher level with the softening temperature as a reference. Note that the polyimide obtained in Example 5 and the polyimide obtained in Comparative Example 4 have different types of tetracarboxylic dianhydride used in the production and have different structure portions of the repeating unit derived from the tetracarboxylic dianhydride . From these results , the polyimide (polyimide containing the repeating unit represented by the general formula (1)) formed by using the tetracarboxylic dianhydride obtained in Example 2 (tetracarboxylic dianhydride represented by the general formula (18)) was found to have a higher heat resistance compared to the polyimide obtained by using the tetracarboxylic dianhydride represented by the general formula (19).

### (Example 6)

First, a solution was prepared by dissolving 72 g of acetic acid with 5 g of the tetraester compound obtained in Example 1 and represented by the general formula (17) (5,5'-bi-2-norbornene-5,5',6,6'-tetracarboxylic acid tetramethyl ester) . The solution was added into a flask of capacity 200 mL with a refluxing tube. Next, 0.09 g of trifluoromethanesulfonic acid (CF₃SO₃H) as an acid catalyst (homogeneous acid catalyst) was added into the solution, and 69.0 g of acetic anhydride ((CH₃CO)₂O) was added thereinto. Note that the amount of the acid catalyst used (the amount added into the solution) was such an amount that the mole ratio of the functional group (sulfonic acid) in the acid catalyst ([amount of moles the tetraester compound] : [amount of moles the functional group (sulfonic acid) in the catalyst)]) was 1:0.05 relative to the tetraester compound represented by the general formula (17) (such an amount that the amount of moles of the acid of the catalyst was 0.05 mole equivalents relative to the tetraester compound).

Next, after the atmospheric gas in the flask was substituted with nitrogen, the solution was heated while being stirred by using a magnetic stirrer under a nitrogen stream and under a condition of atmospheric pressure. Refluxing was carried out for 5 hours at a temperature of 118°C inside the flask. After that, vacuum filtration using a filter paper was carried out to obtain a white solid. Next, the obtained white solid was cleaned with ethyl acetate and dried. Thereby, 3.1 g of white powder was obtained.

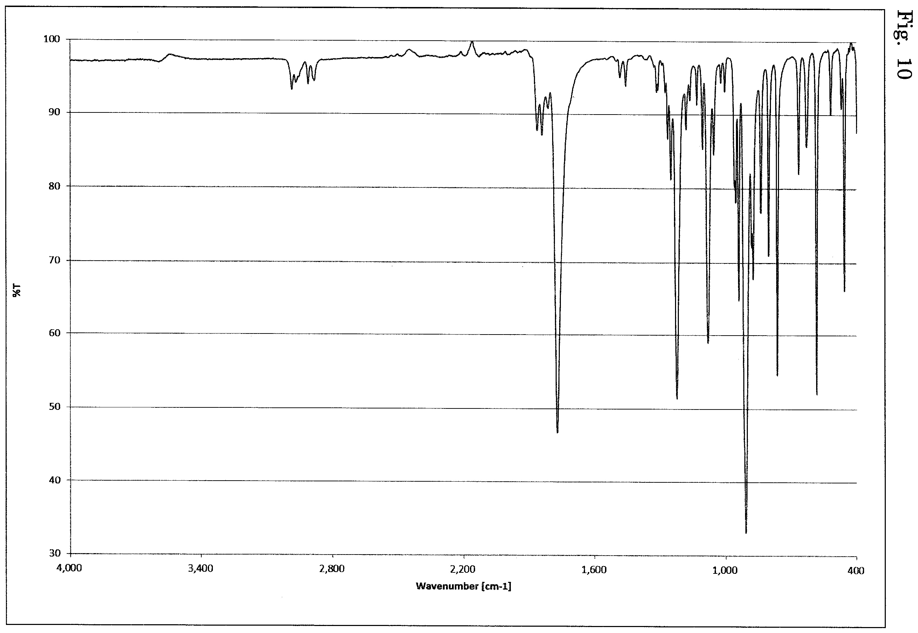

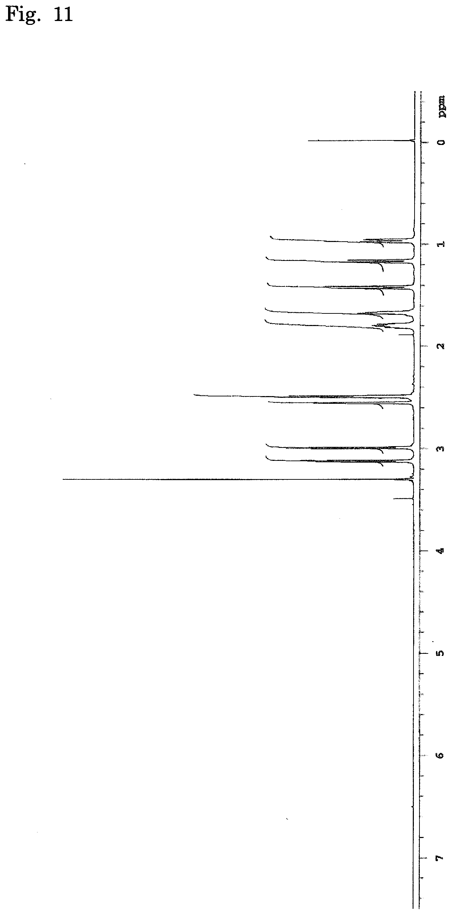

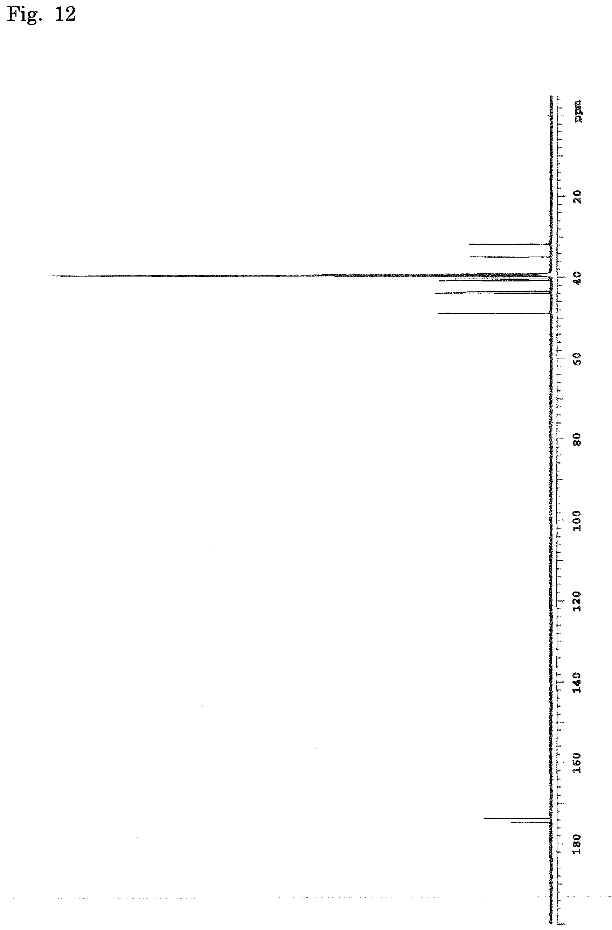

To identify the structure of the thus obtained white powder (product), IR measurement and NMR (¹H-NMR, ¹³C-NMR) measurement were carried out in the same manner as in Example 2. Fig. 10 shows the thus obtained IR spectrum, and Fig. 11 and Fig. 12 show ¹H-NMR and ¹³C-NMR (DMSO-d₆) spectra, respectively. As is apparent from the results and the like shown in Figs. 10 to 12, the product obtained in Example 6 was identified to be a tetracarboxylic dianhydride (5,5'-bi-2-norbornene-5,5',6,6'-tetracarboxylic acid-5,5',6,6'-dianhydride) represented by the above-described general formula (18).

In addition, regarding the thus obtained compound, the yield of the product relative to the theoretical value calculated from the amount of the tetraester compound (raw material) prepared (amount used) represented by the general formula (17) and used in the production was calculated. The yield was calculated to be 78.9%. Moreover, when the obtained product was checked visually, it was white and no color development was observed. From these results, it was found that the method employed in Example 6 also made it possible to efficiently produce the tetracarboxylic dianhydride (5,5'-bi-2-norbornene-5,5',6,6'-tetracarboxylic acid-5,5',6,6'-dianhydride) represented by the general formula (18).

### (Example 7)

### <Step of Preparing Polyamic Acid>

A solution of polyamic acid was obtained by employing the same step as the step of preparing a polyamic acid employed in Example 3 except that 1.232 g of 2,2-bis[4-(4-aminophenoxy)phenyl]propane (BAPP) was used instead of using 0.601 g (3.00 mmol) of 4,4'-diaminodiphenyl ether (4,4'-DDE) as an aromatic diamine, and furthermore, the tetracarboxylic dianhydride obtained in Example 6 employing a production method using an acetic anhydride (tetracarboxylic dianhydride represented by the general formula (18) :5,5'-bi-2-norbornene-5,5',6,6'-tetracarboxylic acid-5,5',6,6'-dianhydride: the compound obtained in Example 2 and the compound obtained in Example 6 are the same compounds although the production methods are different) was used instead of the tetracarboxylic dianhydride obtained in Example 2. 8.89 g of dimethylacetamide (N,N-dimethylacetamide) was added to the thus obtained reaction liquid (solution of polyamic acid) to prepare a coating liquid to be used in the step of preparing a polyimide (thermal imidization step) described below.

### <Step of Preparing Polyimide (Thermal Imidization Step) >

A film made of polyimide (film having sizes of length: 76 mm, width 52 mm, and thickness 7 µm) was obtained in the same manner as in Example 3 except that the coating liquid obtained by using 1.232 g (3.00 mmol) of 2,2-bis[4-(4-aminophenoxy)phenyl]propane (BAPP) instead of the reaction liquid (solution of polyamic acid) was used, and furthermore, in the step of preparing a polyimide (thermal imidization step), the temperature condition of the final heating temperature when curing the coating film was changed from 350°C to 300°C.

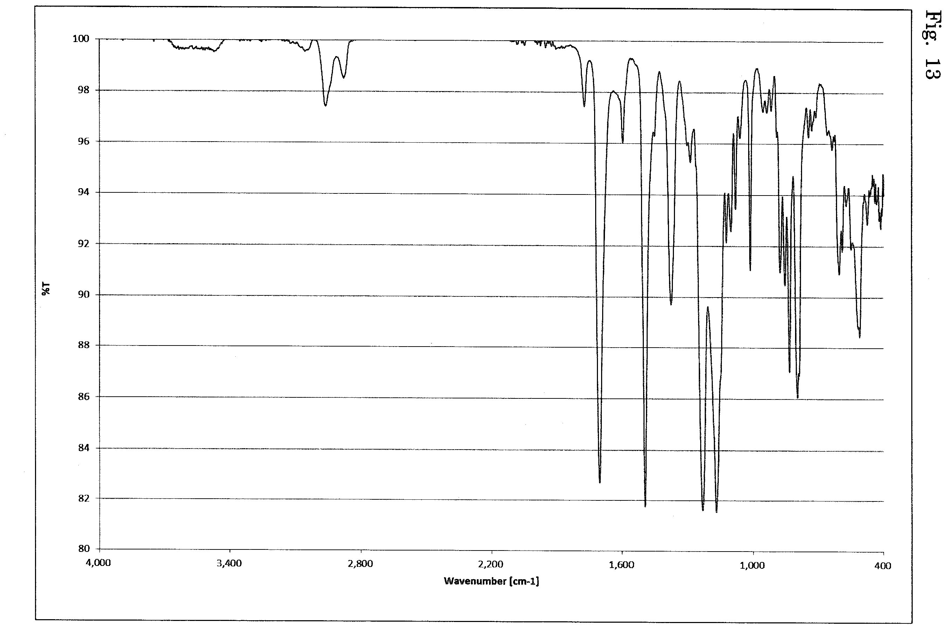

An IR spectrum of the film obtained in Example 7 was measured. Fig. 13 shows the IR spectrum of the obtained film. As is apparent from the results shown in Fig. 13, a C=O stretching vibration of imidocarbonyl was observed at 1702.9 cm⁻¹, indicating that the obtained film was made of a polyimide. Note that, from the type of monomer used, the results of the IR spectrum, and the like, the polyimide obtained in Example 7 was found to be a polyimide which contained the repeating unit represented by the general formula (1) (each of R¹, R², and R³ in the formula is a hydrogen atom, and R⁵ is a group represented by the general formula (12) (Q in the formula is -O-C₆H₄-C(CH₃)₂-C₆H₄-O-)). Table 4 shows evaluation results of characteristics of the thus obtained polyimide.

### (Comparative Example 5)

A film made of polyimide (film having sizes of length 76 mm, width 52 mm, and thickness 13 µm) was obtained by employing the same method as in Comparative Example 1 except that, in the step of preparing a polyamic acid, 0.3695 g (0.90 mmol) of 2,2-bis[4-(4-aminophenoxy)phenyl]propane (BAPP) was used instead of using 0.1802 g (0.90 mmol) of 4,4'-diaminodiphenyl ether as an aromatic diamine.

Note that when the molecular structure of the compound forming the obtained film was identified in the same manner as in Example 3, the compound forming the film obtained in Comparative Example 5 was identified to be a polyimide. In addition, the intrinsic viscosity [η] of the polyamic acid obtained in the step of preparing a polyamic acid was 0.51 dL/g. Furthermore, Table 4 shows evaluation results of characteristics of the thus obtained polyimide.

**[Table 4]**

| | Type of Tetracarboxylic Dianhydride | Type of Aromatic Diamine | Condition of Preparation | | Characteristic of Polyimide | | | | | | |
|---|---|---|---|---|---|---|---|---|---|---|---|
| | | | Stirring Temperature in Step of Preparing Polyamic Acid (Unit: °C) | Final Heating Temperature in Preparing Polyimide (Unit: °C) | Tg (Unit: °C) | Softening Temperature (Unit: °C) | Total Luminous Transmittance (Unit: %) | HAZE | YI | εr | tanδ |
| Example 7 | Compound Represented by General Formula (18) | BAPP | 25 | 300 | 301 | 451 | 89 | 1.3 | 1.0 | 3.17 | 0.0119 |
| Comparative Example 5 | Compound Represented by General Formula (19) | | 25 | 350 | 294 | 375 | 89 | 1.4 | 1.5 | 2.75 | 0.0154 |

### [Evaluation Results of Characteristics of Polyimides Obtained in Example 7 and Comparative Example 5]

From the results shown in Table 4, each of the polyimides obtained in Example 7 and Comparative Example 5 was found to have a sufficiently high light transmittance . In addition, each of the polyimides obtained in Example 7 and Comparative Example 5 had a softening temperature of 375°C or higher and was found to have an excellent heat resistance. Here, comparison between the polyimide obtained in Example 7 and the polyimide obtained in Comparative Example 5 shows that the softening temperature of the polyimide obtained in Example 7 is a higher temperature. The polyimide obtained in Example 7 makes it possible to achieve a heat resistance at a higher level with the softening temperature as a reference. Note that the polyimide obtained in Example 7 and the polyimide obtained in Comparative Example 5 have different types of tetracarboxylic dianhydride used in the production and have different structure portions of the repeating unit derived from the tetracarboxylic dianhydride . From these results , the polyimide (polyimide containing the repeating unit represented by the general formula (1)) formed by using the tetracarboxylic dianhydride obtained in Example 6 (tetracarboxylic dianhydride represented by the general formula (18)) was found to have a higher heat resistance compared to the polyimide obtained by using the tetracarboxylic dianhydride represented by the general formula (19).

In addition, as is apparent from the results shown in Table 4, the polyimide obtained in Example 7 had a value of loss tangent (tanδ) lower than that of the polyimide obtained in Comparative Example 5. As described above, it was found that the polyimide (Example 7: polyimide containing the repeating unit represented by the general formula (1)) formed by using the tetracarboxylic dianhydride obtained in Example 6 (tetracarboxylic dianhydride represented by the general formula (18)) made it possible to achieve a lower loss tangent (tanδ) compared to a polyimide formed by using the tetracarboxylic dianhydride represented by the general formula (19). From these results, it is found that when used in, for example, an interlayer insulating film material for semiconductor, a board film for a flexible printed circuit board, and the like, the polyimide obtained in Example 7 has a lower transmission loss in comparison with the polyimide obtained in Comparative Example 5. For this reason, the polyimide obtained in Example 7 is preferably applicable to, for example, a high frequency band material and the like.

### (Example 8)

### <Step of Preparing Polyamic Acid>

A reaction liquid (solution of polyamic acid) was obtained by employing the same step as the step of preparing a polyamic acid employed in Example 7 except that 1.11 g (3.00 mmol) of 4,4'-bis(4-aminophenoxy)biphenyl (APBP) was used instead of using 1.232 g (3.00 mmol) of 2,2-bis[4-(4-aminophenoxy)phenyl]propane (BAPP) as an aromatic diamine. Note that the intrinsic viscosity [η] of the polyamic acid was 0.619 dL/g.

### <Step of Preparing Polyimide (Thermal Imidization Step) >

A film made of polyimide (film having sizes of length: 76 mm, width 52 mm, and thickness 19 µm) was obtained in the same manner as in Example 7 except that the solution of polyamic acid obtained by using 1.11 g (3.00 mmol) of 4,4'-bis(4-aminophenoxy)biphenyl (APBP) was used as a reaction liquid (solution of polyamic acid).

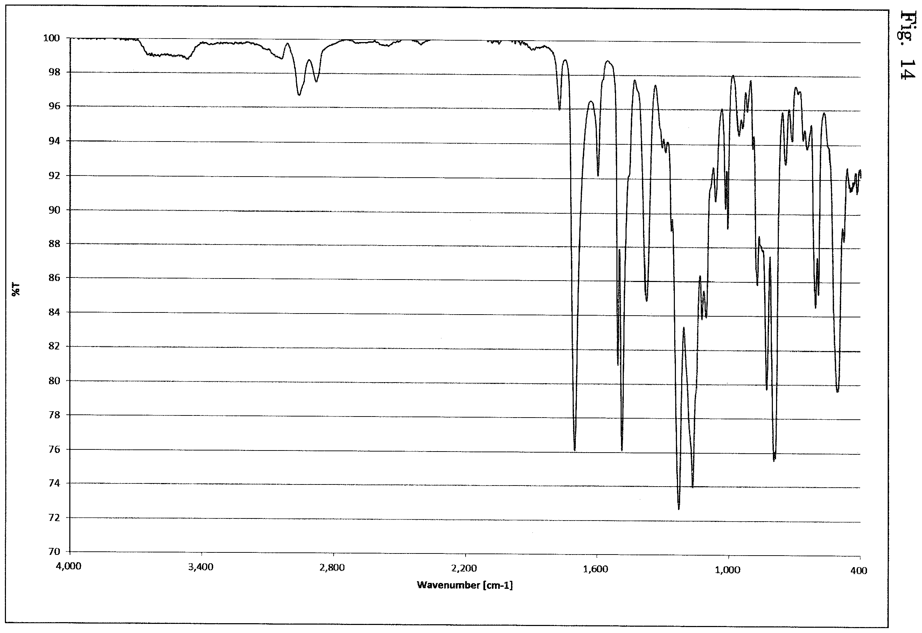

An IR spectrum of the film obtained in Example 8 was measured. Fig. 14 shows the IR spectrum of the obtained film. As is apparent from the results shown in Fig. 14, a C=O stretching vibration of imidocarbonyl was observed at 1701.1 cm⁻¹, indicating that the obtained film was made of a polyimide. Note that, from the type of monomer used, the results of the IR spectrum, and the like, the polyimide obtained in Example 8 was found to be a polyimide which contained the repeating unit represented by the general formula (1) (each of R¹, R², and R³ in the formula is a hydrogen atom, and R⁵ is a group represented by the general formula (12) (Q in the formula is -O-C₆H₄-C₆H₄-O-)). Table 5 shows evaluation results of characteristics of the thus obtained polyimide.

### (Comparative Example 6)

A film made of polyimide (film having sizes of length 76 mm, width 52 mm, and thickness 13 µm) was obtained by employing the same method as in Comparative Example 1 except that, in the step of preparing a polyamic acid, 0.3316 g (0.90 mmol) of 4,4'-bis(4-aminophenoxy)biphenyl (APBP) was used instead of using 0.1802 g (0.90 mmol) of 4,4'-diaminodiphenyl ether as an aromatic diamine.

Note that when the molecular structure of the compound forming the obtained film was identified in the same manner as in Example 3, the compound forming the film obtained in Comparative Example 6 was identified to be a polyimide. In addition, the intrinsic viscosity [η] of the polyamic acid obtained in the step of preparing a polyamic acid was 0.91 dL/g. Furthermore, Table 5 shows evaluation results of characteristics of the thus obtained polyimide.

**[Table 5]**

| | Type of Tetracarboxylic Dianhydride | Type of Aromatic Diamine | Condition of Preparation | | Characteristic of Polyimide | | | | | | |
|---|---|---|---|---|---|---|---|---|---|---|---|
| | | | Stirring Temperature in Step of Preparing Polyamic Acid (Unit: °C) | Final Heating Temperature in Preparing Polyimide (Unit: °C) | Tg (Unit: °C) | Softening Temperature (Unit: °C) | Total Luminous Transmittance (Unit: %) | HAZE | YI | εr | tanδ |
| Example 8 | Compound Represented by General Formula (18) | APBP | 25 | 300 | 295 | 480 | 86 | 3.7 | 5.5 | 3.08 | 0.0086 |
| Comparative Example 6 | Compound Represented by General Formula (19) | | 25 | 350 | 295 | 474 | 87 | 4.9 | 3.9 | 2.76 | 0.0107 |

### [Evaluation Results of Characteristics of Polyimides Obtained in Example 8 and Comparative Example 6]

From the results shown in Table 5, each of the polyimides obtained in Example 8 and Comparative Example 6 was found to have a sufficiently high light transmittance. In addition, each of the polyimides obtained in Example 8 and Comparative Example 6 had a softening temperature of 474°C or higher and was found to have an excellent heat resistance. Here, comparison between the polyimide obtained in Example 8 and the polyimide obtained in Comparative Example 6 shows that the softening temperature of the polyimide obtained in Example 8 is a higher temperature. The polyimide obtained in Example 8 makes it possible to achieve a heat resistance at a higher level with the softening temperature as a reference. Note that the polyimide obtained in Example 8 and the polyimide obtained in Comparative Example 6 have different types of tetracarboxylic dianhydride used in the production and have different structure portions of the repeating unit derived from the tetracarboxylic dianhydride. From these results , the polyimide (polyimide containing the repeating unit represented by the general formula (1)) formed by using the tetracarboxylic dianhydride obtained in Example 6 (tetracarboxylic dianhydride represented by the general formula (18)) was found to have a higher heat resistance compared to the polyimide obtained by using the tetracarboxylic dianhydride represented by the general formula (19).

In addition, as is apparent from the results shown in Table 5, the polyimide obtained in Example 8 had a value of loss tangent (tanδ) lower than that of the polyimide obtained in Comparative Example 6. As described above, it was found that the polyimide (Example 8: polyimide containing the repeating unit represented by the general formula (1)) formed by using the tetracarboxylic dianhydride obtained in Example 6 (tetracarboxylic dianhydride represented by the general formula (18)) made it possible to achieve a lower loss tangent (tanδ) compared to a polyimide formed by using the tetracarboxylic dianhydride represented by the general formula (19). From these results, it is found that when used in, for example, an interlayer insulating film material for semiconductor, a board film for a flexible printed circuit board, and the like, the polyimide obtained in Example 8 has a lower transmission loss in comparison with the polyimide obtained in Comparative Example 6. For this reason, the polyimide obtained in Example 8 is preferably applicable to, for example, a high frequency band material and the like.

### [Industrial Applicability]

As described above, according to the present invention, it is possible to provide a tetracarboxylic dianhydride which is usable as a raw material monomer for producing a polyimide having a sufficient light transmittance and a heat resistance at a higher level, as well as a production method which makes it possible to produce the tetracarboxylic dianhydride efficiently and surely.

In addition, according to the present invention, it is possible to provide a carbonyl compound which can be used for efficiently producing the tetracarboxylic dianhydride as well as a production method which makes it possible to produce the carbonyl compound efficiently and surely.

Moreover, according to the present invention, it is possible to provide a polyimide which can have a sufficient light transmittance and a heat resistance at a higher level and a method for producing a polyimide which makes it possible to produce the polyimide efficiently and surely, as well as to provide a film using the polyimide.

In addition, according to the present invention, it is possible to provide a polyamic acid which can be preferably utilized for producing the polyimide and which can be efficiently produced by using the tetracarboxylic dianhydride and a method for producing a polyamic acid which makes it possible to produce the polyamic acid efficiently and surely, as well as to provide a polyamic acid solution containing the polyamic acid.

As described above, according to the present invention, it is possible to provide a polyimide which can have a sufficient light transmittance and a heat resistance at a higher level. Thus, the tetracarboxylic dianhydride, the polyamic acid, and the polyimide of the present invention are particularly useful as materials and the like for producing, for example, films for flexible wiring boards, heat-resistant insulating tapes, enameled wires, protective coating agents for semiconductors, liquid crystal orientation films, transparent electrically conductive films for organic ELs, flexible substrate films, flexible transparent electrically conductive films, transparent electrically conductive films for organic thin film-type solar cells, transparent electrically conductive films for dye-sensitized-type solar cells, various types of gas barrier film substrates (flexible gas barrier films and the like), films for touch panels, seamless polyimide belts (so-called transfer belts) for copiers, transparent electrode substrates (transparent electrode substrates for organic ELs, transparent electrode substrates for solar cells, transparent electrode substrates for electronic paper, and the like), interlayer insulating films, sensor substrates, substrates for image sensors, reflectors for light-emitting diodes (LED) (reflectors for LED illumination: LED reflectors), covers for LED illumination, covers for LED reflector illumination, coverlay films, highly extensible composite substrates, resists for semiconductors, lithium-ion batteries, substrates for organic memories, substrates for organic transistors, substrates for organic semiconductors, color filter base materials, and the like.

## Claims

1. A tetracarboxylic dianhydride, which is a compound represented by the following general formula (1): 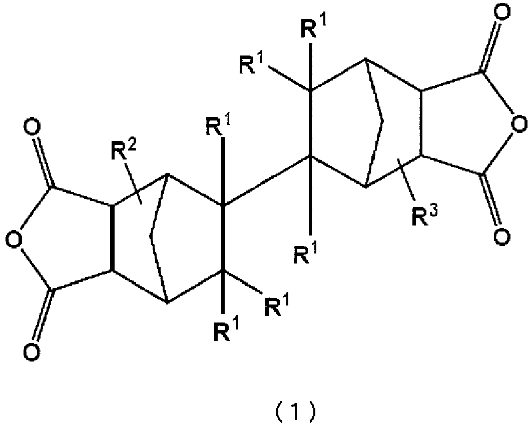
[in the formula (1), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group, and
R² and R³ each independently represent one selected from the group consisting of a hydrogen atom and alkyl groups having 1 to 10 carbon atoms].

2. The tetracarboxylic dianhydride according to claim 1, wherein
each of multiple R¹s, R², and R³ in the general formula (1) is a hydrogen atom.

3. A carbonyl compound, which is a compound represented by the following general formula (2): 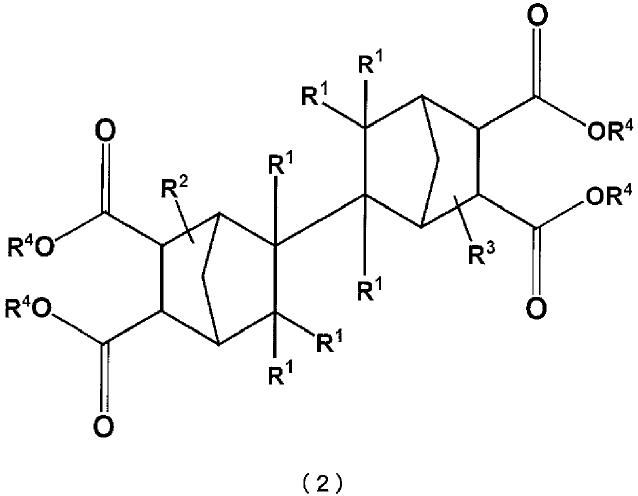
[in the formula (2), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group,
R² and R³ each independently represent one selected from the group consisting of a hydrogen atom and alkyl groups having 1 to 10 carbon atoms, and
multiple R⁴s each independently represent one selected from the group consisting of a hydrogen atom, alkyl groups having 1 to 10 carbon atoms, cycloalkyl groups having 3 to 10 carbon atoms, alkenyl groups having 2 to 10 carbon atoms, aryl groups having 6 to 20 carbon atoms, and aralkyl groups having 7 to 20 carbon atoms].

4. The carbonyl compound according to claim 3, wherein each of multiple R¹s, R², and R³ in the general formula (2) is a hydrogen atom.

5. A method for producing the tetracarboxylic dianhydride of claim 1, comprising
heating the carbonyl compound of claim 2 in a carboxylic acid having 1 to 5 carbon atoms with an acid catalyst being used, to thereby obtain the tetracarboxylic dianhydride of claim 1 represented by the general formula (1) .

6. The method for producing the tetracarboxylic dianhydride according to claim 5, wherein
the heating further uses acetic anhydride.

7. A method for producing the carbonyl compound of claim 3, comprising
reacting a norbornene-based compound represented by the following general formula (3): 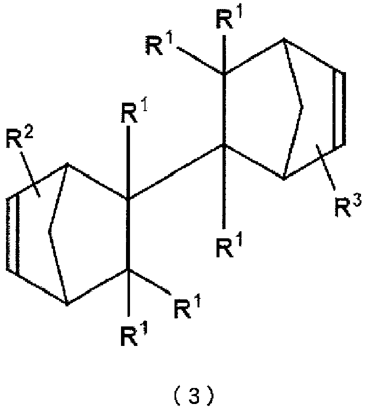
[in the formula (3), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group, and
R² and R³ each independently represent one selected from the group consisting of a hydrogen atom and alkyl groups having 1 to 10 carbon atoms] with an alcohol and carbon monoxide in the presence of a palladium catalyst and an oxidant, to thereby obtain the carbonyl compound of claim 3 represented by the general formula (2).

8. A polyimide comprising a repeating unit represented by the following general formula (4): 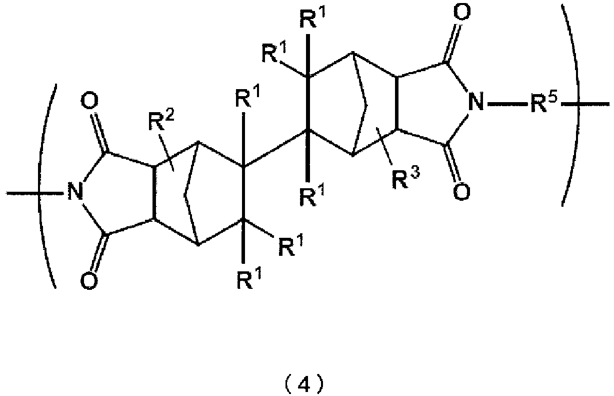
[in the formula (4), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group,
R² and R³ each independently represent one selected from the group consisting of a hydrogen atom and alkyl groups having 1 to 10 carbon atoms, and
R⁵ represents an arylene group having 6 to 40 carbon atoms].

9. A polyamic acid comprising a repeating unit represented by the following general formula (5): 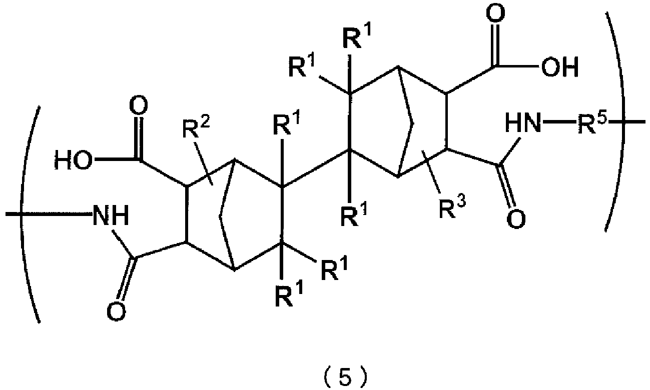
[in the formula (5), multiple R¹s each independently represent one selected from the group consisting of a hydrogen atom, alkyl groups having 1 to 10 carbon atoms, a hydroxy group, and a nitro group, or two R¹s connected to a common carbon atom may together form a methylidene group,
R² and R³ each independently represent one selected from the group consisting of a hydrogen atom and alkyl groups having 1 to 10 carbon atoms, and
R⁵ represents an arylene group having 6 to 40 carbon atoms].

10. A method for producing the polyamic acid of claim 9, comprising
reacting the tetracarboxylic dianhydride of claim 1
with an aromatic diamine represented by the following general formula (6):
H₂N-R⁵-NH₂ (6)
[in the formula (6), R⁵ represents an arylene group having 6 to 40 carbon atoms] in the presence of an organic solvent, to thereby obtain the polyamic acid of claim 9.

11. A method for producing the polyimide of claim 8, comprising
imidizing the polyamic acid of claim 9 to thereby obtain the polyimide of claim 8.

12. The method for producing the polyimide according to claim 11, comprising the step of
reacting the tetracarboxylic dianhydride of claim 1 with an aromatic diamine represented by the following general formula (6):
**H₂N-R⁵-NH₂** **(6)**
[in the formula (6), R⁵ represents an arylene group having 6 to 40 carbon atoms] in the presence of an organic solvent, to thereby obtain the polyamic acid of claim 9.

13. A polyamic acid solution, comprising the polyamic acid according to claim 9 and an organic solvent.

14. A film, comprising the polyimide according to claim 8.

## Patentansprüche

1. Tetracarbonsäuredianhydrid, das eine Verbindung mit der folgenden allgemeinen Formel (1) ist: 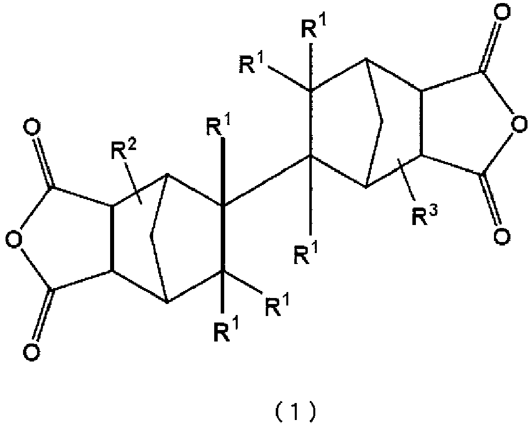
[worin in der Formel (1) mehrere R¹ jeweils unabhängig eines bedeuten, ausgewählt aus der Gruppe, bestehend aus einem Wasserstoffatom, Alkylgruppen mit 1 bis 10 Kohlenstoffatomen, Hydroxygruppe und Nitrogruppe oder worin zwei R¹, die an ein gemeinsames Kohlenstoffatom gebunden sind, eine Methylidengruppe bilden können und
R² und R³ jeweils unabhängig zumindest eines sind, ausgewählt aus der Gruppe, bestehend aus einem Wasserstoffatom und Alkylgruppen mit 1 bis 10 Kohlenstoffatomen].

2. Tetracarbonsäuredianhydrid gemäß Anspruch 1, worin jedes von mehreren R¹, R² und R³ in der allgemeinen Formel (1) ein Wasserstoffatom ist.

3. Carbonylverbindung, die eine Verbindung mit der folgenden allgemeinen Formel (2) ist: 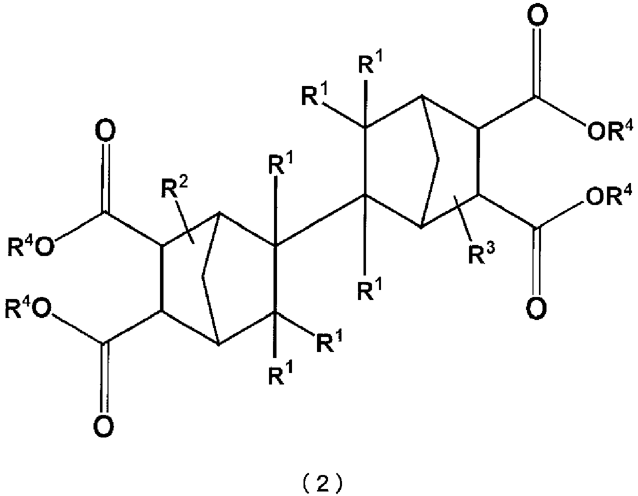
[worin in der Formel (2) mehrere R¹ jeweils unabhängig eines sind, ausgewählt aus der Gruppe, bestehend aus einem Wasserstoffatom, Alkylgruppen mit 1 bis 10 Kohlenstoffatomen, Hydroxygruppe und Nitrogruppe oder zwei Gruppen R¹, die an ein gemeinsames Kohlenstoffatom gebunden sind, zusammen eine Methylidengruppe bilden können,
R² und R³ jeweils unabhängig eines sind, ausgewählt aus der Gruppe, bestehend aus einem Wasserstoffatom und Alkylgruppen mit 1 bis 10 Kohlenstoffatomen und
mehrere R⁴ jeweils unabhängig eines sind, ausgewählt aus der Gruppe, bestehend aus einem Wasserstoffatom, Alkylgruppen mit 1 bis 10 Kohlenstoffatomen,
Cycloalkylgruppen mit 3 bis 10 Kohlenstoffatomen, Alkenylgruppen mit 2 bis 10 Kohlenstoffatomen, Arylgruppen mit 6 bis 20 Kohlenstoffatomen und Aralkylgruppen mit 7 bis 20 Kohlenstoffatomen].

4. Carbonylverbindung gemäß Anspruch 3, worin jedes von mehreren R¹, R² und R³ in der allgemeinen Formel (2) ein Wasserstoffatom ist.

5. Verfahren zur Erzeugung des Tetracarbonsäuredianhydrides gemäß Anspruch 1, enthaltend Erwärmen der Carbonylverbindung gemäß Anspruch 2 in einer Carbonsäure mit 1 bis 5 Kohlenstoffatomen, worin ein saurer Katalysator verwendet wird, unter Erhalt des Tetracarbonsäuredianhydrides nach Anspruch 1 mit der allgemeinen Formel (1).

6. Verfahren zur Erzeugung des Tetracarbonsäuredianhydrides gemäß Anspruch 5, worin beim Erwärmen weiterhin Essigsäureanhydrid verwendet wird.

7. Verfahren zur Erzeugung der Carbonylverbindung gemäß Anspruch 3, enthaltend die Reaktion einer Norbornenbasierten Verbindung mit der folgenden allgemeinen Formel (3): 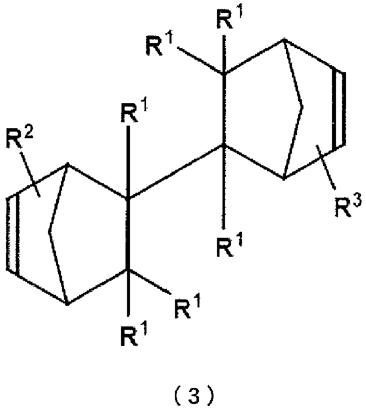
[worin in der Formel (3) mehrere R¹ jeweils unabhängig eines sind, ausgewählt aus der Gruppe, bestehend aus einem Wasserstoffatom, Alkylgruppen mit 1 bis 10 Kohlenstoffatomen, Hydroxygruppe und Nitrogruppe oder zwei R¹, die an ein gemeinsames Kohlenstoffatom gebunden sind, zusammen eine Methylidengruppe bilden können, und
R² und R³ jeweils unabhängig eines sind, ausgewählt aus der Gruppe, bestehend aus einem Wasserstoffatom und Alkylgruppen mit 1 bis 10 Kohlenstoffatomen] mit einem Alkohol und Kohlenmonoxid in der Gegenwart eines Palladiumkatalysators und eines Oxidationsmittels, unter Erhalt der Carbonylverbindung gemäß Anspruch 3 mit der allgemeinen Formel (2).

8. Polyimid, enthaltend eine Wiederholungseinheit mit der folgenden allgemeinen Formel (4): 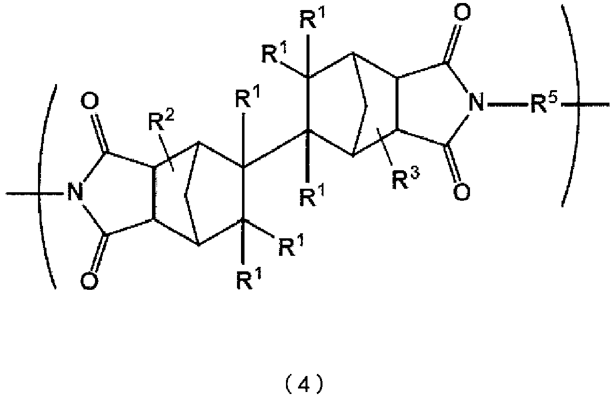
[worin in der Formel (4) mehrere R¹ jeweils unabhängig eines sind, ausgewählt aus der Gruppe, bestehend aus einem Wasserstoffatom, Alkylgruppen mit 1 bis 10 Kohlenstoffatomen, Hydroxygruppe und Nitrogruppe oder zwei R¹, die an ein gemeinsames Kohlenstoffatom gebunden sind, zusammen eine Methylidengruppe bilden können, und
R² und R³ jeweils unabhängig eines sind, ausgewählt aus der Gruppe, bestehend aus einem Wasserstoffatom und Alkylgruppen mit 1 bis 10 Kohlenstoffatomen und
R⁵ eine Arylengruppe mit 6 bis 40 Kohlenstoffatomen ist].

9. Polyamidsäure mit einer Wiederholungseinheit mit der folgenden allgemeinen Formel (5): 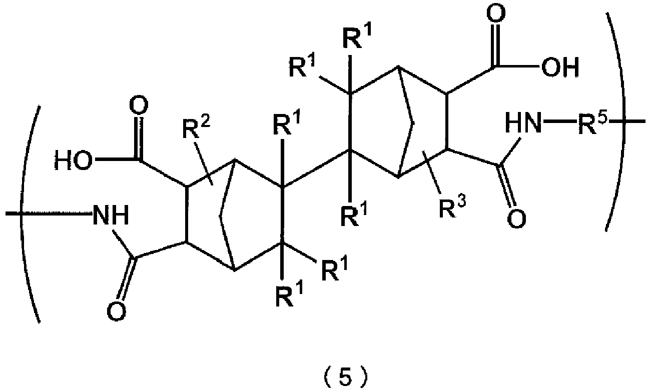
[worin in der Formel (5) mehrere R¹ jeweils unabhängig eines sind, ausgewählt aus der Gruppe, bestehend aus einem Wasserstoffatom, Alkylgruppen mit 1 bis 10 Kohlenstoffatomen, Hydroxygruppe und Nitrogruppe oder zwei R¹, die an ein gemeinsames Kohlenstoffatom gebunden sind, zusammen eine Methylidengruppe bilden können, und
R² und R³ jeweils unabhängig eines sind, ausgewählt aus der Gruppe, bestehend aus einem Wasserstoffatom und Alkylgruppen mit 1 bis 10 Kohlenstoffatomen und
R⁵ eine Arylengruppe mit 6 bis 40 Kohlenstoffatomen ist].

10. Verfahren zur Erzeugung der Polyamidsäure gemäß Anspruch 9, enthaltend die Reaktion des Tetracarbonsäuredianhydrides gemäß Anspruch 1 mit einem aromatischen Diamin mit der folgenden allgemeinen Formel (6) :
**H₂N-R⁵-NH₂** **(6)**
[worin in der Formel (6) R⁵ eine Arylengruppe mit 6 bis 40 Kohlenstoffatomen ist] in der Gegenwart eines organisches Lösungsmittels, unter Erhalt der Polyamidsäure gemäß Anspruch 9.

11. Verfahren zur Erzeugung des Polyimides gemäß Anspruch 8, enthaltend Imidisieren der Polyamidsäure gemäß Anspruch 9, unter Erhalt des Polyamides gemäß Anspruch 8.

12. Verfahren zur Erzeugung des Polyimides gemäß Anspruch 11, enthaltend die Reaktion des Tetracarbonsäuredianhydrides gemäß Anspruch 1 mit einem aromatischen Diamin mit der folgenden allgemeinen Formel (6) :
**H₂N-R⁵-NH₂** **(6)**
[worin in der Formel (6) R⁵ eine Arylengruppe mit 6 bis 40 Kohlenstoffatomen ist] in der Gegenwart eines organisches Lösungsmittels, unter Erhalt der Polyaminsäure gemäß Anspruch 9.

13. Polyamidsäurelösung, enthaltend die Polyamidsäure gemäß Anspruch 9 und ein organisches Lösungsmittel.

14. Film, enthaltend das Polyimid gemäß Anspruch 8.

## Revendications

1. Dianhydride tétracarboxylique, qui est un composé représenté par la formule générale (1) suivante : 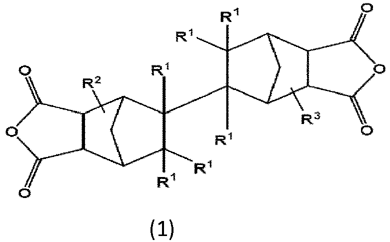
[dans la formule (1), les multiples R¹ représentent chacun indépendamment l'un sélectionné dans le groupe consistant en un atome d'hydrogène, des groupes alkyles ayant de 1 à 10 atomes de carbone, un groupe hydroxy et un groupe nitro, ou deux R¹ reliés à un atome de carbone commun peuvent former ensemble un groupe méthylidène, et
R² et R³ représentent chacun indépendamment l'un sélectionné dans le groupe consistant en un atome d'hydrogène et des groupes alkyles ayant de 1 à 10 atomes de carbone].

2. Dianhydride tétracarboxylique selon la revendication 1, dans lequel
chacun des multiples R¹, R² et R³ dans la formule générale (1) est un atome d'hydrogène.

3. Composé carbonylé, qui est un composé représenté par la formule générale (2) suivante : 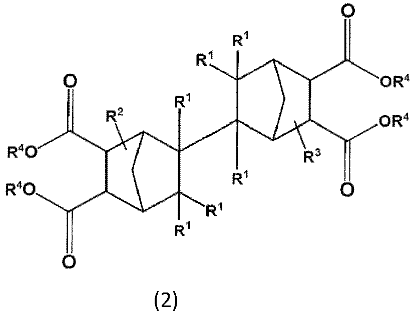
[dans la formule (2), les multiples R¹ représentent chacun indépendamment l'un sélectionné dans le groupe consistant en un atome d'hydrogène, des groupes alkyles ayant de 1 à 10 atomes de carbone, un groupe hydroxy, et un groupe nitro, ou deux R¹ reliés à un atome de carbone commun peuvent former ensemble un groupe méthylidène,
R² et R³ représentent chacun indépendamment l'un sélectionné dans le groupe consistant en un atome d'hydrogène et des groupes alkyles ayant de 1 à 10 atomes de carbone, et
les multiples R⁴ représentent chacun indépendamment l'un sélectionné dans le groupe consistant en un atome d'hydrogène, des groupes alkyles ayant de 1 à 10 atomes de carbone, des groupes cycloalkyles ayant de 3 à 10 atomes de carbone, des groupes alcényles ayant de 2 à 10 atomes de carbone, des groupes aryles ayant de 6 à 20 atomes de carbone et des groupes aralkyles ayant de 7 à 20 atomes de carbone].

4. Composé carbonylé selon la revendication 3, dans lequel
chacun des multiples R¹, R² et R³ dans la formule générale (2) est un atome d'hydrogène.

5. Procédé pour produire le dianhydride tétracarboxylique selon la revendication 1, comprenant
le chauffage du composé carbonylé selon la revendication 2 dans un acide carboxylique ayant de 1 à 5 atomes de carbone avec un catalyseur acide qui est utilisé, pour obtenir, de ce fait, le dianhydride tétracarboxylique selon la revendication 1 représenté par la formule générale (1).

6. Procédé pour produire le dianhydride tétracarboxylique selon la revendication 5, dans lequel
le chauffage utilise en outre de l'anhydride acétique.

7. Procédé pour produire le composé carboxylé selon la revendication 3, comprenant
la mise en réaction d'un composé à base de norbornène représenté par la formule générale (3) suivante : 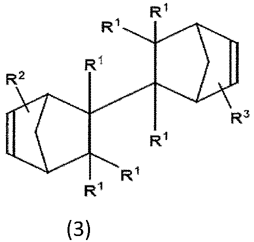
[dans la formule (3), les multiples R¹ représentent chacun indépendamment l'un sélectionné dans le groupe consistant en un atome d'hydrogène, des groupes alkyles ayant de 1 à 10 atomes de carbone, un groupe hydroxy et un groupe nitro, ou deux R¹ reliés à un atome de carbone commun peuvent former ensemble un groupe méthylidène, et
R² et R³ représentent chacun indépendamment l'un sélectionné dans le groupe consistant en un atome d'hydrogène et des groupes alkyles ayant de 1 à 10 atomes de carbone] avec un alcool et un monoxyde de carbone en présence d'un catalyseur au palladium et d'un oxydant, pour obtenir, de ce fait, le composé carboxylé selon la revendication 3 représenté par la formule générale (2).

8. Polyimide comprenant une unité de répétition représentée par la formule générale (4) suivante : 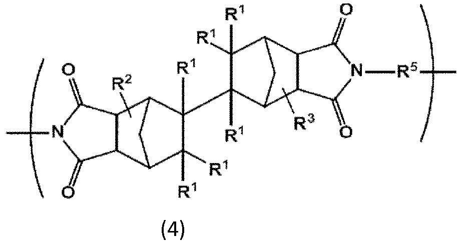
[dans la formule (4), les multiples R¹ représentent chacun indépendamment l'un sélectionné dans le groupe consistant en un atome d'hydrogène, des groupes alkyles ayant de 1 à 10 atomes de carbone, un groupe hydroxy, et un groupe nitro, ou deux R¹ reliés à un atome de carbone commun peuvent former ensemble un groupe méthylidène,
R² et R³ représentent chacun indépendamment l'un sélectionné dans le groupe consistant en un atome d'hydrogène et des groupes alkyles ayant de 1 à 10 atomes de carbone, et
R⁵ représente un groupe arylène ayant de 6 à 40 atomes de carbone].

9. Acide polyamique comprenant une unité de répétition représentée par la formule générale (5) suivante : 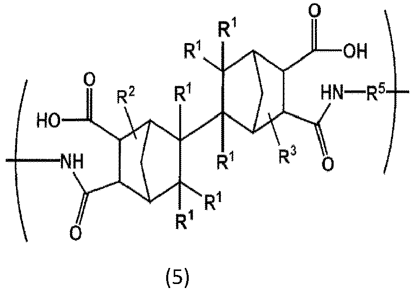
[dans la formule (5), les multiples R¹ représentent chacun indépendamment l'un sélectionné dans le groupe consistant en un atome d'hydrogène, des groupes alkyles ayant de 1 à 10 atomes de carbone, un groupe hydroxy et un groupe nitro, ou deux R¹ reliés à un atome de carbone commun peuvent former ensemble un groupe méthylidène,
R² et R³ représentent chacun indépendamment l'un sélectionné dans le groupe consistant en un atome d'hydrogène et des groupes alkyles ayant de 1 à 10 atomes de carbone, et
R⁵ représente un groupe arylène ayant de 6 à 40 atomes de carbone].

10. Procédé pour produire l'acide polyamique selon la revendication 9, comprenant :
la mise en réaction du dianhydride tétracarboxylique selon la revendication 1 avec une diamine aromatique représentée par la formule générale (6) suivante :
H₂N-R⁵-NH₂ (6)
[dans la formule (6), R⁵ représente un groupe arylène ayant de 6 à 40 atomes de carbone] en présence d'un solvant organique, pour obtenir, de ce fait, l'acide polyamique selon la revendication 9.

11. Procédé pour produire le polyimide selon la revendication 8, comprenant
l'imidisation de l'acide polyamique selon la revendication 9 pour obtenir, de ce fait, le polyimide selon la revendication 8.

12. Procédé pour produire le polyimide selon la revendication 11, comprenant l'étape suivante
mise en réaction du dianhydride tétracarboxylique selon la revendication 1 avec une diamine aromatique représentée par la formule générale (6) suivante :
H₂N-R⁵-NH₂ (6)
[dans la formule (6), R⁵ représente un groupe arylène ayant de 6 à 40 atomes de carbone] en présence d'un solvant organique, pour obtenir, de ce fait, l'acide polyamique selon la revendication 9.

13. Solution d'acide polyamique, comprenant l'acide polyamique selon la revendication 9 et un solvant organique.

14. Film, comprenant le polyimide selon la revendication 8.
